# EUROPÄISCHE PATENTSCHRIFT

(11) **EP 1 383 777 B1**
(45) Veröffentlichungstag und Bekanntmachung des Hinweises auf die Patenterteilung: **16.11.2005**
(21) Anmeldenummer: 02761895.8
(22) Anmeldetag: 28.03.2002
(51) Int. Cl.: C07F 9/00, C07F 9/572, C07F 9/74, C07F 9/92

(54) **LIGANDEN FÜR PNICOGENCHELATKOMPLEXE MIT EINEM METALL DER VIII. NEBENGRUPPE UND VERWENDUNG DER KOMPLEXE ALS KATALYSATOREN FÜR HYDROFORMYLIERUNG, CARBONYLIERUNG, HYDROCYANIERUNG ODER HYDRIERUNG**
LIGANDS FOR PNICOGEN CHELATE COMPLEXES WITH A METAL OF SUBGROUP VIII AND USE OF THE COMPLEXES AS CATALYSTS FOR HYDROFORMYLATION, CARBONYLATION, HYDROCYANATION OR HYDROGENATION
LIGANDS POUR DES COMPLEXES CHELATE DE PNICTOGENE A BASE D'UN METAL DU GROUPE VIII DE LA CLASSIFICATION PERIODIQUE ET UTILISATION DE CES COMPLEXES COMME CATALYSEURS POUR L'HYDROFORMYLATION, LA CARBONYLATION, L'HYDROCYANATION OU L'HYDROGENATION

(30) Priorität: 29.03.2001 DE 10115689; 24.08.2001 DE 10141494
(43) Veröffentlichungstag der Anmeldung: 28.01.2004
(73) Patentinhaber: BASF Aktiengesellschaft, 67056 Ludwigshafen (DE)
(72) Erfinder: AHLERS, Wolfgang, 67549 Worms (DE); PACIELLO, Rocco, 67098 Bad Dürkheim (DE); VOGT, Dieter, 5600 MB Eindhoven (NL); HOFMANN, Peter, 69118 Heidelberg (DE)
(74) Vertreter: Pohl, Michael Friedrich
(86) Internationale Anmeldenummer: PCT/EP2002/003543
(87) Internationale Veröffentlichungsnummer: WO 2002/083695

(56) Entgegenhaltungen:
- WO-A-01/58589
- WO-A-98/19985

## Beschreibung

Die vorliegende Erfindung betrifft neue Pnicogenchelatverbindungen, Katalysatoren umfassend Pnicogenchelatkomplexe mit einem Metall der VIII. Nebengruppe des Periodensystems der Elemente, die als Liganden mindestens eine Pnicogenchelatverbindung der Formel I enthalten, sowie ein Verfahren zur Herstellung von Aldehyden und/oder Alkoholen durch die Hydroformylierung von C₃- bis C₂₀-Olefinen unter Verwendung dieser Katalysatoren. Die Erfindung betrifft weiterhin ein Verfahren zur Herstellung von 2-Propylheptanol, umfassend die Hydroformylierung von Buten, eine Aldolkondensation der so erhaltenen Hydroformylierungsprodukte und die katalytische Hydrierung der Kondensationsprodukte.

Seit der Entdeckung der Hydroformylierungsreaktion durch Otto Roelen im Jahre 1938 hat sich die Hydroformylierung zu dem Paradebeispiel der Anwendung der Homogenkatalyse mittels organometallischer Katalysatoren im industriellen Maßstab entwickelt. So werden auf Basis homogen katalysierter Hydroformylierungsverfahren weltweit mehrere Millionen Tonnen Aldehyde und/oder Alkohole produziert. Als Katalysatoren werden hierzu hauptsächlich Kobalt- und Rhodiumcarbonylverbindungen eingesetzt, die gegebenenfalls mittels anderer Liganden, z. B. Triphenylphosphinliganden, hinsichtlich ihrer Reaktivität und Selektivität modifiziert sein können. Für die Herstellung von C₃-C₅-Aldehyden aus linearen olefinen mit endständiger Doppelbindung ("α-Olefine") ist der Einsatz modifizierter Rhodiumcarbonylverbindungen inzwischen die Methode der Wahl, wohingegen für die Herstellung längerkettiger Aldehyde und/oder Alkohole immer noch die Kobaltcarbonyl-katalysierte Hydroformylierung in Gebrauch ist, obgleich die Kobalt-Katalyse im Vergleich zur Katalyse mit ligand-modifizierten Rhodiumverbindungen den Nachteil hat, dass bei höherem Druck gearbeitet werden muss.

Der Grund hierfür ist im unterschiedlichen katalytischen Verhalten dieser Katalysatormetalle zu suchen: Gerade bei der Herstellung längerkettiger Aldehyde dienen oftmals technisch verfügbare Olefingemische als Ausgangsmaterial, die sowohl endständige als auch interne Olefine beinhalten. Bei der Hydroformylierung endständiger Olefine können sich, je nach Ort der Anlagerung des CO-Moleküls an die Doppelbindung lineare Aldehyde, auch als n-Aldehyde bezeichnet, oder verzweigte Aldehyde, auch als iso-Aldehyde bezeichnet, bilden, wobei in der Regel ein möglichst hoher n-Anteil der Aldehyde im Reaktionsgemisch erwünscht ist. Während das Rhodium-Triphenylphosphin-Katalysatorsystem zu hohen n-Selektivitäten bei der Bildung kurzkettiger Aldehyde führt, konnte sich dieses System für die Hydroformylierung längerkettiger Olefine nicht in gleicher Weise etablieren. Ein Grund hierfür ist zum einen die vom Rhodium-Katalysator bewirkte Isomerisierung endständiger Doppelbindungen zu internen Doppelbindungen, die unter den Bedingungen der Hydroformylierungsreaktion in nennenswertem Umfang stattfindet, zum anderen die mangelnde Hydroformylierungsaktivität des Rhodium-Triphenylphosphin-Katalysators bezüglich interner Doppelbindungen.

Die Hydroformylierung längerkettiger Olefine ist im Hinblick auf die Herstellung von Weichmacheralkoholen (Alkohole zur Herstellung von Ester-Weichmachern) und Tensidalkoholen von großer wirtschaftlicher Bedeutung. So werden zur Modifizierung der thermoplastischen Eigenschaften einer Vielzahl großtechnisch wichtiger Produkte, wie speziell Kunststoffe, aber auch Lacke, Beschichtungsmittel, Dichtungsmassen etc. in großen Mengen sogenannte Weichmacher eingesetzt. Eine wichtige Klasse von Weichmachern sind die Ester-Weichmacher, zu denen unter anderem Phthalsäureester, Adipinsäureester, Trimellithsäureester, Phosphorsäureester etc. zählen. Zur Herstellung von Ester-Weichmachern mit guten anwendungstechnischen Eigenschaften besteht ein Bedarf an Weichmacheralkoholen mit etwa 6 bis 12 Kohlenstoffatomen, die zu einem geringen Grad verzweigt sind (sogenannte semilinearer Alkohole), und an entsprechenden Gemischen davon. Dazu zählt insbesondere 2-Propylheptanol und es enthaltende Alkoholgemische.

Die DE-A-100 03 482 beschreibt ein integriertes Verfahren zur Herstellung von C₉-Alkoholen und C₁₀-Alkoholen aus Buten und Butan enthaltenden C₄-Kohlenwasserstoffgemischen, bei dem man unter anderem das Kohlenwasserstoffgemisch einer Hydroformylierung unterzieht und die dabei erhaltenen C₅-Aldehyde einer Aldolkondensation und anschließenden katalytischen Hydrierung zu C₁₀-Alkoholen unterzieht.

Wie eingangs ausgeführt kommt es bei der Hydroformylierung von Olefinen mit mehr als 2 C-Atomen aufgrund der möglichen CO-Anlagerung an jedes der beiden C-Atome einer Doppelbindung zur Bildung von Gemischen isomerer Aldehyde. Zusätzlich kann es auch zu einer Doppelbindungsisomerisierung kommen, d. h. zu einer Verschiebung interner Doppelbindungen auf eine terminale Position und umgekehrt. Bei der Herstellung von 2-Propylheptanol oder von Alkoholgemischen mit hohem Anteil von 2-Propylheptanol durch Hydroformylierung von Buten und anschließender Aldolkondensation kann es somit bei der Hydroformylierung leicht nicht nur zur Bildung von n-Valeraldehyd, sondern auch von unerwünschten Produktaldehyden kommen, wodurch das gesamte Verfahren wirtschaftlich benachteiligt wird.

Werden zur Hydroformylierung technische Gemische, beispielsweise C₄-Schnitte eingesetzt, die in großen Mengen sowohl aus FCC-Anlagen als auch aus Steamcrackern zur Verfügung stehen und die im Wesentlichen aus einem Gemisch von 1-Buten und 2-Buten sowie im Allgemeinen Butan bestehen, so muss der eingesetzte Hydroformylierungskatalysator möglichst selektiv die Hydroformylierung terminaler Olefine (1-Buten) ermöglichen und/oder zu einer Verschiebung interner Doppelbindungen auf eine terminale Position befähigt sein. An der Bereitstellung solcher Hydroformylierungskatalysatoren besteht auch allgemein ein großes technisches Interesse.

Aufgrund der vorstehend geschilderten Problematik führte dies in jüngster Zeit zu intensiven Forschungsarbeiten zur Entwicklung neuer Liganden, neuer Katalysatorsysteme und Verfahren zur Hydroformylierung langkettiger und/oder interner Olefine.

WO 95/30680 und van Leeuwen et al., Organometallics 14, 3081 (1995) beschreiben Chelatphosphine mit Xanthen-Rückgrat, deren Anwendung bei der Rhodium-katalysierten Hydroformylierung endständiger Olefine zu hohen n-Selektivitäten führt. Für die Hydroformylierung interner Olefine sind diese Katalysatorsysteme jedoch nicht geeignet.

Börner et al., Angew. Chem. 112, 1694 (2000) berichten über die Hydroformylierung interner, linearer Olefine mit Hilfe von mit Bisphenolmonoether-monophosphonit-modifizierten Rhodiumkatalysatoren. Die mit diesem Verfahren erzielte n-Selektivität von 35 bis 48 % ist gering.

Van Leeuwen et al., Organometallics 18, 4765 (1999), erzielten bei der Umsetzung interner Olefine mit Rhodiumkatalysatoren, welche mittels eines mit zwei Phenoxaphosphin-Gruppen substituierten Xanthen-Chelatphosphin-Liganden modifiziert waren, nach 17 Stunden einen maximalen Olefinumsatz von 67 %.

US-A 3816452 betrifft die Herstellung unterschiedlich substituierter Pyrrolyl-mono-phosphane und deren Verwendung als Flammschutzmittel.

K. G. Moloy et al., J. Am. Chem. Soc. 117, 7696 (1995) beschreibt die Herstellung, elektronische und Komplexbildungs-Eigenschaften von Bis-(dipyrrolylphosphino)ethan.

Herstellung und physiko-chemische Eigenschaften von Platinkomplexen dieses Liganden sind Gegenstand des Artikels von Smith et al., Organometallics 19, 1427 (2000). Eine konkrete Anwendung dieser Verbindungen und ihrer Metallkomplexe für katalytische Zwecke wird nicht erwähnt.

Trzeciak et al. verwenden Trispyrrolylphosphan-Rhodium-Komplexe zur Hydrierung von Arenen (J. Organomet. Chem. 552, 159 (1998)) und für die Rhodium-katalysierte Hydroformylierung (J. Chem. Soc., Dalton Trans. 1831 (1997)). Bei der Hydroformylierung von 1-Hexen werden in erheblichem Umfang Nebenprodukte, die durch die Isomerisierung von 1-Hexen zu 2-Hexen entstanden sind, gefunden. Gemäß den Darlegungen von Trzeciak et al. in J. Organomet. Chem. 575, 87 (1999) sind Rhodium-Komplexe, die als Liganden Diphenyl-2-hydroxy-phenyl-phosphan und Tris-pyrrolyl-phosphan enthalten, bei der Hydroformylierung praktisch inaktiv. C. R. Acad. Sci., Série IIc, 235 (1999) betrifft die Hydroformylierung von Vinylsilanen mittels Trispyrrolylphosphan-modifizierten Rhodiumkatalysatoren.

Die WO 00/56451 betrifft am Phosphoratom unter anderem mit Pyrrolderivaten substituierte, cyclische Oxaphosphorine und die Verwendung dieser als Liganden in Katalysatoren zur Hydroformylierung.

J. Shen et al. beschreiben in Organometallics 1998, 17, S. 3000-3005 kalorimetrische Studien an Diphosphin-Chelatliganden, wobei unter anderem Hydrazid-verbrückte Diphenylphosphine und Alkylen-verbrückte Dipyrrolphosphine eingesetzt werden.

H. Brunner und H. Weber beschreiben in Chem. Ber. 118, S. 3380-3395 (1985) optisch aktive Aminophosphane und deren Einsatz in der enantioselektiven Hydrosilylierung. Diese Liganden werden durch Kondensation von 2-Pyrrolcarbaldehyd bzw. 2-Acetylpyrrol mit 1-Phenylethylamin und gegebenenfalls weiteren Folgereaktionen hergestellt und können Pyrrolstickstoff-phosphonierte Gruppen aufweisen.

Die WO 01/58589 beschreibt Verbindungen des Phosphors, Arsens und des Antimons, basierend auf Diaryl-anellierten Bi-cyclo[2.2.2]-Grundkörpern und Katalysatoren, die diese als Liganden enthalten. Dabei können an das Atom der 5. Hauptgruppe prinzipiell auch Hetarylreste gebunden sein.

Die DE-A-100 23 471 beschreibt ein Verfahren zur Hydroformylierung unter Einsatz eines Hydroformylierungskatalysators, der wenigstens einen Phosphinliganden umfasst, der zwei Triarylphosphingruppen aufweist, wobei jeweils ein Arylrest der beiden Triarylphosphingruppen über eine Einfachbindung an eine nichtaromatische 5- bis 8-gliedrige carbocyclische oder heterocyclische verbrückende Gruppe gebunden ist. Dabei können die Phosphoratome als weitere Substituenten unter anderem auch Hetarylgruppen aufweisen.

Die deutsche Patentanmeldung P 100 46 026.7 beschreibt ein Hydroformylierungsverfahren, bei dem man als Katalysator einen Komplex auf Basis einer Phosphor-, Arsen- oder Antimon-haltigen Verbindung als Liganden einsetzt, wobei diese Verbindung jeweils zwei ein P-, As- oder Sb-Atom und wenigstens zwei weitere Heteroatome aufweisende Gruppen gebunden an ein Xanthen-artiges Molekülgerüst aufweist.

US-A 5,710,344 betrifft die Hydroformylierung von Olefinen mittels Rhodiumkatalysatoren, die mit Chelatphosphordiamidit-Liganden mit Bisphenol- oder Bisnaphthol-Rückgrat und deren Phosphoratome unsubstituierte Pyrrolyl-, Imidazolyl- oder Indolylgruppen tragen können, modifiziert sind.

Gimbert et al., J. Org. Chem. 64, 3493 (1999) berichten über Kobaltkomplexe mit N-Methyl-verbrückten Bis-pyrrolyl-phosphinen und deren Einsatz als Reagenzien bei der Pauson-Khand-Reaktion.

Benincori et al. beschreiben die Herstellung von 3,3'-Dimethyl-1,1'-bis(diphenylphosphino)-2,2'-bisindol und einen Komplex dieser Verbindung mit Palladium sowie verschiedenerlei physiko-chemische Eigenschaften dieser Verbindungen.

Chirale Katalysatorsysteme aus Komplexen dieser Verbindungen mit einem Übergangsmetall zur Durchführung stereokontrollierter Reduktionen und Isomerisierungen sind Gegenstand von WO 96/01831.

EP-A 754 715 betrifft Komplexe von Alkylen-verbrückten Di(pyrrolyl-phenyl-phosphinen) mit einem Gruppe VIII-Metall und die Verwendung dieser Komplexe als Katalysatoren zur Herstellung von Polyketonen.

Van Leeuwen et al., Organometallics 19, 2504 (2000) beschreiben die Synthese von Phosphordiamid-Chelatliganden mit Bisphenol- oder Xanthen-Rückgrat, deren Diamid-Einheit durch Biuret-Gruppen gebildet wird, sowie die katalytischen Eigenschaften der Rhodium-Komplexe dieser Verbindungen bei der Hydroformylierung.

WO 98/42716 betrifft ein Syntheseverfahren zur Herstellung von 2,2'-Bis-phosphino-1,1'-binaphthyl-Liganden, deren Phosphoratome pyrrolyl-Gruppen tragen.

DE-A 199 13 352 betrifft am Phosphoratom mit Pyrrolderivaten substituierte, cyclische Oxaphosphorine und die Verwendung dieser Liganden in Katalysatoren zur Hydroformylierung.

Die WO-A-99/52915 beschreibt chirale phosphoratomhaltige Liganden auf Basis von bicyclischen Verbindungen von carbocyclischen und heterocyclischen 5- bis 6-atomigen Verbindungen. Dabei sind die den Bicyclus bildenden aromatische Ringe über eine Einfachbindung zwischen zwei Ringkohlenstoffatomen miteinander verknüpft.

WO 99/52632 betrifft ein Verfahren zur Hydrocyanierung unter Verwendung phosphorhaltiger Chelatliganden mit u. a. 1,1'-Bisphenol- oder 1,1'-Bisnaphthol-Rückgrat, in denen das Phosphoratom mit Pyrrolgruppen substituiert sein kann.

Der vorliegenden Erfindung liegt somit die Aufgabe zugrunde, geeignete Liganden zur Verfügung zu stellen, die die Hydroformylierung längerkettiger, endständiger oder interner Olefine oder von technischen Gemischen aus Olefinen mit endständiger und interner Doppelbindung, z. B. 1-Buten/2-Buten-Gemische, zu Aldehydprodukten mit hoher Linearität bei gutem Umsatz ermöglichen. Der Erfindung liegt weiterhin die Aufgabe zugrunde, ein verbessertes Verfahren zur Herstellung von 2-Propylheptanol zur Verfügung zu stellen.

Dementsprechend wurden Pnicogenchelatliganden der allgemeinen Formel I 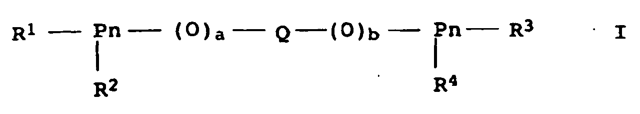 in der
- Q: eine Brückengruppe der Formel 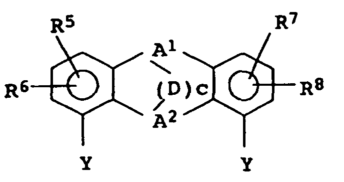
ist,
worin
- A¹ und A²: unabhängig voneinander für O, S, SiR^{a}R^{b}, NR^{c} oder CR^{d}R^{e} stehen, wobei
- R^{a}, R^{b} und R^{c}: unabhängig voneinander für Wasserstoff, Alkyl, Cycloalkyl, Heterocycloalkyl, Aryl oder Hetaryl stehen,
- R^{d} und R^{e}: unabhängig voneinander für Wasserstoff, Alkyl, Cycloalkyl, Beterocycloalkyl, Aryl oder Hetaryl stehen oder die Gruppe R^{d} gemeinsam mit einer weiteren Gruppe R^{d} oder die Gruppe R^{e} gemeinsam mit einer weiteren Gruppe R^{e} eine intramolekulare Brückengruppe D bilden,
- D: eine zweibindige Brückengruppe, ausgewählt aus den Gruppen 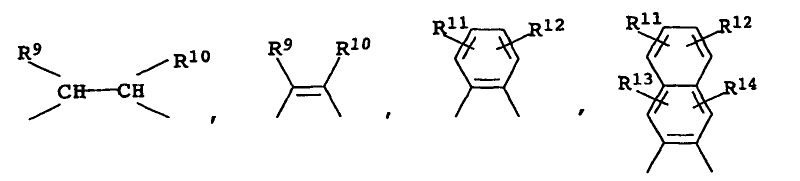
ist, in denen
- R⁹ und R¹⁰: unabhängig voneinander für Wasserstoff, Alkyl, Cycloalkyl, Aryl, Halogen, Trifluormethyl, Carboxyl, Carboxylat oder Cyano stehen oder miteinander zu einer C₃- bis C₄-Alkylenbrücke verbunden sind,
- R¹¹, R¹², R¹³ und R¹⁴: unabhängig voneinander für Wasserstoff, Alkyl, Cycloalkyl, Aryl, Halogen, Trifluormethyl, COOH, Carboxylat, Cyano, Alkoxy, SO₃H, Sulfonat, NE¹E², Alkylen-NE¹E²E³⁺X⁻, Acyl oder Nitro stehen,
- c: 0 oder 1 ist,
- Y: eine chemische Bindung darstellt,
- R⁵, R⁶, R⁷ und R⁸: unabhängig voneinander für Wasserstoff, Alkyl, Cycloalkyl, Heterocycloalkyl, Aryl, Hetaryl, COOR^{f}, COO⁻M⁺, SO₃R^{f}, SO⁻₃M⁺, NE¹E², NE¹E²E³⁺X⁻, Alkylen-NE¹E²E³⁺X⁻, OR^{f}, SR^{f}, (CHR^{g}CH₂P)ₓR^{f}, (CH₂N(E¹))ₓR^{f}, (CH₂CH₂N(E¹))ₓR^{f}, Halogen, Trifluormethyl, Nitro, Acyl oder Cyano stehen,
worin
- R^{f}, E¹, E² und E³: jeweils gleiche oder verschiedene Reste, ausgewählt unter Wasserstoff, Alkyl, Cycloalkyl oder Aryl bedeuten,
- R^{g}: für Wasserstoff, Methyl oder Ethyl steht,
- M⁺: für ein Kation steht,
- X⁻: für ein Anion steht, und
- x: für eine ganze Zahl von 1 bis 120 steht,
oder
- R⁵ und/oder R⁷: zusammen mit zwei benachbarten Kohlenstoffatomen des Benzolkerns, an den sie gebunden sind, für ein kondensiertes Ringsystem, mit 1, 2 oder 3 weiteren Ringen stehen,
- a und b: unabhängig voneinander die Zahl 0 oder 1 bedeuten
- Pn: für ein Pnicogenatom ausgewählt aus den Elementen Phosphor, Arsen oder Antimon steht,
und
- R¹, R², R³, R⁴: unabhängig voneinander für Hetaryl, Hetaryloxy, Alkyl, Alkoxy, Aryl, Aryloxy, Cycloalkyl, Cycloalkoxy, Heterocycloalkyl, Heterocycloalkoxy oder eine NE¹E²-Gruppe stehen, mit der Maßgabe, dass R¹ und R³ über das Stickstoffatom an das Pnicogenatom Pn gebundene Pyrrolgruppen sind oder worin R¹ gemeinsam mit R² und/oder R³ gemeinsam mit R⁴ eine mindestens eine über das pyrrolische Stickstoffatom an das Pnicogenatom Pn gebundene Pyrrolgruppe enthaltende zweibindige Gruppe E der Formel

Py-I-W
worin
- Py: eine Pyrrolgruppe ist,
- I: für eine chemische Bindung oder für O, S, SiR^{a}R^{b}, NR^{c} oder CR^{h}Rⁱ steht,
- W: für Cycloalkyl, Cycloalkoxy, Aryl, Aryloxy, Hetaryl oder Hetaryloxy steht,
und
- R^{h} und Rⁱ: unabhängig voneinander für Wasserstoff, Alkyl, Cycloalkyl, Heterocycloalkyl, Aryl oder Hetaryl stehen,
oder eine über die Stickstoffatome an das Pnicogenatom Pn gebundene Bispyrrolgruppe der Formel

Py-I-Py

bilden,
wobei der Ausdruck Pyrrolgruppe unsubstituierte oder substituierte Pyrolyl-, Imidazolyl-, Pyrazolyl-, Indolyl-, Purinyl-, Indazolyl-, Benzotriazolyl-, 1,2,3-Triazolyl, 1,3,4-Triazolyl- und Carbazolylgruppen umfaßt gefunden.

In einer speziellen Ausführungsform betrifft die vorliegende Erfindung Pnicogenchelatliganden, bei denen eine substituierte und/oder in ein anelliertes Ringsystem integrierte Pyrrolgruppe über ihr pyrrolisches Stickstoffatom kovalent mit dem Pnicogenatom verknüpft ist.

Außerdem betrifft die vorliegende Erfindung Katalysatoren, umfassend Pnicogenchelatkomplexe mit einem Metall der VIII. Nebengruppe des Periodensystems der Element, die als Liganden mindestens einen Pnicogenchelatliganden der der allgemeinen Formel I enthalten.

Ein weiterer Gegenstand der vorliegenden Erfindung ist ein Verfahren zur Hydroformylierung von Verbindungen, die wenigstens eine ethylenisch ungesättigte Doppelbindung enthalten und speziell ein Verfahren zur Herstellung von Aldehyden und/oder Alkoholen durch die Hydroformylierung von C₃-C₂₀-Olefinen, vorzugsweise von C₄-C₂₀-Olefinen, bei erhöhtem Druck und erhöhter Temperatur mittels CO/H₂-Gemischen in Gegenwart einer homogen im Reaktionsmedium gelösten Metallkomplexverbindung eines Metalls der VIII. Nebengruppe des Periodensystems der Elemente als Katalysator und von freiem Liganden, in dem man als Katalysator Pnicogenchelatkomplexe und als freien Liganden Pnicogenchelatliganden der allgemeinen Formel I einsetzt.

Ein weiterer Gegenstand der Erfindung ist ein Verfahren zur Herstellung von 2-Propylheptanol, das die Hydroformylierung von Buten, eine Aldolkondensation der so erhaltenen Hydroformylierungsprodukte und die anschließende Hydrierung der Kondensationsprodukte umfasst, wobei als Hydroformylierungskatalysator ein Komplex eines Metalls der VIII. Nebengruppe mit wenigstens einer Pnicogenchelatverbindung der allgemeinen Formel I als Liganden eingesetzt wird.

Für den Zweck der Erläuterung der vorliegenden Erfindung umfasst der Ausdruck 'Alkyl' geradkettige und verzweigte Alkylgruppen. Vorzugsweise handelt es sich dabei um geradkettige oder verzweigte C₁-C₂₀-Alkyl, bevorzugterweise C₁-C₁₂-Alkyl-, besonders bevorzugt C₁-C₈-Alkyl- und ganz besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Ausdruck "Alkyl" umfasst auch substituierte Alkylgruppen, welche im allgemeinen 1, 2, 3, 4 oder 5, bevorzugt 1, 2 oder 3 und besonders bevorzugt 1 Substituenten, ausgewählt aus den Gruppen Cycloalkyl, Aryl, Hetaryl, Halogen, NE¹E², NE¹E²E³⁺, Carboxyl, Carboxylat, -SO₃H und Sulfonat, tragen können.

Der Ausdruck "Alkylen" im Sinne der vorliegenden Erfindung steht für geradkettige oder verzweigte Alkandiyl-Gruppen mit 1 bis 4 Kohlenstoffatomen.

Der Ausdruck "Cycloalkyl" umfasst im Sinne der vorliegenden Erfindung unsubstituierte als auch substituierte Cycloalkylgruppen, vorzugsweise C₅- bis C₇-Cycloalkylgruppen, wie Cyclopentyl, Cyclohexyl oder Cycloheptyl, die im Falle einer Substitution, im allgemeinen 1, 2, 3, 4 oder 5, bevorzugt 1, 2 oder 3 und besonders bevorzugt 1 Substituenten, ausgewählt aus den Gruppen Alkyl, Alkoxy und Halogen, tragen können.

Der Ausdruck "Heterocycloalkyl" im Sinne der vorliegenden Erfindung umfasst gesättigte, cycloaliphatische Gruppen mit im allgemeinen 4 bis 7, vorzugsweise 5 oder 6 Ringatomen, in denen 1 oder 2 der Ringkohlenstoffatome durch Heteroatome, ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, COOR^{f}, COO⁻M⁺ und NE¹E², bevorzugt Alkyl, tragen können. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

Der Ausdruck "Aryl" umfasst im Sinne der vorliegenden Erfindung unsubstituierte als auch substituierte Arylgruppen, und steht vorzugsweise für Phenyl, Tolyl, Xylyl, Mesityl, Naphthyl, Fluorenyl, Anthracenyl, Phenanthrenyl oder Naphthacenyl, besonders bevorzugt für Phenyl oder Naphthyl, wobei diese Arylgruppen im Falle einer Substitution im allgemeinen 1, 2, 3, 4 oder 5, vorzugsweise 1, 2 oder 3 und besonders bevorzugt 1 Substituenten, ausgewählt aus den Gruppen Alkyl, Alkoxy, Carboxyl, Carboxylat, Trifluormethyl, -SO₃H, Sulfonat, NE¹E², Alkylen-NE¹E², Nitro, Cyano oder Halogen, tragen können.

Der Ausdruck "Hetaryl" umfasst im Sinne der vorliegenden Erfindung unsubstituierte oder substituierte, heterocycloaromatische Gruppen, vorzugsweise die Gruppen Pyridyl, Chinolinyl, Acridinyl, Pyridazinyl, Pyrimidinyl, Pyrazinyl, sowie die Untergruppe der "Pyrrolgruppe", wobei diese heterocycloaromatischen Gruppen im Falle einer Substitution im allgemeinen 1, 2 oder 3 Substituenten, ausgewählt aus den Gruppen Alkyl, Alkoxy, Carboxyl, Carboxylat, -SO₃H, Sulfonat, NE¹E², Alkylen-NE¹E², Trifluormethyl oder Halogen, tragen können.

Der Ausdruck "Pyrrolgruppe" steht im Sinne der vorliegenden Erfindung für eine Reihe unsubstituierter oder substituierter, heterocycloaromatischer Gruppen, die strukturell vom Pyrrolgrundgerüst abgeleitet sind und ein pyrrolisches Stickstoffatom im Heterocyclus enthalten, das kovalent mit anderen Atomen, beispielsweise einem Pnicogenatom, verknüpft werden kann. Der Ausdruck "Pyrrolgruppe" umfasst somit die unsubstituierten oder substituierten Gruppen Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, die im Falle einer Substitution im allgemeinen 1, 2 oder 3, vorzugsweise 1 oder 2, besonders bevorzugt 1 Substituenten, ausgewählt aus den Gruppen Alkyl, Alkoxy, Acyl, Carboxyl, Carboxylat, -SO₃H, Sulfonat, NE¹E², Alkylen-NE¹E², Trifluormethyl oder Halogen, tragen können.

Dementsprechend umfasst der Ausdruck "Bispyrrolgruppe" im Sinne der vorliegenden Erfindung zweibindige Gruppen der Formel

Py-I-Py,

die zwei durch direkte chemische Bindung oder Alkylen-, Oxa-, Thio-, Imino-, Silyl oder Alkyliminogruppen vermittelte Verknüpfung, verbundene Pyrrolgruppen enthalten, wie die Bisindoldiyl-Gruppe der Formel 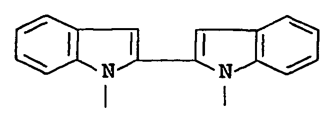 als Beispiel für eine Bispyrrolgruppe, die zwei direkt verknüpfte Pyrrolgruppen, in diesem Falle Indolyl, enthält, oder die Bis-pyrroldiyl-methan-Gruppe der Formel 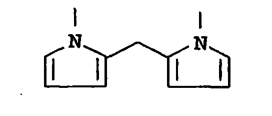 als Beispiel für eine Bispyrrolgruppe, die zwei über eine Methylengruppe verknüpfte Pyrrolgruppen, in diesem Falle Pyrrolyl, enthält. Wie die Pyrrolgruppen können auch die Bispyrrolgruppen unsubstituiert oder substituiert sein und im Falle einer Substitution pro Pyrrolgruppeneinheit im Allgemeinen 1, 2 oder 3, vorzugsweise 1 oder 2, insbesondere 1 Substituenten, ausgewählt aus Alkyl, Alkoxy, Carboxyl, Carboxylat, -SO₃H, Sulfonat, NE¹E², Alkylen-NE¹E², Trifluormethyl oder Halogen, tragen, wobei bei diesen Angaben zur Anzahl möglicher Substituenten die Verknüpfung der Pyrrolgruppeneinheiten durch direkte chemische Bindung oder durch die mittels der vorstehend genannten Gruppen vermittelte Verknüpfung nicht als Substitution betrachtet wird.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäure- oder Sulfonsäureesterfunktion oder eine Carbonsäure- oder Sulfonsäureamidfunktion. Dazu zählen z. B. die Ester mit C₁-C₄-Alkanolen, wie Methanol, Ethanol, n-Propanol, Isopropanol, n-Butanol, sec.-Butanol und tert.-Butanol.

Die obigen Erläuterungen zu den Ausdrücken "Alkyl", "Cycloalkyl", "Aryl", "Heterocycloalkyl" und "Hetaryl" gelten entsprechend für die Ausdrücke "Alkoxy", "Cycloalkoxy", "Aryloxy", "Heterocycloalkoxy" und "Hetaryloxy".

Der Ausdruck "Acyl" steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propionyl-, Butyryl-, Pentanoyl-, Hexanoyl-, Heptanoyl-, 2-Ethylhexanoyl-, 2-Propylheptanoyl-, Benzoyl- oder Naphthoyl-Gruppe.

Die Gruppen NE¹E² und NE⁴E⁵ stehen vorzugsweise für N,N-Dimethylamino, N,N-Diethylamino, N,N-Dipropylamino, N,N-Diisopropylamino, N,N-Di-n-butylamino, N,N-Di-t.-butylamino, N,N-Dicyclohexylamino oder N,N-Diphenylamino.

Halogen steht für Fluor, Chlor, Brom und Iod, bevorzugt für Fluor, Chlor und Brom.

M⁺ steht für ein Kationäquivalent, d. h. für ein einwertiges Kation oder den einer positiven Einfachladung entsprechenden Anteil eines mehrwertigen Kations. Das Kation M⁺ dient lediglich als Gegenion zur Neutralisation negativ geladener Substituentengruppen, wie der COO⁻ oder der Sulfonat-Gruppe und kann im Prinzip beliebig gewählt werden. Vorzugsweise werden deshalb Alkalimetall-, insbesondere Na⁺, K⁺-, Li⁺-Ionen oder Onium-Ionen, wie Ammonium-, Mono-, Di-, Tri-, Tetra-Alkyl-ammonium-, Phosphonium-, Tetra-Alkyl-phosphonium oder Tetra-Aryl-phosphonium-Ionen verwendet.

Entsprechendes gilt für das Anionäquivalent X⁻, das lediglich als Gegenion positiv geladener Substituentengruppen, wie den Ammoniumgruppen, dient und beliebig gewählt werden kann unter einwertigen Anionen und den einer negativen Einfachladung entsprechenden Anteilen eines mehrwertigen Anions, wobei im Allgemeinen Halogenid-Ionen X⁻ bevorzugt sind, insbesondere Chlorid und Bromid.

Die Werte für x stehen für eine ganze Zahl von 1 bis 240, vorzugsweise für eine ganze Zahl von 3 bis 120.

Kondensierte Ringsysteme können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante, bzw. zwei Atome gemeinsam, und einer peri-Anellierung, bei der ein Kohlenstoffatom mehr als zwei Ringen angehört. Bevorzugt unter den kondensierten Ringsystemen sind ortho-kondensierte Ringsysteme.

Y stellt eine chemische Bindung, also den Anknüpfungspunkt der Brückengruppe Q an die Gruppen -O-, oder im Falle wenn a und/oder b gleich 0 ist, an die Gruppen PnR¹R² bzw. PnR³R⁴ dar.

In der Brückengruppe Q können die Gruppen A¹ und A² im Allgemeinen unabhängig voneinander für O, S, SiR^{a}R^{b}, NR^{c} oder CR^{d}R^{e} stehen, wobei die Substituenten R^{a}, R^{b} und R^{c} im Allgemeinen unabhängig voneinander die Bedeutung Wasserstoff, Alkyl, Cycloalkyl, Heterocycloalkyl, Aryl oder Hetaryl haben können, wohingegen die Gruppen R^{d} und R^{e} unabhängig voneinander für Wasserstoff, Alkyl, Cycloalkyl, Heterocycloalkyl, Aryl oder Hetaryl stehen oder die Gruppe R^{d} gemeinsam mit einer weiteren Gruppe R^{d} oder die Gruppe R^{e} gemeinsam mit einer weiteren Gruppe R^{e} eine intramolekulare Brückengruppe D bilden können.

D ist eine zweibindige Brückengruppe, die im Allgemeinen ausgewählt ist aus den Gruppen 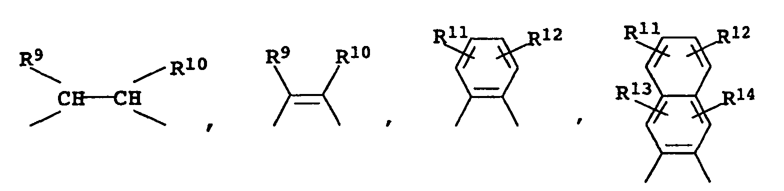 in denen R⁹ und R¹⁰ unabhängig voneinander für Wasserstoff, Alkyl, Cycloalkyl, Aryl, Halogen, Trifluormethyl, Carboxyl, Carboxylat oder Cyano stehen oder miteinander zu einer C₃-C₄-Alkylengruppe verbunden sind und R¹¹, R¹², R¹³ und R¹⁴ unabhängig voneinander für Wasserstoff, Alkyl, Cycloalkyl, Aryl, Halogen, Trifluormethyl, COOH, Carboxylat, Cyano, Alkoxy, SO₃H, Sulfonat, NE¹E², Alkylen-NE¹E²E³⁺X⁻, Aryl oder Nitro stehen können. Vorzugsweise stehen die Gruppen R⁹ und R¹⁰ für Wasserstoff, C₁-C₁₀-Alkyl oder Carboxylat und die Gruppen R¹¹, R¹², R¹³ und R¹⁴ für Wasserstoff, C₁-C₁₀-Alkyl, Halogen, insbesondere Fluor, Chlor oder Brom, Trifluormethyl, C₁-C₄-Alkoxy, Carboxylat, Sulfonat oder C₁-C₈-Aryl. Besonders bevorzugt stehen R⁹, R¹⁰, R¹¹, R¹², R¹³ und R¹⁴ für Wasserstoff. Für den Einsatz in einem wässrigen Reaktionsmedium sind solche Pnicogenchelatverbindungen bevorzugt, in denen 1, 2 oder 3, vorzugsweise 1 oder 2, insbesondere 1 der Gruppen R¹¹, R¹², R¹³ und/oder R¹⁴ für eine COO⁻Me⁺, eine SO₃⁻M⁺ oder eine NE¹E²E³⁺X⁻-Gruppe stehen, wobei M⁺ und X⁻ die vorstehend genannte Bedeutung haben.

Besonders bevorzugte Brückengruppen D sind die Ethylengruppe 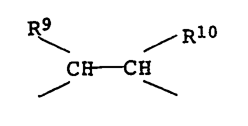 und die 1,2-Phenylengruppe

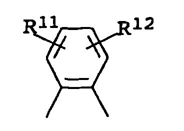

Wenn R^{d} mit einer weiteren Gruppe R^{d} oder R^{e} mit einer weiteren Gruppe R^{e} eine intramolekulare Brückengruppe D bildet, d. h. der Index c ist in diesem Falle gleich 1, ergibt es sich zwangsläufig, dass sowohl A¹ als auch A² eine CR^{d}R^{e}-Gruppe sind und die Brückengruppe Q in diesem Falle ein Triptycen-artiges Kohlenstoffgerüst hat.

Bevorzugte Brückengruppen Q sind außer denen mit Triptycen-artigem Kohlenstoffgerüst solche, in denen der Index c für 0 steht und die Gruppen A¹ und A² ausgewählt sind aus den Gruppen O, S und CR^{d}R^{e}, insbesondere unter O, S, der Methylengruppe (R^{d} = R^{e} = H), der Dimethylmethylengruppe (R^{d} = R^{e} = CH₃), der Di-n-propyl-methylengruppe (R^{d} = R^{e} = n-Propyl) oder der Di-n-butylmethylengruppe (R^{d} = R^{e} = n-Butyl). Insbesondere sind solche Brückengruppen Q bevorzugt, in denen A¹ von A² verschieden ist, wobei A¹ bevorzugt eine CR^{d}R^{e}-Gruppe und A² bevorzugt eine O- oder S-Gruppe, besonders bevorzugt eine Oxagruppe O ist.

Besonders bevorzugte Brückengruppen Q sind somit solche, die aus einem Triptycen-artigen oder Xanthen-artigen (A¹:CR^{d}R^{e}, A²:O) Gerüst aufgebaut sind.

Die Substituenten R⁵, R⁶, R⁷ und R⁸ stehen im Allgemeinen für Wasserstoff, Alkyl, Cycloalkyl, Heterocycloalkyl, Aryl und Hetaryl. Bevorzugt stehen R⁵ und R⁷ für Wasserstoff und R⁶ und R⁸ für C₁-C₄-Alkyl, wie z. B. Methyl, Ethyl, n-Propyl, n-Butyl oder tert.-Butyl. Es versteht sich von selbst, dass die nicht mit Substituenten besetzten Positionen der Phenylringe der Brückengruppe Q ein Wasserstoffatom tragen.

Da die Substituenten R⁵, R⁶, R⁷ und R⁸ in der Regel, außer einem Einfluss auf die Löslichkeit, praktisch keinen Beitrag zur katalytischen Aktivität der aus den erfindungsgemäßen Pnicogenchelatliganden hergestellten Katalysatoren leisten, stehen R⁵, R⁶, R⁷ und R⁸ bevorzugt für Wasserstoff.

Wenn R⁵ und/oder R⁷ für ein ankondensiertes, also anelliertes, Ringsystem stehen, so handelt es sich bevorzugt um Benzol- oder Naphthalinringe. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, SO₃H, Sulfonat, NE¹E², Alkylen-NE¹E², Trifluormethyl, Nitro, COOR^{f}, Alkoxycarbonyl, Acyl und Cyano. Anellierte Naphthalinringe sind vorzugsweise unsubstituiert oder weisen im nicht anellierten Ring und/oder im anellierten Ring insgesamt 1, 2 oder 3, insbesondere 1 oder 2 der zuvor bei den anellierten Benzolringen genannten Substituenten auf.

Ist der Einsatz der erfindungsgemäßen Pnicogenchelatverbindungen in einem wässrigen Hydroformylierungsmedium vorgesehen, steht wenigstens einer der Reste R⁵, R⁶, R⁷ und/oder R⁸ für eine polare (hydrophile) Gruppe, wobei dann in der Regel bei der Komplexbildung mit einem Gruppe VIII Metall wasserlösliche Pnicogenchelatkomplexe resultieren. Bevorzugt sind die polaren Gruppen ausgewählt unter COOR^{f}, COO⁻M⁺, SO₃R^{f}, SO₃⁻M⁺, NE¹E², Alkylen-NE¹E², NE¹E²E³⁺X⁻, Alkylen-NE¹E²E³⁺X⁻, OR^{f}, SR^{f}, (CHR^{g}CH₂O)ₓR^{f} oder (CH₂CH₂N(E¹))ₓR^{f}, worin R^{f}, E¹, E², E³, R^{g}, M⁺, X⁻ und x die zuvor angegebenen Bedeutungen besitzen.

Die Brückengruppe Q ist über die chemische Bindung Y entweder direkt oder über eine Oxagruppe O mit den Gruppen PnR¹R² bzw. PnR³R⁴ verbunden.

Pn steht für ein Atom aus der Pnicogengruppe, ausgewählt aus Phosphor, Arsen oder Antimon. Besonders bevorzugt steht Pn für Phosphor.

Die einzelnen Pnicogenatome Pn der erfindungsgemäßen Pnicogenchelatverbindungen sind jeweils über zwei kovalente Bindungen mit zwei Substituenten R¹ und R² bzw. R³ und R⁴ verbunden, wobei die Substituenten R¹, R², R³ und R⁴ unabhängig voneinander für Hetaryl, Hetaryloxy, Alkyl, Alkoxy, Aryl, Aryloxy, Cycloalkyl, Cycloalkoxy, Heterocycloalkyl, Heterocycloalkoxy oder eine NE¹E²-Gruppe stehen können, mit der Maßgabe, dass R¹ und R³ über das pyrrolische Stickstoffatom an das Pnicogenatom Pn gebundene Pyrrolgruppen sind. Vorteilhaft stehen auch die Substituenten R² und/oder R⁴ für über das pyrrolische Stickstoffatom an das Pnicogenatom Pn gebundene Pyrrolgruppen. Weiterhin vorteilhaft kann der Substituent R¹ gemeinsam mit dem Substituenten R² oder der Substituent R³ gemeinsam mit dem Substituenten R⁴ oder der Substituent R¹ gemeinsam mit dem Substituenten R² und der Substituent R³ gemeinsam mit dem Substituenten R⁴ eine über die pyrrolischen Stickstoffatome an das Pnicogenatom Pn gebundene Bispyrrolgruppe bilden.

Die Bedeutung der einzelnen im vorstehenden Absatz genannten Ausdrücke entspricht der eingangs gegebenen Definition.

In einer weiteren vorteilhaften Ausgestaltung der vorliegenden Erfindung kann der Substituent R¹ gemeinsam mit dem Substituenten R² oder der Substituent R³ gemeinsam mit dem Substituenten R⁴ oder der Substituent R¹ gemeinsam mit dem Substituenten R² und der Substituent R³ gemeinsam mit dem Substituenten R⁴ eine über das pyrrolische Stickstoffatom an das Pnicogenatom Pn gebundene Pyrrolgruppe enthaltende zweibindige Gruppe der Formel

Py-I-W

bilden,
worin
- Py: eine Pyrrolgruppe ist,
- I: für eine chemische Bindung oder für O, S, SiR^{a}R^{b}, NR^{c} oder CR^{h}Rⁱ steht,
- W: für Cycloalkyl, Cycloalkoxy, Aryl, Aryloxy, Hetaryl oder Hetaryloxy steht
und
- R^{h} und Rⁱ: unabhängig voneinander für Wasserstoff, Alkyl, Cycloalkyl, Heterocycloalkyl, Aryl oder Hetaryl stehen,
wobei die hierbei verwendeten Bezeichnungen die eingangs erläuterte Bedeutung haben.

Bevorzugte zweibindige Gruppen der Formel

Py-I-W

sind z. B.

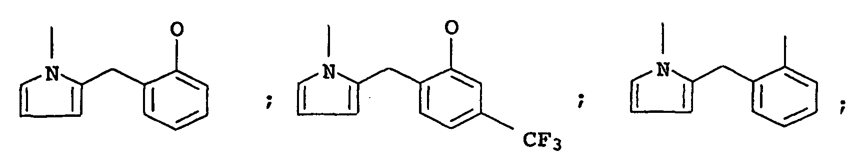

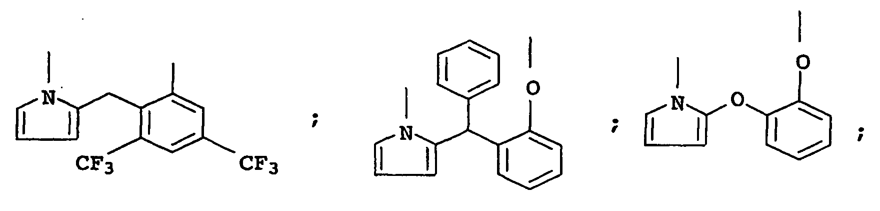

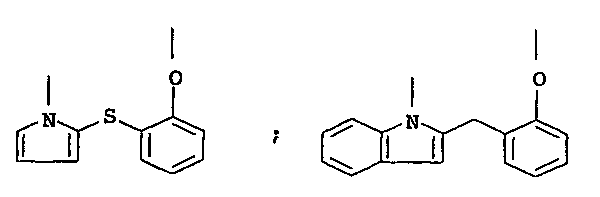

Lediglich zur Veranschaulichung der erfindungsgemäßen Pnicogenchelatverbindung werden im folgenden einige vorteilhafte Verbindungen aufgelistet: 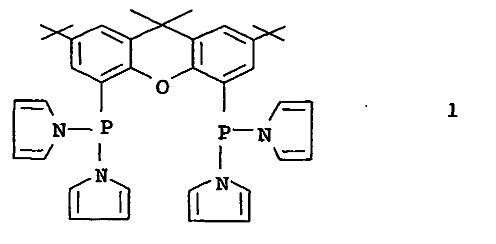 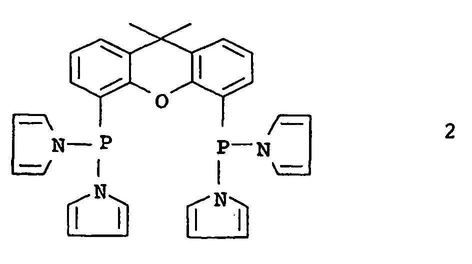 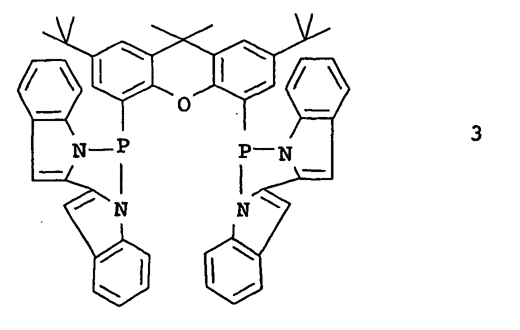 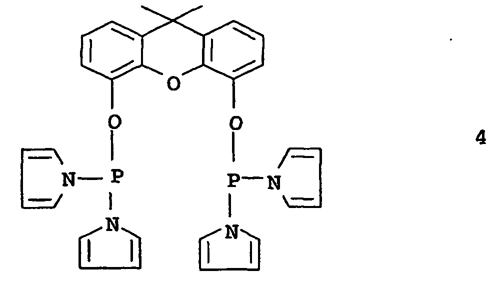 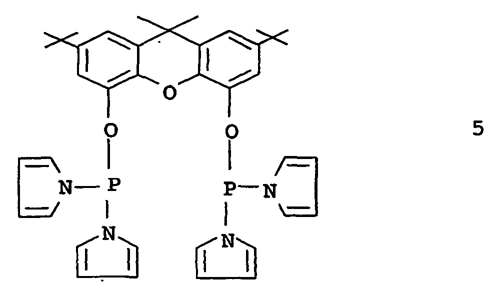 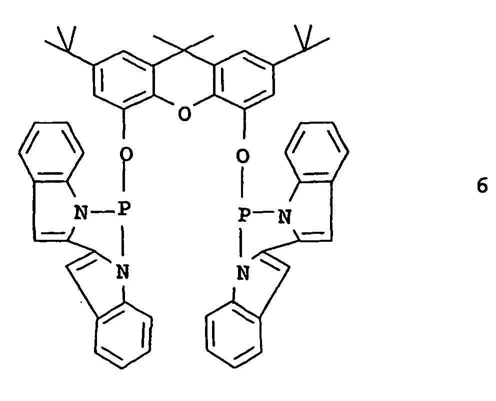 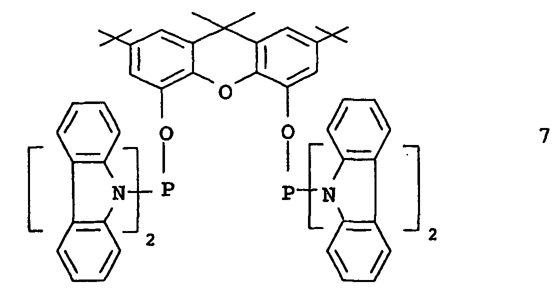 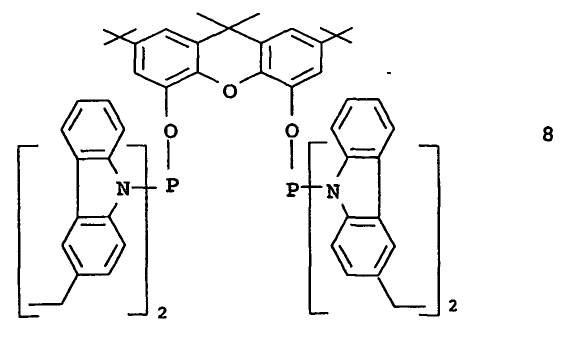 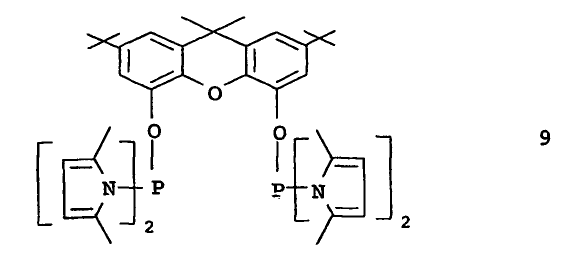 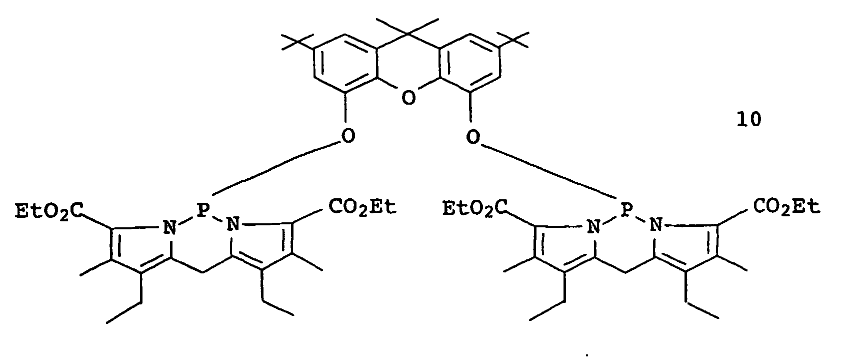 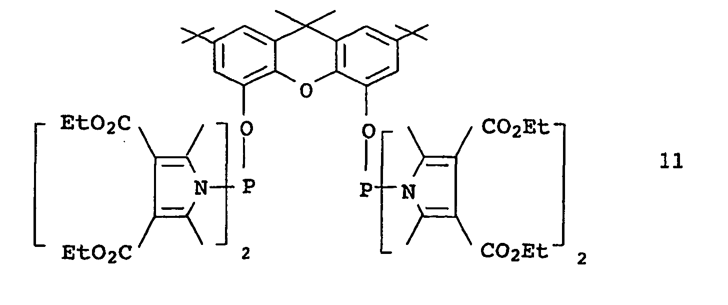 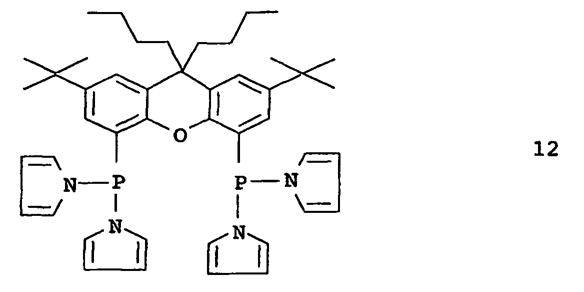 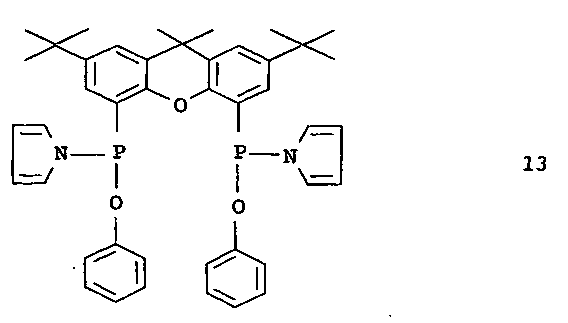 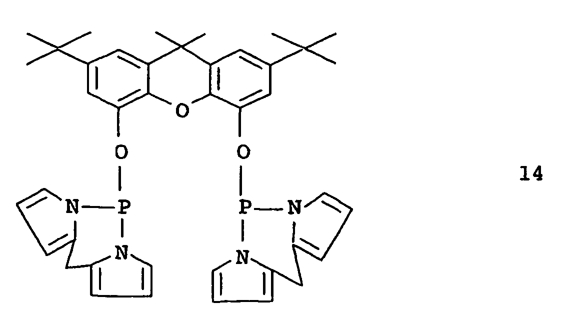 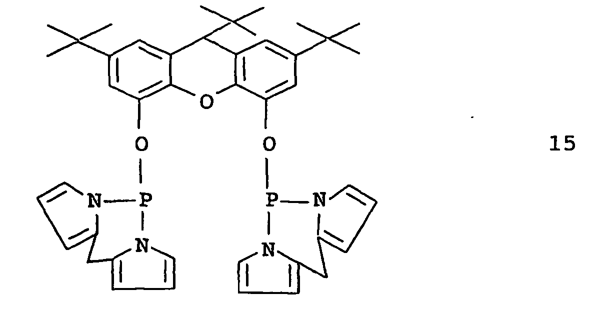 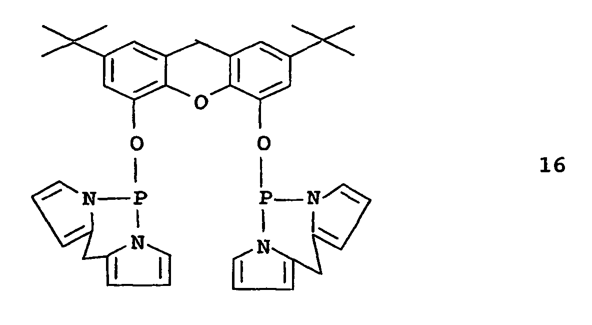 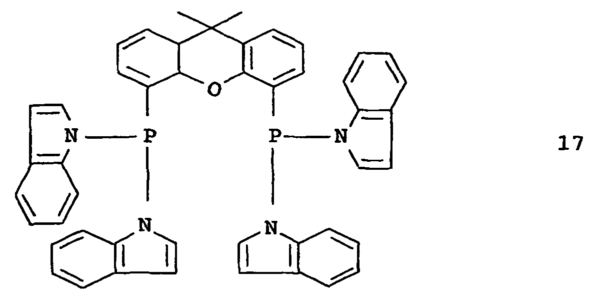 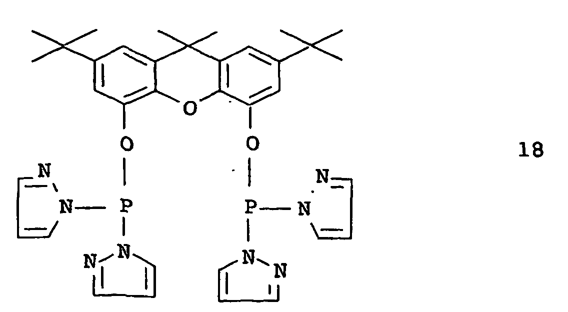 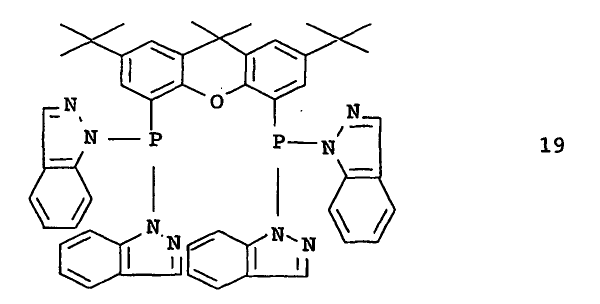 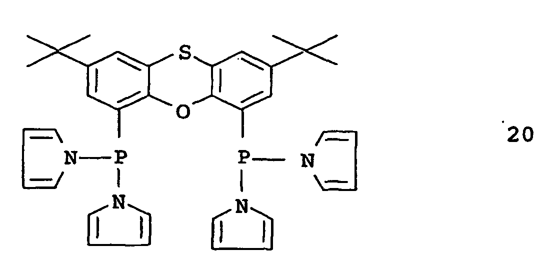 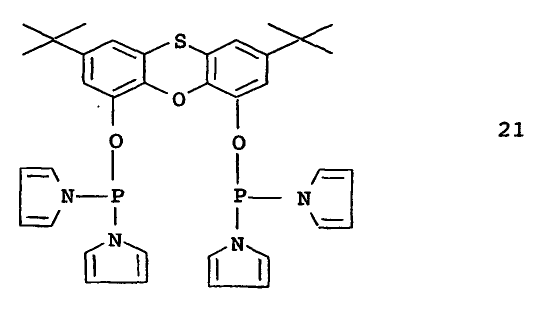 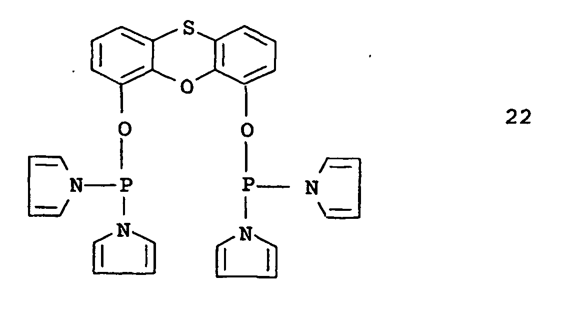 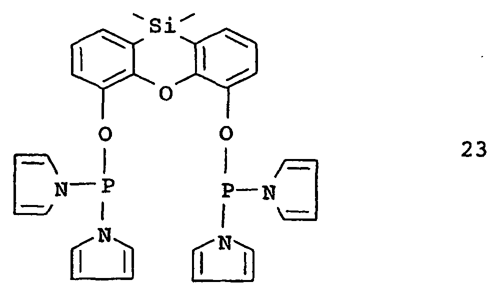 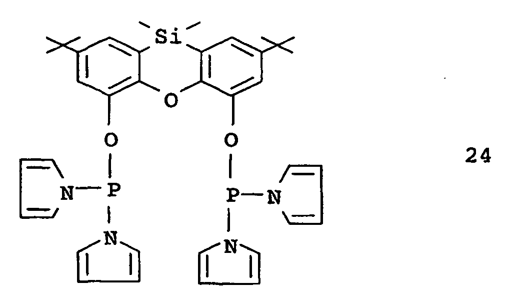 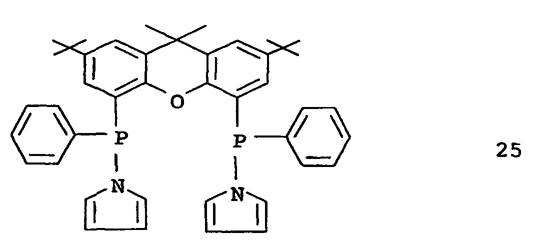 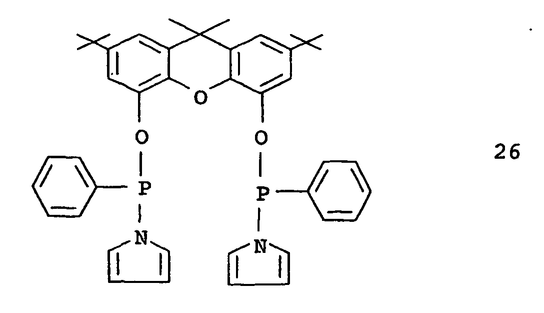 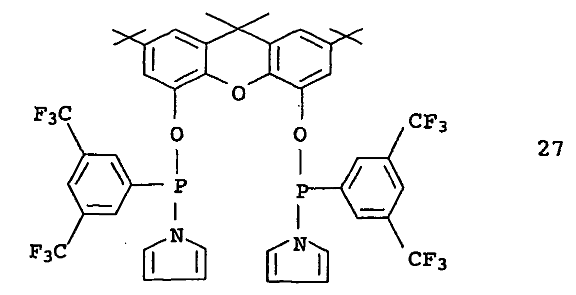 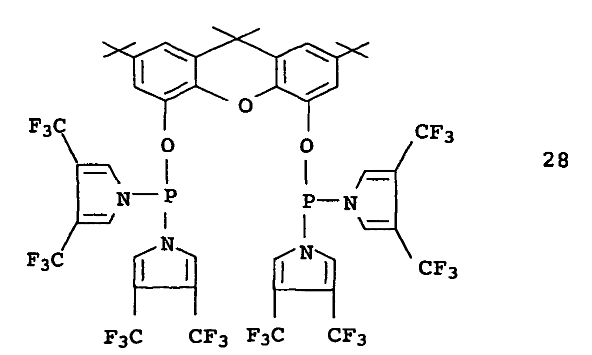 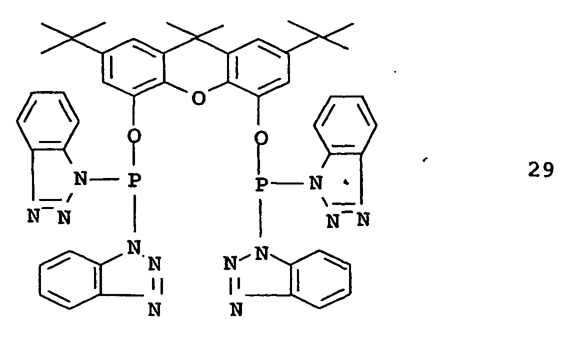 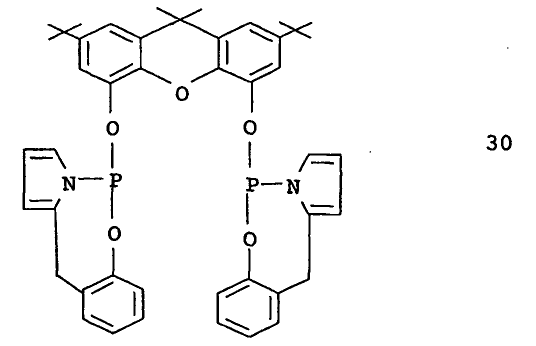 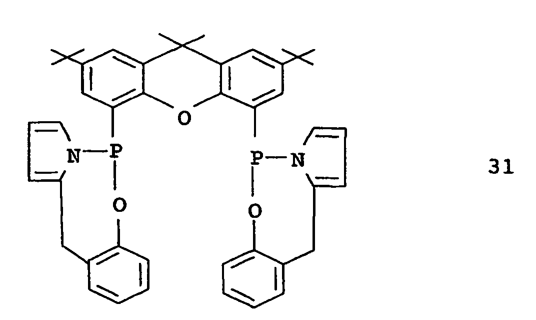 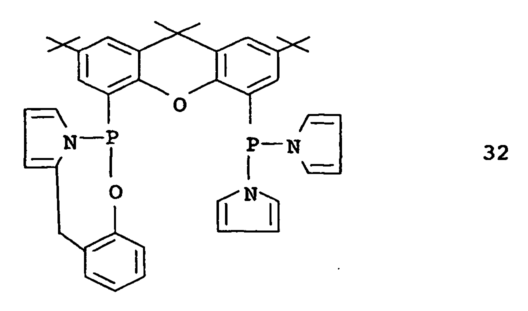 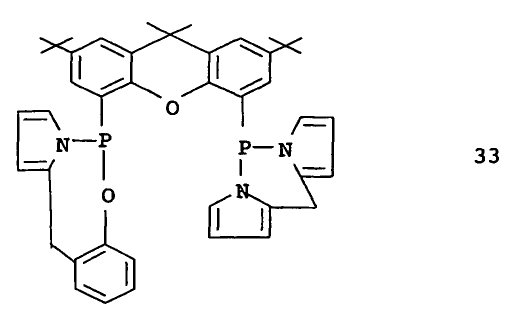 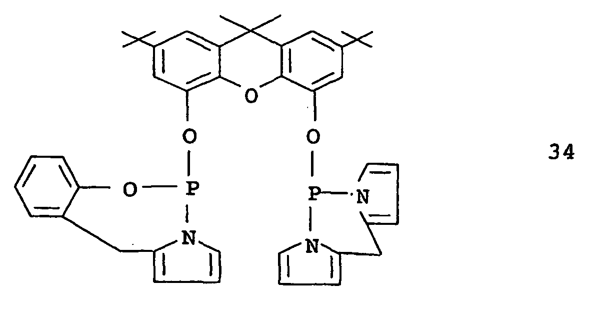 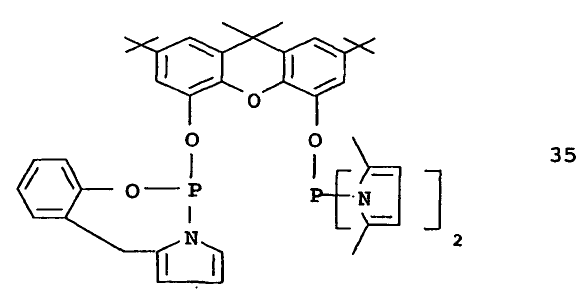 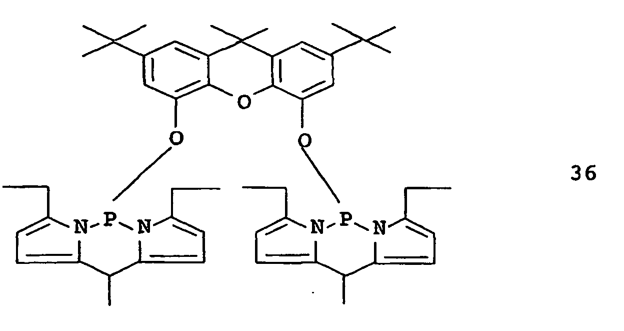 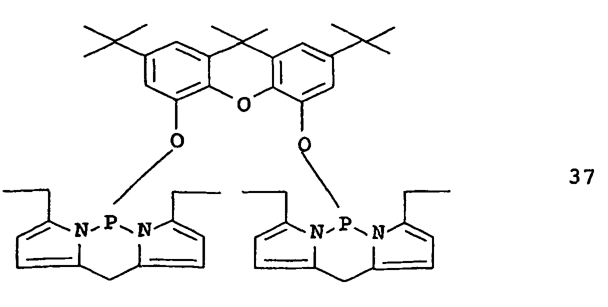 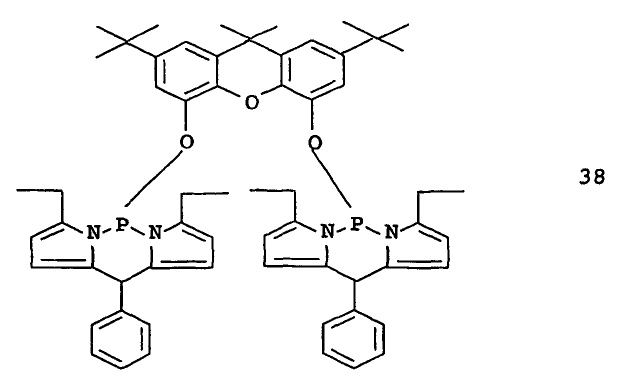 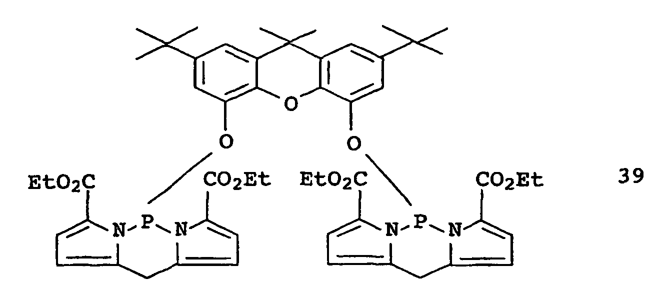 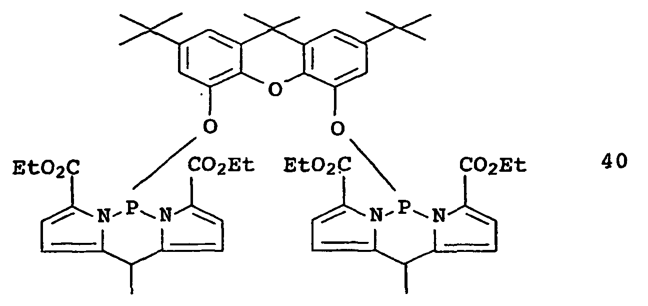 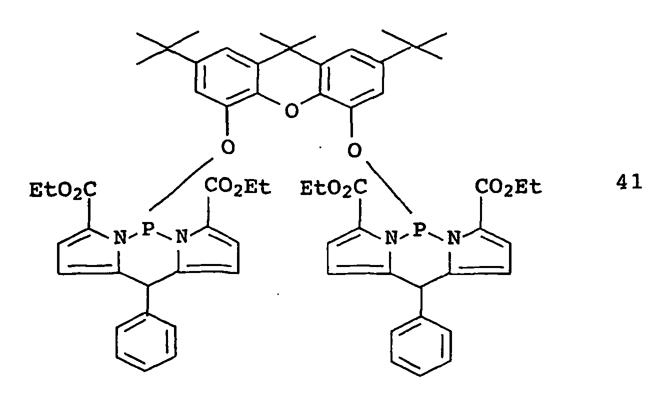 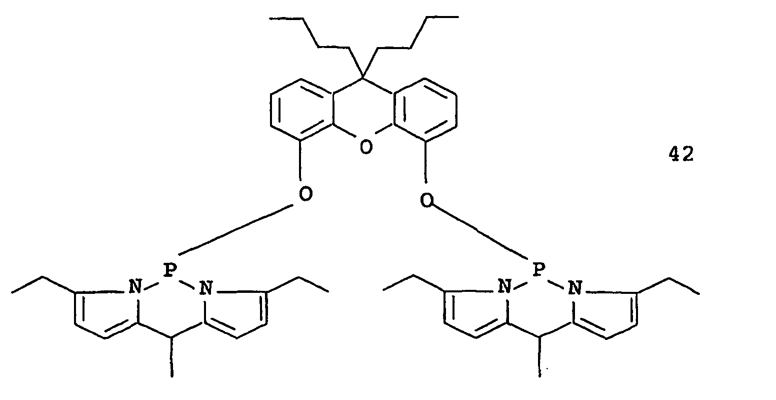 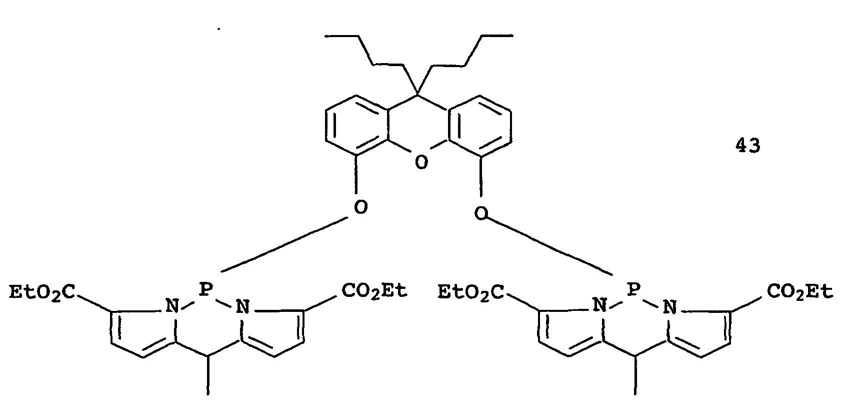 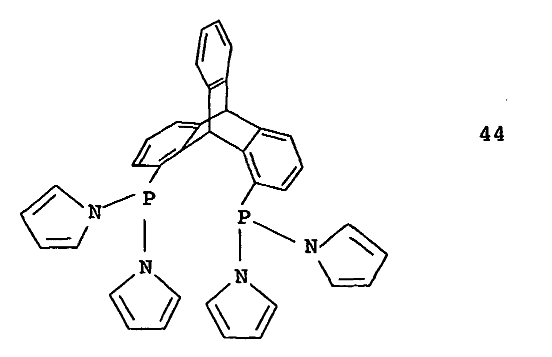 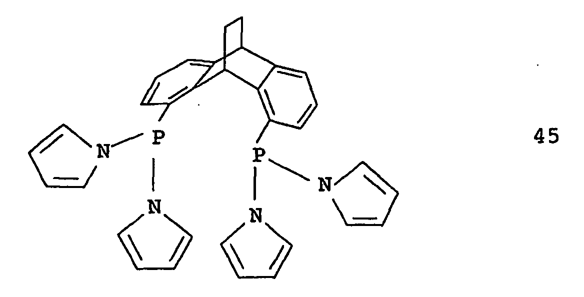 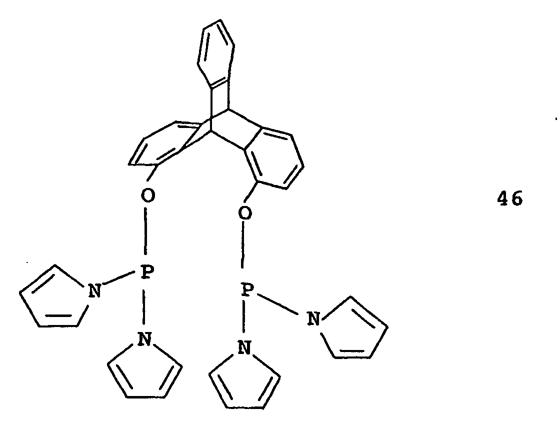 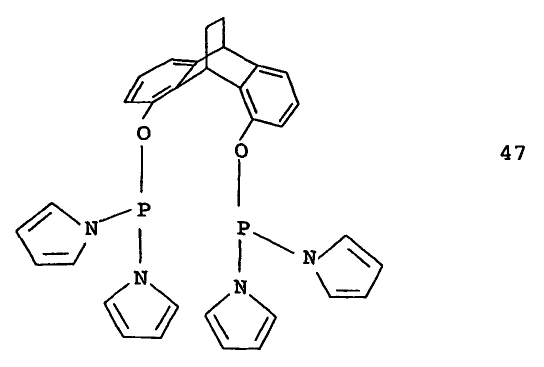 Et : Ethyl

In einer bevorzugten Ausführungsform sind die erfindungsgemäßen und erfindungsgemäß eingesetzten Pnicogenchelatverbindungen ausgewählt unter Verbindungen der allgemeinen Formel II 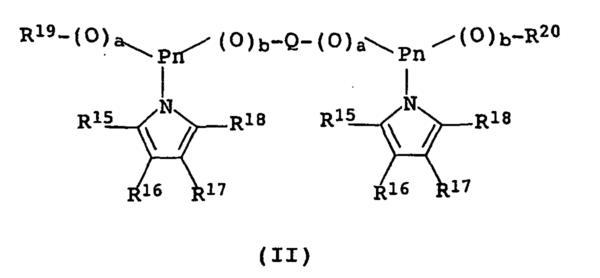 worin
- R¹⁵, R¹⁶, R¹⁷ und R¹⁸: unabhängig voneinander für Wasserstoff, Alkyl, Cycloalkyl, Heterocycloalkyl, Aryl, Hetaryl, W'COOR^{k}, W'COO⁻M⁺, W'(SO₃)R^{k}, W'(SO₃)⁻M⁺, W'PO₃(R^{k})(R¹), W'(PO₃)²⁻(M⁺)₂, W'NE⁴E⁵, W'(NE⁴E⁵E⁶)⁺X⁻, W'OR^{k}, W'SR^{k}, (CHR¹CH₂O)_{y}R^{k}, (CH₂NE⁴)_{y}R^{k}, (CH₂CH₂NE⁴)_{y}R^{k}, Halogen, Trifluormethyl, Nitro, Acyl oder Cyano stehen,
worin
W' für eine Einfachbindung, ein Heteroatom oder eine zweiwertige verbrückende Gruppe mit 1 bis 20 Brückenatomen steht
R^{k}, E⁴, E⁵, E⁶ jeweils gleiche oder verschiedene Reste, ausgewählt unter Wasserstoff, Alkyl, Cycloalkyl oder Aryl bedeuten,
R¹ für Wasserstoff, Methyl oder Ethyl steht,
M⁺ für ein Kationäquivalent steht,
X⁻ für ein Anionäquivalent steht und
y für eine ganze Zahl von 1 bis 240 steht,
wobei jeweils zwei benachbarte Reste R¹⁵, R¹⁶, R¹⁷ und R¹⁸ zusammen mit den Kohlenstoffatomen des Pyrrolrings, an die sie gebunden sind, auch für ein kondensiertes Ringsystem mit 1, 2 oder 3 weiteren Ringen stehen können,
mit der Maßgabe, dass wenigstens einer der Reste R¹⁵, R¹⁶, R¹⁷ oder R¹⁸ nicht für Wasserstoff steht, und dass R¹⁹ und R²⁰ nicht mit einander verknüpft sind,
- R¹⁹ und R²⁰: unabhängig voneinander für Cycloalkyl, Heterocycloalkyl, Aryl oder Hetaryl stehen,
- a und b: unabhängig voneinander die Zahl 0 oder 1 bedeuten,
- Pn: für ein Pnicogenatom, ausgewählt aus den Elementen Phosphor, Arsen oder Antimon, bevorzugt für Phosphor, steht,
- Q: eine Brückengruppe der Formel 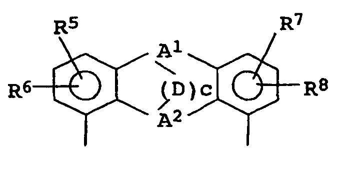
ist,
worin
- A¹ und A²: unabhängig voneinander für O, S, SiR^{a}R^{b}, NR^{c} oder CR^{d}R^{e} stehen, wobei
- R^{a},R^{b} und R^{c}: unabhängig voneinander für Wasserstoff, Alkyl, Cycloalkyl, Heterocycloalkyl, Aryl oder Hetaryl stehen,
- R^{d} und R^{e}: unabhängig voneinander für Wasserstoff, Alkyl, Cycloalkyl, Heterocycloalkyl, Aryl oder Hetaryl stehen oder die Gruppe R^{d} gemeinsam mit einer weiteren Gruppe R^{d} oder die Gruppe R^{e} gemeinsam mit einer weiteren Gruppe R^{e} eine intramolekulare Brückengruppe D bilden,
- D: eine zweibindige Brückengruppe, ausgewählt aus den Gruppen 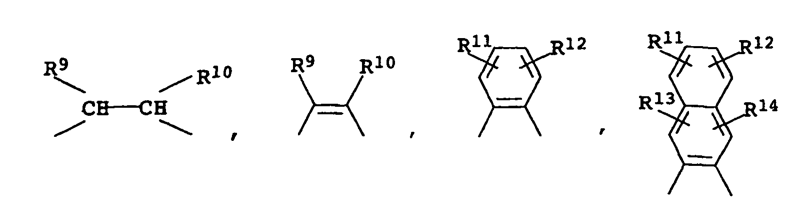
ist, in denen
- R⁹ und R¹⁰: unabhängig voneinander für Wasserstoff, Alkyl, Cycloalkyl, Aryl, Halogen, Trifluormethyl, Carboxyl, Carboxylat oder Cyano stehen oder miteinander zu einer C₃- bis C₄-Alkylenbrücke verbunden sind,
- R¹¹,R¹²,R¹³ und R¹⁴: unabhängig voneinander für Wasserstoff, Alkyl, Cycloalkyl, Aryl, Halogen, Trifluormethyl, COOH, Carboxylat, Cyano, Alkoxy, SO₃H, Sulfonat, NE¹E², Alkylen-NE¹E²E³⁺X⁻, Acyl oder Nitro stehen,
- c: 0 oder 1 ist,
- R⁵, R⁶, R⁷ und R⁸: unabhängig voneinander für Wasserstoff, Alkyl, Cycloalkyl, Heterocycloalkyl, Aryl, Hetaryl, COOR^{f}, COO⁻M⁺, SO₃R^{f}, SO⁻₃M⁺, NE¹E², NE¹E²E³⁺X⁻, Alkylen-NE¹E²E³⁺X⁻, OR^{f}, SR^{f}, (CHR^{g}CH₂O)ₓR^{f}, (CH₂N(E¹))ₓR^{f}, (CH₂CH₂N(E¹))ₓR^{f}, Halogen, Trifluormethyl, Nitro, Acyl oder Cyano stehen,
worin
- R^{f}, E¹, E² und E³: jeweils gleiche oder verschiedene Reste, ausgewählt unter Wasserstoff, Alkyl, Cycloalkyl oder Aryl bedeuten,
- R^{g}: für Wasserstoff, Methyl oder Ethyl steht,
- M⁺: für ein Kation steht,
- X⁻: für ein Anion steht, und
- x: für eine ganze Zahl von 1 bis 120 steht,
oder
- R⁵ und/oder R⁷: zusammen mit zwei benachbarten Kohlenstoffatomen des Benzolkerns, an den sie gebunden sind, für ein kondensiertes Ringsystem, mit 1, 2 oder 3 weiteren Ringen stehen.

Bevorzugt stehen in den Verbindungen der Formel II die Pnicogenatome Pn beide für Phosphor.

Bezüglich geeigneter und bevorzugter Ausführungsformen der Brückengruppe Q wird auf die vorherigen Ausführungen in vollem Umfang Bezug genommen.

Die Reste R¹⁵ bis R¹⁸ können jeweils unabhängig voneinander gleiche oder verschiedene Bedeutungen aufweisen.

Bevorzugt sind Verbindungen der allgemeinen Formel II, wobei in den Pyrrolgruppen jeweils einer oder zwei der Reste R¹⁵, R¹⁶, R¹⁷ und R¹⁸ für einen der zuvor genannten, von Wasserstoff verschiedenen Substituenten stehen und die übrigen für Wasserstoff stehen. Bevorzugt sind Verbindungen der Formel II, bei denen die Pyrrolgruppen in 2-Position, 2,5-Position oder-3,4-Position einen von Wasserstoff verschiedenen Substituenten tragen.

Vorzugsweise sind die von Wasserstoff verschiedenen Substituenten R¹⁵ bis R¹⁸ unabhängig voneinander ausgewählt unter C₁- bis C₈-, vorzugsweise C₁- bis C₄-Alkyl, speziell Methyl, Ethyl, Isopropyl und tert.-Butyl, Alkoxycarbonyl, wie Methoxycarbonyl, Ethoxycarbonyl, Isopropyloxycarbonyl und tert.-Butyloxycarbonyl sowie Trifluormethyl.

Bevorzugt sind Verbindungen der allgemeinen Formel II, worin die Reste R¹⁵ und R¹⁶ und/oder R¹⁷ und R¹⁸ zusammen mit den Kohlenstoffatomen des Pyrrolrings, an die sie gebunden sind, für ein kondensiertes Ringsystem mit 1, 2 oder 3 weiteren Ringen stehen. Wenn R¹⁵ und R¹⁶ und/oder R¹⁷ und R¹⁸ für ein ankondensiertes, also anelliertes Ringsystem stehen, so handelt es sich bevorzugt um Benzol- oder Naphthalinringe. Anellierte Benzolringe sind vorzugsweise unsubstituiert und weisen 1, 2 oder 3, insbesondere 1 oder 2 Substituenten auf, die ausgewählt sind unter Alkyl, Alkoxy, Halogen, SO₃H, Sulfonat, NE⁴E⁵, Alkylen-NE⁴E⁵, Trifluormethyl, Nitro, COOR^{k}, Alkoxycarbonyl, Acyl und Cyano. Anellierte Naphthalinringe sind vorzugsweise unsubstituiert oder weisen im nichtanellierten Ring und/oder im anellierten Ring je 1, 2 oder 3, insbesondere 1 oder 2 der zuvor bei den anellierten Benzolringen genannten Substituenten auf. Wenn R¹⁵ und R¹⁶ für ein ankondensiertes Ringsystem stehen, so stehen R¹⁷ und R¹⁸ vorzugsweise für Wasserstoff oder steht R¹⁸ für Wasserstoff und R¹⁷ für einen Substituenten, der ausgewählt ist unter C₁- bis C₈-Alkyl, vorzugsweise C₁- bis C₄-Alkyl, speziell Methyl, Ethyl, Isopropyl oder tert.-Butyl.

Ist der Einsatz der Verbindungen der Formel II in einem wässrigen Hydroformylierungsmedium vorgesehen, steht wenigstens einer der Reste R¹⁵, R¹⁶, R¹⁷ und/oder R¹⁸ für eine polare (hydrophile) Gruppe, wobei dann in der Regel bei der Komplexbildung mit einem Gruppe VIII Metall wasserlösliche Komplexe resultieren. Bevorzugt sind die polaren Gruppen ausgewählt unter COOR^{k}, COO⁻M⁺, SO₃R^{k}, SO₃⁻M⁺, NE⁴E⁵, Alkylen-NE⁴E⁵, NE⁴E⁵E⁶⁺X⁻, Alkylen-NE⁴E⁵E⁶⁺X⁻, OR^{k}, SR^{k}, (CHR¹CH₂O)_{y}R^{k} oder (CH₂CH₂N(E⁴))_{y}R^{k}, worin R^{k}, E⁴, E⁵, E⁶, R¹, M⁺, X⁻ und y die zuvor angegebenen Bedeutungen besitzen.

Vorzugsweise sind die Verbindungen der Formel II ausgewählt unter Verbindungen der allgemeinen Formeln II.1 bis II.3 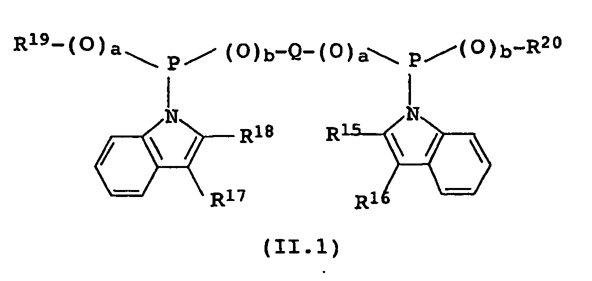 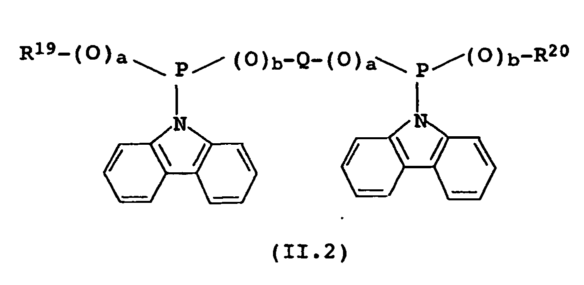 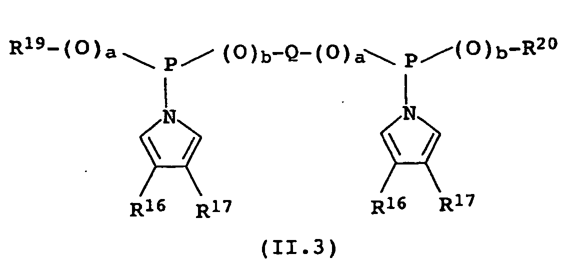 worin
- R¹⁵, R¹⁶, R¹⁷, R¹⁸, Q, a und b: die zuvor angegebenen Bedeutungen besitzen, wobei in der Formel II.3 wenigstens einer der Reste R¹⁶ oder R¹⁷ nicht für Wasserstoff steht,
- R¹⁹ und R²⁰: unabhängig voneinander für Cycloalkyl, Heterocycloalkyl, Aryl oder Hetaryl stehen.

Vorzugsweise stehen in den Verbindungen der Formel II.1 die Reste R¹⁵ bis R¹⁸ alle für Wasserstoff. Des Weiteren vorzugsweise stehen R¹⁵ und R¹⁸ für Wasserstoff und sind R¹⁶ und R¹⁷ ausgewählt unter C₁-C₈-Alkyl, vorzugsweise C₁-C₄-Alkyl, wie Methyl, Ethyl, Isopropyl und tert.-Butyl.

Vorzugsweise sind in den Verbindungen der Formel II.3 die Reste R¹⁶ und R¹⁷ ausgewählt unter C₁-C₈-Alkyl, besonders bevorzugt C₁-C₄-Alkyl, wie Methyl, Ethyl, Isopropyl und tert.-Butyl, sowie COOR^{k}, worin R^{k} für C₁-C₄-Alkyl, wie Methyl, Ethyl, Isopropyl und tert.-Butyl steht.

Lediglich zur Veranschaulichung werden im Folgenden einige Verbindungen der Formel II aufgelistet: 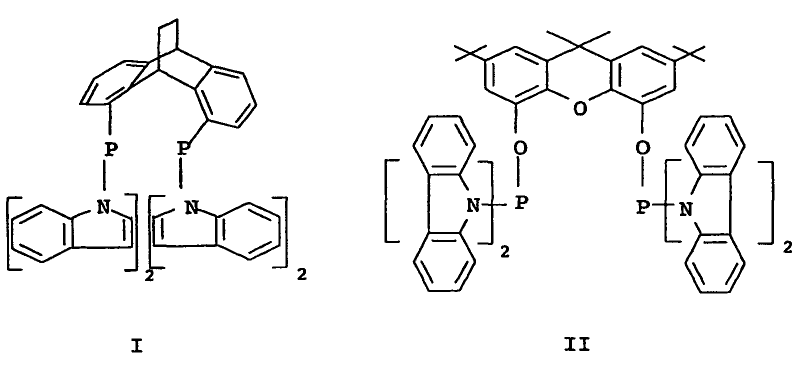 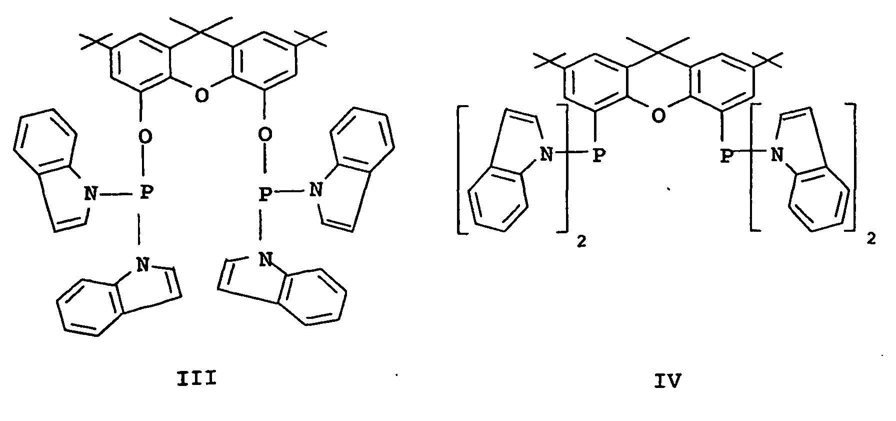 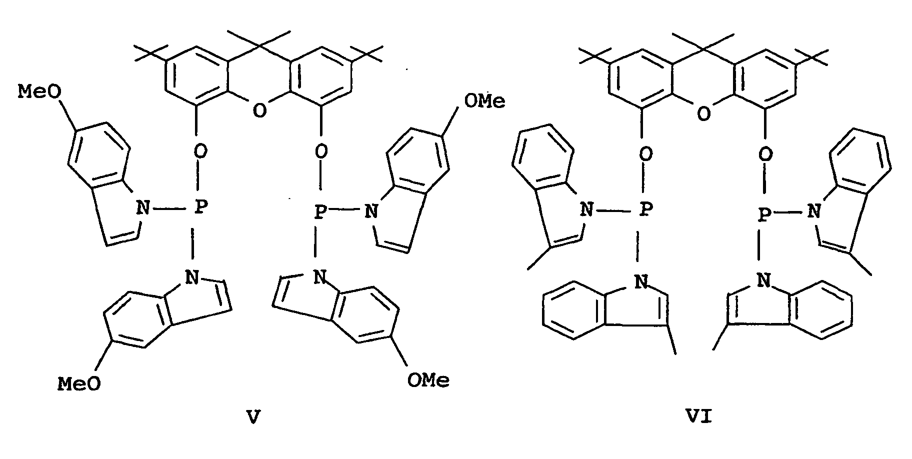 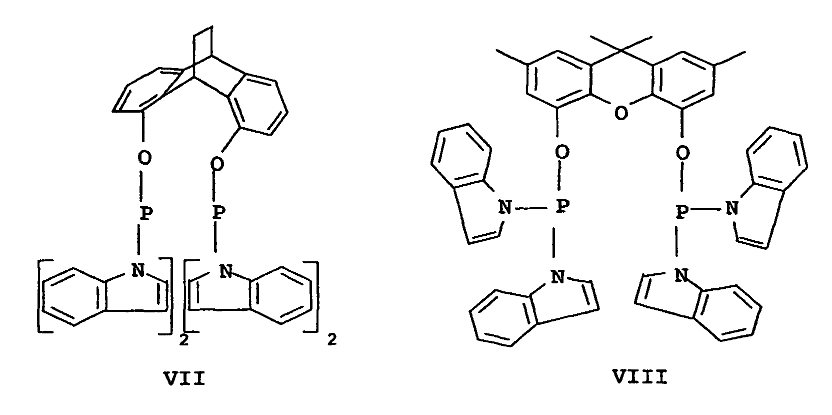 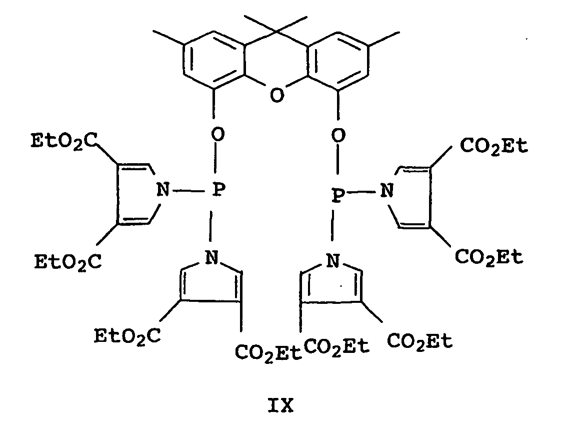 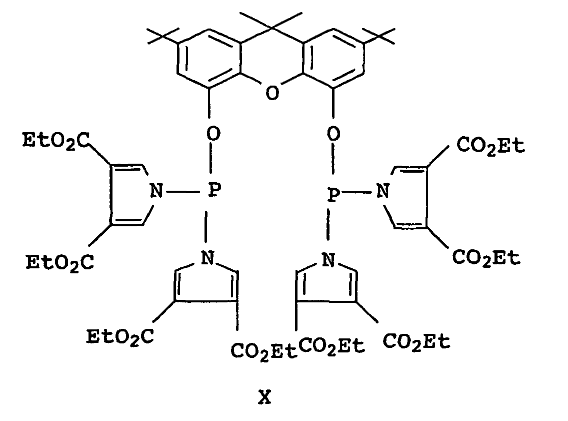 Me = Methyl
Et = Ethyl
R^{m} = H, Carboxylat
Rⁿ = H, Carboxylat

Die Herstellung der erfindungsgemäßen Pnicogenchelatverbindungen kann zweckmäßigerweise ausgehend von Verbindungen der allgemeinen Formel III 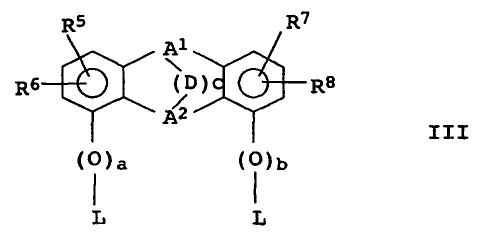 erfolgen, in der A¹, A², D, R⁵, R⁶, R⁷, R⁸, a, b und c die genannte Bedeutung haben und L für eine Abgangsgruppe steht, die falls a und b für die Zahl 0 stehen, beispielsweise für Wasserstoff (wenn A² eine Oxa-Gruppe ist), Halogen, insbesondere Flur, Chlor, Brom, SO₃Me mit Me = Wasserstoff oder Alkalimetall, insbesondere Li, Na oder K, stehen kann, oder falls a und b für die Zahl 1 stehen, beispielsweise für Wasserstoff, C(O)CF₃, SO₂CH₃, SO₂-Tolyl oder SO₂CF₃ stehen kann.

Die Ausgangsverbindungen der allgemeinen Formel III mit a, b, c = 0 können gemäß den von van Leuwen et al., Organometallics 14, 3081 (1995) angegebenen Methoden hergestellt werden.

Die Ausgangsverbindungen der allgemeinen Formel III mit a, b = 1, c = 0, und L = H, können z.B. aus den entsprechenden 1,8-Dibromverbindungen der allgemeinen Formel III (a, b = 0; L = Br) z.B. durch Metallierung mit Alkalimetallorganylen, wie n-Butyllithium, tert. Butyllithium oder dergleichen, anschließende Umsetzung mit einem Boran, vorzugsweise BH₃ und Oxidation der dabei gebildeten Diboranverbindung mit einem Peroxid, vorzugsweise Wasserstoffperoxid in Gegenwart von wässrigem Alkalimetallhydroxid, vorzugsweise Lithium-, Natrium- oder Kaliumhydroxid, erhalten werden.

Zur Anknüpfung der Gruppen PnR¹R² und PnR³R⁴ werden die Ausgangsverbindungen der allgemeinen Formel III vorteilhaft mit einer Halogenverbindung der Formel HalPnR¹R² bzw. HalPnR³R⁴ in Gegenwart einer Base umgesetzt. Hal steht hierbei vorzugsweise für Chlor oder Brom.

Die Verbindungen HalPnR¹R² bzw. HalPnR³R⁴ können beispielsweise in Analogie zur Methode von Petersen et al, J. Am. Chem. Soc. 117, 7696 (1995) durch Umsetzung der betreffenden Pyrrolverbindung, z. B. Pyrrol, Indol, Benzotriazol, Carbazol, 2,2'-Dipyrrolmethan, 2,2'-Bisindol - mit dem betreffenden Pnicogentrihalogenid, z. B. Phosphortrichlorid, in Gegenwart eines tertiären Amins, z. B. Triethylamin, erhalten werden, wobei die Stöchiometrie dieser Umsetzung zu beachten ist.

In Analogie zu dieser Vorgehensweise können z. B. aus den betreffenden Hydroxyaryl-pyrrolyl-Verbindungen durch Umsetzung mit dem Pnicogentrihalogenid in Gegenwart eines tertiären Amins die entsprechenden Ausgangsverbindungen HalPnR¹R² bzw. HalPnR³R⁴ erhalten.

Durch stufenweise Synthese sind weitere Ausgangsverbindungen HalPnR¹R² bzw. HalPnR³R⁴ erhältlich. So kann z. B. durch Umsetzung von Phenol mit Phosphortrichlorid in Gegenwart eines tertiären Amins, z. B. Triethylamin, das Phenoxyphosphordichlorid erzeugt werden, das nach Umsetzung mit einem Äquivalent der betreffenden Pyrrolverbindung, z. B. Pyrrol, in Gegenwart eines tertiären Amins, Phenoxy-pyrrolyl-phosphorchlorid ergibt.

Die Herstellung der 2,2'-Bisindol-Ausgangsverbindungen kann in Analogie zu Tetrahedron 51, 5637 (1995) und Tetrahedron 51, 12801 (1995) erfolgen, die Herstellung der Bis-2,2'-pyrrolyl-methane entsprechend den Angaben von J. Org. Chem. 64, 1391 (1999) und die Herstellung der 2'-Pyrrolyl-o-phenoxy-methane nach J. Org. Chem. 46, 5060 (1981).

Die Herstellung der Ausgangsverbindungen III mit Triptycen-artigem Kohlenstoffgerüst kann auf bekannte Weise aus den entsprechenden Anthrachinonderivaten über die entsprechenden Anthracenvorstufen (Herstellung siehe z. B.: J. Org. Chem. 45, 1807 (1980); J. Org. Chem. 38, 1167 (1973); Bull Chem. Soc. Jm. 44, 1649 (1971); J. Am. Chem. Soc. 34, 3089 (1969); J. Org. Chem. 39, 770 (1974); Chem. Ber. 124, 333 (1991); J. Org. Chem. 51, 921 (1986)) durch deren Diels-Alder-Reaktion mit den entsprechenden Olefinen, Acetylenen oder Arinen erfolgen. Angaben zur Durchführung dieser Diels-Alder-Reaktionen finden sich in Chem. Ber. 119, 1016 (1986) und J. C. S. Chem. Comm. 961 (1999).

Zur Herstellung der Pnicogenchelatverbindungen der allgemeinen Formeln I bzw. II aus den Verbindungen der allgemeinen Formel III durch deren Umsetzung mit den Verbindungen HalPnR¹R² bzw. HalPnR³R⁴ müssen die Verbindungen der allgemeinen Formel III zunächst aktiviert werden.

Für Verbindungen der allgemeinen Formel III mit a, b = 0 gelingt dies vorteilhaft durch Metallierung mittels einer Alkalimetallorganyl-Verbindung, vorzugsweise mit einer Alkyllithiumverbindung, wie n-Butyllithium, tert.-Butyllithium oder Methyllithium, wobei die Abgangsgruppe L in separater Umsetzung durch das betreffende Alkalimetallatom, vorzugsweise Lithium, ersetzt wird.

Nach Zugabe von HalPnR¹R² und HalPnR³R⁴ zu dieser metallierten Verbindung bilden sich die entsprechenden Pnicogenchelatverbindungen der allgemeinen Formel I bzw. II mit a, b = 0.

Für die Aktivierung der Verbindungen der allgemeinen Formel III mit a, b = 1 ist in der Regel keine separate Aktivierung mit Alkalimetallorganyl-Verbindungen erforderlich. Im Allgemeinen führt die Umsetzung dieser Verbindungen mit den Verbindungen HalPnR¹R² und HalPnR³R⁴ in Gegenwart einer Base, vorzugsweise einem tertiären Amin, wie Triethylamin, oder einem Alkalimetall- oder Erdalkalimetallhydrid, beispielsweise Natriumhydrid, Kaliumhydrid oder Calciumhydrid, direkt zu den erfindungsgemäßen Pnicogenchelatverbindungen der allgemeinen Formel I bzw. II mit a, b = 0.

Anstelle von Verbindungen der Formel III (mit a, b = 0) mit L = Halogen oder SO₃Me können auch solche Verbindungen III mit L = Wasserstoff lithiiert werden, in denen in der Metaposition von A² (A² = O oder S) sich jeweils Wasserstoff, eine Alkoxygruppe oder Alkoxycarbonylgruppe befindet. Derartige Reaktionen sind unter dem Begriff "ortho-Lithiierung" in der Literatur beschrieben (siehe z. B. D. W. Slocum, J. Org. Chem., 41, 3652-3654 (1976); J. M. Mallan, R. L. Bebb, Chem. Rev., 1969, 693ff; V. Snieckus, Chem. Rev., 1980, 6, 879-933). Die dabei erhaltenen Organolithiumverbindungen können dann mit den Phosphorhalogenverbindungen in der oben angegebenen Weise zu den Chelatverbindungen der Formel I bzw. II umgesetzt werden.

In analoger Weise gelingt die Herstellung der Arsen- und der Antimonverbindungen gemäß Formel I bzw. II.

Im Allgemeinen werden unter Hydroformylierungsbedingungen aus den jeweils eingesetzten Katalysatoren oder Katalysatorvorstufen katalytisch aktive Spezies der allgemeinen Formel HgZ_{d}(CO)ₑG_{f} gebildet, worin Z für ein Metall der VIII. Nebengruppe, G für eine Phosphor-, Arsen- oder Antimon-haltigen Liganden der Formel I bzw. II und d, e, f, g für ganze Zahlen, abhängig von der Wertigkeit und Art des Metalls sowie der Bindigkeit des Liganden G, stehen. Vorzugsweise stehen e und f unabhängig voneinander mindestens für einen Wert von 1, wie z. B. 1, 2 oder 3. Die Summe aus e und f steht bevorzugt für einen Wert von 2 bis 5. Dabei können die Komplexe des Metalls Z mit den erfindungsgemäßen Liganden G gewünschtenfalls zusätzlich noch mindestens einen weiteren, nicht-erfindungsgemäßen Liganden, z. B. aus der Klasse der Triarylphosphine, insbesondere Triphenylphosphin, Triarylphosphite, Triarylphosphinite, Triarylphosphonite, Phosphabenzole, Trialkylphosphine oder Phosphametallocene enthalten. Derlei Komplexe des Metalls Z mit erfindungsgemäßen und nicht-erfindungsgemäßen Liganden bilden sich z. B. in einer Gleichgewichtsreaktion nach Zusatz eines nicht-erfindungsgemäßen Liganden zu einem Komplex der allgemeinen Formel H_{g}Zₐ(CO)ₑG_{f}.

Nach einer bevorzugten Ausführungsform werden die Hydroformylierungskatalysatoren in situ, in dem für die Hydroformylierungsreaktion eingesetzten Reaktor, hergestellt. Gewünschtenfalls können die erfindungsgemäßen Katalysatoren jedoch auch separat hergestellt und nach üblichen Verfahren isoliert werden. Zur in situ-Herstellung der erfindungsgemäßen Katalysatoren kann man wenigstens eine Verbindung der allgemeinen Formel I bzw. II, eine Verbindung oder einen Komplex eines Metalls der VIII. Nebengruppe, gewünschtenfalls einen oder mehrere weitere zusätzliche, nicht-erfindungsgemäße Liganden und gegebenenfalls ein Aktivierungsmittel in einem inerten Lösungsmittel unter den Hydroformylierungsbedingungen umsetzen.

Geeignete Rhodiumverbindungen oder -komplexe sind z. B. Rhodium(II)- und Rhodium(III)-salze, wie Rhodium(III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)-säure, Trisammoniumhexachlororhodat(III) etc. Weiterhin eignen sich Rhodiumkomplexe, wie Rhodiumbiscarbonylacetylacetonat, Acetylacetonatobisethylenrhodium(I) etc. Vorzugsweise werden Rhodiumbiscarbonylacetylacetonat oder Rhodiumacetat eingesetzt.

Ebenfalls geeignet sind Rutheniumsalze oder -verbindungen. Geeignete Rutheniumsalze sind beispielsweise Ruthenium(III)chlorid, Ruthenium(IV)-, Ruthenium(VI)- oder Ruthenium(VIII)oxid, Alkalisalze der Rutheniumsauerstoffsäuren wie K₂RuO₄ oder KRuO₄ oder Komplexverbindungen, wie z. B. RuHCl(CO)(PPh₃)₃. Auch können die Metallcarbonyle des Rutheniums wie Trisrutheniumdodecacarbonyl oder Hexarutheniumoctadecacarbonyl, oder Mischformen, in denen CO teilweise durch Liganden der Formel PR₃ ersetzt sind, wie Ru(CO)₃(PPh₃)₂, im erfindungsgemäßen Verfahren verwendet werden.

Geeignete Kobaltverbindungen sind beispielsweise Kobalt(II)chlorid, Kobalt(II)sulfat, Kobalt(II)carbonat, Kobalt(II)nitrat, deren Amin- oder Hydratkomplexe, Kobaltcarboxylate, wie Kobaltacetat, Kobaltethylhexanoat, Kobaltnaphthenoat, sowie der Kobalt-Caprolactamat-Komplex. Auch hier können die Carbonylkomplexe des Kobalts wie Dikobaltoctacarbonyl, Tetrakobaltdodecacarbonyl und Hexakobalthexadecacarbonyl eingesetzt werden.

Die genannten und weitere geeignete Verbindungen des Kobalts, Rhodiums, Rutheniums und Iridiums sind bekannt, kommerziell erhältlich oder ihre Herstellung ist in der Literatur hinreichend beschrieben oder sie können vom Fachmann analog zu den bereits bekannten Verbindungen hergestellt werden.

Geeignete Aktivierungsmittel sind z. B. Brönsted-Säuren, Lewis-Säuren, wie z. B. BF_{3,} AlCl₃, ZnCl₂, und Lewis-Basen.

Als Lösungsmittel werden vorzugsweise die Aldehyde eingesetzt, die bei der Hydroformylierung der jeweiligen Olefine entstehen, sowie deren höher siedende Folgereaktionsprodukte, z. B. die Produkte der Aldolkondensation. Ebenfalls geeignete Lösungsmittel sind Aromaten, wie Toluol und Xylole, Kohlenwasserstoffe oder Gemische von Kohlenwasserstoffen, auch zum Verdünnen der oben genannten Aldehyde und der Folgeprodukte der Aldehyde. Weitere Lösungsmittel sind Ester aliphatischer Carbonsäuren mit Alkanolen, beispielsweise Essigester oder Texanol®, Ether wie tert.-Butylmethylether und Tetrahydrofuran. Bei ausreichend hydrophilisierten Liganden können auch Alkohole, wie Methanol, Ethanol, n-Propanol, Isopropanol, n-Butanol, Isobutanol, Ketone, wie Aceton und Methylethylketon etc., eingesetzt werden. Ferner können als Lösungsmittel auch sogenannte "Ionische Flüssigkeiten" verwendet werden. Hierbei handelt es sich um flüssige Salze, beispielsweise um N,N'-Di-alkylimidazoliumsalze wie die N-Butyl-N'-methylimidazoliumsalze, Tetraalkylammoniumsalze wie die Tetra-n-butylammoniumsalze, N-Alkylpyridiniumsalze wie die n-Butylpyridiniumsalze, Tetraalkylphosphoniumsalze wie die Trishexyl(tetradecyl)phosphoniumsalze, z. B. die Tetrafluoroborate, Acetate, Tetrachloroaluminate, Hexafluorophosphate, Chloride und Tosylate.

Weiterhin ist es möglich die Umsetzungen auch in Wasser oder wässrigen Lösungsmittelsystemen, die neben Wasser ein mit Wasser mischbares Lösungsmittel, beispielsweise einen Alkohol wie Methanol, Ethanol, n-Propanol, Isopropanol, n-Butanol, Isobutanol, ein Keton wie Aceton und Methylethylketon oder ein anderes Lösungsmittel enthalten. Zu diesem Zweck setzt man Liganden der Formel I bzw. II ein, die mit polaren Gruppen, beispielsweise ionischen Gruppen wie SO₃Me, CO₂Me mit Me = Na, K oder NH₄ oder wie N(CH₃)₃⁺ modifiziert sind. Die Umsetzungen erfolgen dann im Sinne einer Zweiphasenkatalyse, wobei der Katalysator sich in der wässrigen Phase befindet und Einsatzstoffe und Produkte die organische Phase bilden. Auch die Umsetzung in den "Ionischen Flüssigkeiten" kann als Zweiphasenkatalyse ausgestaltet sein.

Das Molmengenverhältnis von Pnicogenchelatverbindung I bzw. II zum Metall der VIII. Nebengruppe im Hydroformylierungsmedium liegt im Allgemeinen in einem Bereich von etwa 1:1 bis 1000:1, vorzugsweise von 1:1 bis 100:1, insbesondere von 1:1 bis 50:1.

Als Substrate für das erfindungsgemäße Hydroformylierungsverfahren kommen prinzipiell alle Verbindungen in Betracht, welche eine oder mehrere ethylenisch ungesättigte Doppelbindungen enthalten. Dazu zählen z. B. Olefine, wie α-Olefine, interne geradkettige und interne verzweigte Olefine. Geeignete α-Olefine sind z. B. Propen, 1-Buten, 1-Penten, 1-Hexen, 1-Hepten, 1-Octen, 1-Nonen, 1-Decen, 1-Undecen, 1-Dodecen etc.

Geeignete verzweigte, interne Olefine sind vorzugsweise C₄-C₂-Olefine, wie 2-Methyl-2-Buten, 2-Methyl-2-Penten, 3-Methyl-2-Penten, verzweigte, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen-Gemische etc.

Geeignete zu hydroformylierende Olefine sind weiterhin C₅-C₈-Cycloalkene, wie Cyclopenten, Cyclohexen, Cyclohepten, Cycloocten und deren Derivate, wie z. B. deren C₁-C₂₀-Alkylderivate mit 1 bis 5 Alkylsubstituenten. Geeignete zu hydroformylierende Olefine sind weiterhin Vinylaromaten, wie Styrol, α-Methylstyrol, 4-Isobutylstyrol etc. Geeignete zu hydroformylierende Olefine sind weiterhin α,β-ethylenisch ungesättigte Mono- und/oder Dicarbonsäuren, deren Ester, Halbester und Amide, wie Acrylsäure, Methacrylsäure, Maleinsäure, Fumarsäure, Crotonsäure, Itaconsäure, 3-Pentensäuremethylester, 4-Pentensäuremethylester, Ölsäuremethylester, Acrylsäuremethylester, Methacrylsäuremethylester, ungesättigte Nitrile, wie 3-Pentennitril, 4-Pentennitril, Acrylnitril, Vinylether, wie Vinylmethylether, Vinylethylether, Vinylpropylether etc., C₁-C₂₀-Alkenole, -Alkendiole und -Alkadienole, wie 2,7-Octadienol-1. Geeignete Substrate sind weiterhin Di- oder Polyene mit isolierten oder konjugierten Doppelbindungen. Dazu zählen z. B. 1,3-Butadien, 1,4-Pentadien, 1,5-Hexadien, 1,6-Heptadien, 1,7-Octadien, Vinylcyclohexen, Dicyclopentadien, 1,5,9-Cyclooctatrien sowie Butadienhomo- und -copolymere.

Bevorzugt ist die zur Hydroformylierung eingesetzte ungesättigte Verbindung ausgewählt unter internen linearen Olefinen und Olefingemischen, die wenigstens ein internes lineares Olefin enthalten. Geeignete lineare (geradkettige) interne Olefine sind vorzugsweise C₄-C₂₀-Olefine, wie 2-Buten, 2-Penten, 2-Hexen, 3-Hexen, 2-Hepten, 3-Hepten, 2-Octen, 3-Octen, 4-Octen etc. und Mischungen davon.

Vorzugsweise wird in dem erfindungsgemäßen Hydroformylierungsverfahren ein großtechnisch zugängliches Olefingemisch eingesetzt, das insbesondere wenigstens ein internes lineares Olefin enthält. Dazu zählen z. B. die durch gezielte Ethen-Oligomerisierung in Gegenwart von Alkylaluminiumkatalysatoren erhaltenen Ziegler-Olefine. Dabei handelt es sich im Wesentlichen um unverzweigte Olefine mit endständiger Doppelbindung und gerader Kohlenstoffatomanzahl. Dazu zählen weiterhin die durch Ethen-Oligomerisierung in Gegenwart verschiedener Katalysatorsysteme erhaltenen Olefine, z. B. die in Gegenwart von Alkylaluminiumchlorid/Titantetrachlorid-Katalysatoren erhaltenen, überwiegend linearen α-Olefine und die in Gegenwart von Nickel-Phosphinkomplex-Katalysatoren nach dem Shell Higher Olefin Process (SHOP) erhaltenen α-Olefine. Geeignete technisch zugängige Olefingemische werden weiterhin bei der Paraffin-Dehydrierung entsprechender Erdölfraktionen, z. B. der sog. Petroleum- oder Dieselölfraktionen, erhalten. Zur Überführung von Paraffinen, vorwiegend von n-Paraffinen in Olefine, werden im Wesentlichen drei Verfahren eingesetzt:
- thermisches Cracken (Steamcracken),
- katalytisches Dehydrieren und
- chemisches Dehydrieren durch Chlorieren und Dehydrochlorieren.

Dabei führt das thermische Cracken überwiegend zu α-Olefinen, während die anderen Varianten Olefingemische ergeben, die im Allgemeinen auch größere Anteile an Olefinen mit innenständiger Doppelbindung aufweisen. Geeignete Olefingemische sind weiterhin die bei Metathese- bzw. Telomerisationsreaktionen erhaltenen Olefine. Dazu zählen z. B. die Olefine aus dem Phillips-Triolefin-Prozess, einem modifizierten SHOP-Prozess aus Ethylen-Oligomerisierung, Doppelbindungs-Isomerisierurig und anschließender Metathese (Ethenolyse).

Geeignete in dem erfindungsgemäßen Hydroformylierungsverfahren einsetzbare technische Olefingemische sind weiterhin ausgewählt unter Dibutenen, Tributenen, Tetrabutenen, Dipropenen, Tripropenen, Tetrapropenen, Mischungen von Butenisomeren, insbesondere Raffinat II, Dihexenen, Dimeren und Oligomeren aus dem Dimersol®-Prozess von IFP, Octolprozess® von Hüls, Polygas®-prozess etc.

Bevorzugt ist ein Verfahren, das dadurch gekennzeichnet ist, dass der Hydroformylierungskatalysator in situ hergestellt wird, wobei man mindestens eine Verbindung der Formel I bzw. II, eine Verbindung oder einen Komplex eines Metalls der VIII. Nebengruppe und gegebenenfalls ein Aktivierungsmittel in einem inerten Lösungsmittel unter den Hydroformylierungsbedingungen zur Reaktion bringt.

Die Hydroformylierungsreaktion kann kontinuierlich, semikontinuierlich oder diskontinuierlich erfolgen.

Geeignete Reaktoren für die kontinuierliche Umsetzung sind dem Fachmann bekannt und werden z. B. in Ullmanns Encyklopädie der technischen Chemie, Bd. 1, 3. Aufl., 1951, S. 743 ff. beschrieben.

Geeignete druckfeste Reaktoren sind dem Fachmann ebenfalls bekannt und werden z. B. in Ullmanns Encyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die Zusammensetzung des im erfindungsgemäßen Verfahren eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5:95 bis 70:30, bevorzugt etwa 40:60 bis 60:40. Insbesondere bevorzugt wird ein molares Verhältnis von Kohlenmonoxid und Wasserstoff im Bereich von etwa 1:1 eingesetzt.

Die Temperatur bei der Hydroformylierungsreaktion liegt im Allgemeinen in einem Bereich von etwa 20 bis 180 °C, bevorzugt etwa 50 bis 150 °C. Die Reaktion wird in der Regel bei dem Partialdruck des Reaktionsgases bei der gewählten Reaktionstemperatur durchgeführt. Im Allgemeinen liegt der Druck in einem Bereich von etwa 1 bis 700 bar, bevorzugt 1 bis 600 bar, insbesondere 1 bis 300 bar. Der Reaktionsdruck kann in Abhängigkeit von der Aktivität des eingesetzten erfindungsgemäßen Hydroformylierungskatalysators variiert werden. Im Allgemeinen erlauben die erfindungsgemäßen Katalysatoren auf Basis von Phosphor-, Arsen- oder Antimon-haltigen Verbindungen eine Umsetzung in einem Bereich niedriger Drücke, wie etwa im Bereich von 1 bis 100 bar.

Die erfindungsgemäß eingesetzten und die erfindungsgemäßen Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

Die zuvor beschriebenen, erfindungsgemäßen Katalysatoren, die chirale Verbindungen der allgemeinen Formel I umfassen, eignen sich zur enantioselektiven Hydroformylierung.

Die zuvor beschriebenen Katalysatoren können auch in geeigneter Weise, z. B. durch Anbindung über als Ankergruppen geeignete funktionelle Gruppen, Adsorption, Pfropfung, etc. an einen geeigneten Träger, z. B. aus Glas, Kieselgel, Kunstharzen etc., immobilisiert werden. Sie eignen sich dann auch für einen Einsatz als Festphasenkatalysatoren.

Überraschenderweise haben die aus den erfindungsgemäßen Pnicogenchelatverbindungen der allgemeinen Formel I bzw. II hergestellten Katalysatoren nicht nur eine hohe Aktivität bezüglich der Hydroformylierung endständiger Olefine, sondern ebenfalls bezüglich der isomerisierenden Hydroformylierung von Olefinen mit internen Doppelbindungen zu Aldehydprodukten mit hoher Linearität. Vorteilhafterweise findet unter den Bedingungen der Hydroformylierung mit den erfindungsgemäßen Katalysatoren eine Hydrierung der Olefine nur in sehr geringem Ausmaß statt.

Überraschenderweise weisen insbesondere Katalysatoren auf Basis von Pnicogenchelatverbindungen der allgemeinen Formel II eine hohe Stabilität unter den Hydroformylierungsbedingungen auf, so dass mit ihnen in der Regel längere Katalysatorstandzeiten erzielt werden, als mit aus dem Stand der Technik bekannten Katalysatoren auf Basis herkömmlicher ein- und mehrzähniger Liganden. So haben die Erfinder gefunden, dass Pyrrolphosphorverbindungen aus dem Stand der Technik, bei denen eine oder mehrere unsubstituierte Pyrrolgruppen über ihr Stickstoffatom an das Phosphoratom gebunden sind, leicht zur Zersetzung bzw. zur Ausbildung von unerwünschten Umsetzungsprodukten neigen. So wird beim Einsatz von einzähnigen Liganden auf Basis unsubstituierter Pyrrolgruppen, wie beispielsweise Trispyrrolylphosphin, aber auch beim Einsatz von Chelatliganden mit von den erfindungsgemäßen Liganden verschiedenen Brückengruppen, beispielsweise den in der US 5,710,344 beschriebenen, eine merkliche Zersetzung bereits durch sichtbares Licht und/oder Temperaturen im Bereich der Raumtemperatur induziert und kann auch durch den Einsatz eines Schutzgases nicht verhindert werden. In Gegenwart von Aldehyden kann es zu einer merklichen Bildung von polymeren Verunreinigungen kommen. Beim Einsatz von derartigen Pyrrolphosphorverbindungen als Liganden für Hydroformylierungskatalysatoren kommt es somit zu einem Verlust an Katalysator und Wertprodukt, der sich insbesondere bei mehrstufigen Verfahren, die einen solchen Hydroformylierungsschritt umfassen (z. B. die Herstellung von 2-Propylheptanol), negativ auf die Wirtschaftlichkeit auswirkt. Überraschenderweise wurde nun gefunden, dass beim Einsatz von Pnicogenverbindungen, insbesondere von Phosphorverbindungen, der allgemeinen Formel II, bei denen eine substituierte und/oder in ein anelliertes Ringsystem integrierte Pyrrolgruppe über ihr pyrrolisches Stickstoffatom kovalent mit dem Phosphoratom verknüpft ist, die Bildung unerwünschter Produkte im Wesentlichen unterbleibt.

Ein weiterer Gegenstand der Erfindung ist ein Verfahren zur Herstellung von 2-Propylheptanol, bei dem man
a) Buten oder ein Buten enthaltendes C₄-Kohlenwasserstoffgemisch in Gegenwart eines Hydroformylierungskatalysators mit Kohlenmonoxid und Wasserstoff unter Erhalt eines n-Valeraldehyd enthaltenden Hydroformylierungsprodukts hydroformyliert, wobei der Hydroformylierungskatalysator wenigstens einen Komplex eines Metalls der VIII. Nebengruppe mit wenigstens einem Liganden der allgemeinen Formeln I oder II, wie zuvor definiert, umfasst.
b) gegebenenfalls das Hydroformylierungsprodukt einer Auftrennung unter Erhalt einer an n-Valeraldehyd angereicherten Fraktion unterzieht,
c) das in Schritt a) erhaltene Hydroformylierungsprodukt oder die in Schritt b) erhaltene an n-Valeraldehyd angereicherte Fraktion einer Aldolkondensation unterzieht,
d) die Produkte der Aldolkondensation mit Wasserstoff katalytisch zu Alkoholen hydriert, und
e) gegebenenfalls die Hydrierprodukte einer Auftrennung unter Erhalt einer an 2-Propylheptanol angereicherten Fraktion unterzieht.

Vorzugsweise wird in Schritt a) ein Hydroformylierungskatalysator eingesetzt, der wenigstens einen Komplex eines Metalls der VIII. Nebengruppe mit wenigstens einem Liganden der allgemeinen Formel II umfasst. Bezüglich geeigneter und bevorzugter Liganden der Formel II wird auf die vorherigen Ausführungen Bezug genommen.

### a) Hydroformylierung

Als Einsatzmaterial für die Hydroformylierung eignet sich sowohl im Wesentlichen reines 1-Buten als auch Gemische von 1-Buten mit 2-Buten und technisch erhältliche C₄-Kohlenwasserstoffströme, die 1-Buten und/oder 2 Buten enthalten. Vorzugsweise eignen sich C₄-Schnitte, die in großen Mengen aus FCC-Anlagen und aus Steamcrackern zur Verfügung stehen. Diese bestehen im Wesentlichen aus einem Gemisch der isomeren Butene und Butan.

Als Einsatzmaterial geeignete C₄-Kohlenwasserstoffströme enthalten z. B. 50 bis 99, vorzugsweise 60 bis 90 Mol-% Butene und 1 bis 50, vorzugsweise 10 bis 40 Mol-% Butane. Vorzugsweise umfasst die Butenfraktion 40 bis 60 Mol-% 1-Buten, 20 bis 30 Mol-% 2-Buten und weniger als 5 Mol-%, insbesondere weniger als 3 Mol-% Isobuten (bezogen auf die Butenfraktion). Als besonders bevorzugter Einsatzstoff wird das sogenannte Raffinat II verwendet, bei dem es sich um ein Isobuten-abgereicherten C₄-Schnitt aus einer FCC-Anlage oder einen Steamcracker handelt.

Hydroformylierungskatalysatoren auf Basis der erfindungsgemäß eingesetzten Pnicogenchelatverbindungen als Liganden weisen vorteilhafterweise eine hohe n-Selektivität, auch beim Einsatz von 2-Buten und 2-butenhaltigen Kohlenwasserstoffgemischen als Einsatzmaterial auf. Somit können in dem erfindungsgemäßen Verfahren auch solche Einsatzstoffe wirtschaftlich eingesetzt werden, da der angestrebte n-Valeraldehyd in guten Ausbeuten resultiert.

Bezüglich geeigneter und bevorzugter Hydroformylierungskatalysatoren, Aktivierungsmitteln, Lösungsmitteln, Reaktionsbedingungen und Reaktoren für die Hydroformylierung in Schritt a) wird auf die vorherigen allgemeinen Ausführungen zur Hydroformylierung in vollem Umfang Bezug genommen.

Die erfindungsgemäßen Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

### b) Auftrennung

Nach einer geeigneten Verfahrensvariante wird die in Schritt a) nach Abtrennung des Katalysatorsystems erhaltene produktangereicherte Fraktion einer weiteren Auftrennung zum Erhalt einer an n-Valeraldehyd angereicherten Fraktion unterzogen. Die Auftrennung des Hydroformylierungsprodukts in eine n-Valeraldehyd angereicherte Fraktion und eine n-Valeraldehyd abgereicherte Fraktion erfolgt nach üblichen, dem Fachmann bekannten Verfahren. Bevorzugt ist die Destillation unter Einsatz bekannter Trennapparaturen, wie Destillationskolonnen, z. B. Bodenkolonnen, die gewünschtenfalls mit Glocken, Siebplatten, Siebböden, Ventilen etc. ausgerüstet sein können, Verdampfer, wie Dünnschichtverdampfer, Fallfilmverdampfer, Wischblattverdampfer etc.

### c) Aldolkondensation

Zwei Moleküle C₅-Aldehyd können zu α,β-ungesättigten C₁₀-Aldehyden kondensiert werden. Die Aldolkondensation erfolgt auf an sich bekannte Weise z. B. durch Einwirkung einer wässrigen Base, wie Natronlauge oder Kalilauge. Alternativ kann auch ein heterogener basischer Katalysator, wie Magnesium- und/oder Aluminiumoxid, verwendet werden (vgl. z. B. die EP-A 792 862). Dabei resultiert bei der Kondensation von zwei Molekülen n-Valeraldehyd 2-Propyl-2-heptenal. Sofern das in Schritt a) bzw. nach der Auftrennung in Schritt b) erhaltene Hydroformylierungsprodukt noch weitere C₅-Aldehyde, wie 2-Methylbutanal und gegebenenfalls 2,2-Dimethylpropanal bzw. 3-Methylbutanal aufweist, so untergehen diese ebenfalls eine Aldolkondensation, wobei dann die Kondensationsprodukte aller möglichen Aldehydkombinationen resultieren, beispielsweise 2-Propyl-4-methyl-2-hexenal. Ein Anteil dieser Kondensationsprodukte, z. B. von bis zu 30 Gew.-%, steht einer vorteilhaften Weiterverarbeitung zu als Weichmacheralkoholen geeigneten 2-Propylheptanol-haltigen C₁₀-Alkoholgemischen nicht entgegen.

### d) Hydrierung

Die Produkte der Aldolkondensation können mit Wasserstoff katalytisch zu C₁₀-Alkoholen, wie insbesondere 2-Propylheptanol, hydriert werden.

Für die Hydrierung der C₁₀-Aldehyde zu den C₁₀-Alkoholen sind prinzipiell auch die Katalysatoren der Hydroformylierung zumeist bei höherer Temperatur geeignet; im Allgemeinen werden jedoch selektivere Hydrierkatalysatoren vorgezogen, die in einer separaten Hydrierstufe eingesetzt werden. Geeignete Hydrierkatalysatoren sind im Allgemeinen Übergangsmetalle, wie z. B. Cr, Mo, W, Fe, Rh, Co, Ni, Pd, Pt, Ru usw. oder deren Mischungen, die zur Erhöhung der Aktivität und Stabilität auf Trägern, wie z. B. Aktivkohle, Aluminiumoxid, Kieselgur usw. aufgebracht werden können. Zur Erhöhung der katalytischen Aktivität können Fe, Co und bevorzugt Ni, auch in Form der Raney-Katalysatoren, als Metallschwamm mit einer sehr großen Oberfläche verwendet werden. Die Hydrierung der C₁₀-Aldehyde erfolgt in Abhängigkeit von der Aktivität des Katalysators, vorzugsweise bei erhöhten Temperaturen und erhöhtem Druck. Vorzugsweise liegt die Hydriertemperatur bei etwa 80 bis 250 °C, bevorzugt liegt der Druck bei etwa 50 bis 350 bar.

Das rohe Hydrierungsprodukt kann nach üblichen Verfahren, z. B. durch Destillation, zu den C₁₀-Alkoholen aufgearbeitet werden.

### e) Auftrennung

Gewünschtenfalls können die Hydrierprodukte einer weiteren Auftrennung unter Erhalt einer an 2-Propylheptanol angereicherten Fraktion und einer an 2-Propylheptanol abgereicherten Fraktion unterzogen werden. Diese Auftrennung kann nach üblichen, dem Fachmann bekannten Verfahren, wie z. B. durch Destillation, erfolgen.

Hydroformylierungskatalysatoren, die einen Komplex wenigstens eines Metalls der VIII. Nebengruppe des Periodensystems aufweisen, der als Liganden mindestens eine Pnicogenchelatverbindung der Formel I und insbesondere eine Pyrrolphosphorverbindung der allgemeinen Formel II mit substituiertem und/oder anelliertem Pyrrolgerüst aufweist, eignen sich in vorteilhafter Weise für den Einsatz in einem Verfahren zur Herstellung von 2-Propylheptanol. Dabei weisen die Katalysatoren eine hohe n-Selektivität auf, so dass sowohl beim Einsatz von im Wesentlichen reinem 1-Buten als auch beim Einsatz von 1-Buten/2-Buten-haltigen Kohlenwasserstoffgemischen, wie beispielsweise C₄-Schnitten eine gute Ausbeute an n-Valeraldehyd erhalten wird. Des Weiteren eignen sich die erfindungsgemäß eingesetzten Katalysatoren auch zur Doppelbindungsisomerisierung von einer innenständigen auf eine endständige Position, so dass auch beim Einsatz von 2-Buten und höhere Konzentrationen an 2-Buten-haltigen Kohlenwasserstoffgemischen n-Valeraldehyd in guten Ausbeuten erhalten wird. Vorteilhafterweise zeigen die erfindungsgemäß eingesetzten Katalysatoren auf Basis von substituierten bzw. anellierten Pyrrolgerüsten im Wesentlichen keine Zersetzung unter den Hydroformylierungsbedingungen, d. h. in Anwesenheit von Aldehyden. Vorteilhafterweise werden auch in Gegenwart von Luftsauerstoff und/oder Licht und/oder Säuren und/oder bei Raumtemperatur und erhöhten Temperaturen, wie bis zu etwa 150 °C, im Wesentlichen keine Zersetzungsprodukte gebildet, so dass auf den Einsatz aufwendiger Maßnahmen zur Stabilisierung des eingesetzten Hydroformylierungskatalysators, insbesondere bei der Aufarbeitung, verzichtet werden kann.

Ein weiterer Gegenstand der Erfindung ist die Verwendung von Katalysatoren, umfassend wenigstens einen Komplex eines Metalls der VIII. Nebengruppe mit wenigstens einer Verbindung der allgemeinen Formel I bzw. II, wie zuvor beschrieben, zur Hydroformylierung, Hydrocyanierung, Carbonylierung und zur Hydrierung.

Wie erwähnt stellt die Hydrocyanierung von Olefinen ein weiteres Einsatzgebiet für die erfindungsgemäßen Katalysatoren dar. Auch die erfindungsgemäßen Hydrocyanierungskatalysatoren umfassen Komplexe eines Metalls der VIII. Nebengruppe, insbesondere Cobalt, Nickel, Ruthenium, Rhodium, Palladium, Platin, bevorzugt Nickel, Palladium und Platin und ganz besonders bevorzugt Nickel. In der Regel liegt das Metall im erfindungsgemäßen Metallkomplex nullwertig vor. Die Herstellung der Metallkomplexe kann, wie bereits für den Einsatz als Hydroformylierungskatalysatoren zuvor beschrieben, erfolgen. Gleiches gilt für die in situ-Herstellung der erfindungsgemäßen Hydrocyanierungskatalysatoren.

Ein zur Herstellung eines Hydrocyanierungskatalysators geeigneter Nickelkomplex ist z. B. Bis(1,5-cyclooctadien)nickel(0).

Gegebenenfalls können die Hydrocyanierungskatalysatoren, analog zu dem bei den Hydroformylierungskatalysatoren beschriebenen Verfahren, in situ hergestellt werden.

Ein weiterer Gegenstand der Erfindung ist daher ein Verfahren zur Herstellung von Nitrilen durch katalytische Hydrocyanierung, in dem die Hydrocyanierung in Gegenwart mindestens eines der zuvor beschriebenen erfindungsgemäßen Katalysatoren erfolgt. Geeignete Olefine für die Hydrocyanierung sind allgemein die zuvor als Einsatzstoffe für die Hydroformylierung genannten Olefine. Eine spezielle Ausführungsform des erfindungsgemäßen Verfahrens betrifft die Herstellung von Gemischen monoolefinischer C₅-Mononitrile mit nichtkonjugierter C□ C- und C□ N-Bindung durch katalytische Hydrocyanierung von 1,3-Butadien oder 1,3-Butadien-haltigen Kohlenwasserstoffgemischen und die Isomerisierung/Weiterreaktion zu gesättigten C₄-Dinitrilen, vorzugsweise Adipodinitril in Gegenwart mindestens eines erfindungsgemäßen Katalysators. Bei der Verwendung von Kohlenwasserstoffgemischen zur Herstellung von monoolefinischer C₅-Mononitrilen nach dem erfindungsgemäßen Verfahren wird vorzugsweise ein Kohlenwasserstoffgemisch eingesetzt, das einen 1,3-Butadiengehalt von mindestens 10 Vol.-%, bevorzugt mindestens 25 Vol.-%, insbesondere mindestens 40 Vol.-%, aufweist.

1,3-Butadien-haltige Kohlenwasserstoffgemische sind in großtechnischem Maßstab erhältlich. So fällt z. B. bei der Aufarbeitung von Erdöl durch Steamcracken von Naphtha ein als C₄-Schnitt bezeichnetes Kohlenwasserstoffgemisch mit einem hohen Gesamtolefinanteil an, wobei etwa 40 % auf 1,3-Butadien und der Rest auf Monoolefine und mehrfach ungesättigte Kohlenwasserstoffe sowie Alkane entfällt. Diese Ströme enthalten immer auch geringe Anteile von im Allgemeinen bis zu 5 % an Alkinen, 1,2-Dienen und Vinylacetylen.

Reines 1,3-Butadien kann z. B. durch extraktive Destillation aus technisch erhältlichen Kohlenwasserstoffgemischen isoliert werden.

Die erfindungsgemäßen Katalysatoren lassen sich vorteilhaft zur Hydrocyanierung solcher olefinhaltiger, insbesondere 1,3-Butadien-haltiger Kohlenwasserstoffgemische einsetzen, in der Regel auch ohne vorherige destillative Aufreinigung des Kohlenwasserstoffgemischs. Möglicherweise enthaltene, die Effektivität der Katalysatoren beeinträchtigende Olefine, wie z. B. Alkine oder Cumulene, können gegebenenfalls vor der Hydrocyanierung durch selektive Hydrierung aus dem Kohlenwasserstoffgemisch entfernt werden. Geeignete Verfahren zur selektiven Hydrierung sind dem Fachmann bekannt.

Die erfindungsgemäße Hydrocyanierung kann kontinuierlich, semikontinuierlich oder diskontinuierlich erfolgen. Geeignete Reaktoren für die kontinuierliche Umsetzung sind dem Fachmann bekannt und werden z. B. in Ullmanns Enzyklopädie der technischen Chemie, Band 1, 3. Auflage, 1951, S. 743 ff. beschrieben. Vorzugsweise wird für die kontinuierliche Variante des erfindungsgemäßen Verfahrens eine Rührkesselkaskade oder ein Rohrreaktor verwendet. Geeignete, gegebenenfalls druckfeste Reaktoren für die semikontinuierliche oder kontinuierliche Ausführung sind dem Fachmann bekannt und werden z. B. in Ullmanns Enzyklopädie der technischen Chemie, Band 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die erfindungsgemäßen Hydrocyanierungskatalysatoren lassen sich nach üblichen, dem Fachmann bekannten Verfahren vom Austrag der Hydrocyanierungsreaktion abtrennen und können im Allgemeinen erneut für die Hydrocyanierung eingesetzt werden.

Ein weiterer Gegenstand der Erfindung ist ein Verfahren zur Carbonylierung von Verbindungen, die wenigstens eine ethylenisch ungesättigte Doppelbindung enthalten, durch Umsetzung mit Kohlenmonoxid und wenigstens einer Verbindung mit einer nucleophilen Gruppe in Gegenwart eines Carbonylierungskatalysators, in dem man als Carbonylierungskatalysator einen Katalysator auf Basis eines Pnicögenchelat-Liganden der allgemeinen Formel I bzw. II einsetzt.

Auch die erfindungsgemäßen Carbonylierungskatalysatoren umfassen Komplexe eines Metalls der VIII. Nebengruppe, bevorzugt Nickel, Cobalt, Eisen, Ruthenium, Rhodium und Palladium, insbesondere Palladium. Die Herstellung der Metallkomplexe kann wie bereits zuvor bei den Hydroformylierungskatalysatoren und Hydrocyanierungskatalysatoren beschrieben erfolgen. Gleiches gilt für die in situ-Herstellung der erfindungsgemäßen Carbonylierungskatalysatoren.

Geeignete Olefine für die Carbonylierung sind die allgemein zuvor als Einsatzstoffe für die Hydroformylierung und Hydrocyanierung genannten Olefine.

Vorzugsweise sind die Verbindungen mit einer nucleophilen Gruppe, ausgewählt unter Wasser, Alkoholen, Thiolen, Carbonsäureestern, primären und sekundären Aminen.

Eine bevorzugte Carbonylierungsreaktion ist die Überführung von Olefinen mit Kohlenmonoxid und Wasser zu Carbonsäuren (Hydrocarboxylierung). Dazu zählt insbesondere die Umsetzung von Ethylen mit Kohlenmonoxid und Wasser zu Propionsäure.

Ein weiterer Gegenstand der Erfindung ist die Verwendung von Katalysatoren, umfassend eine erfindungsgemäße P-, As- oder Sb-haltige Verbindung, wie zuvor beschrieben, zur Hydroformylierung, Hydrocyanierung, Carbonylierung, Hydrierung, Olefinoligomerisierung und -polymerisierung und zur Metathese.

Die Erfindung wird anhand der folgenden, nicht einschränkenden Beispiele näher erläutert.

### Beispiele

Es wurden die folgenden Liganden eingesetzt:

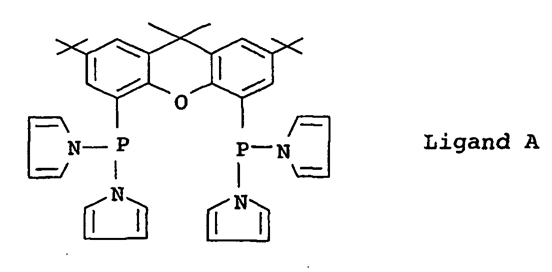

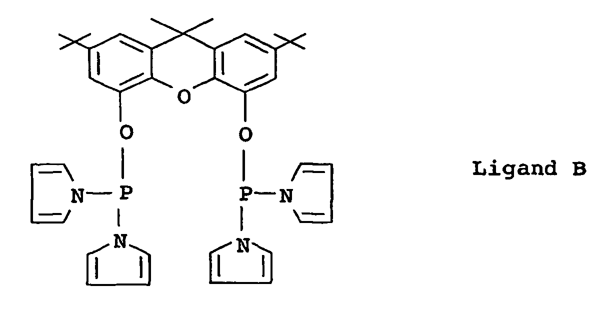

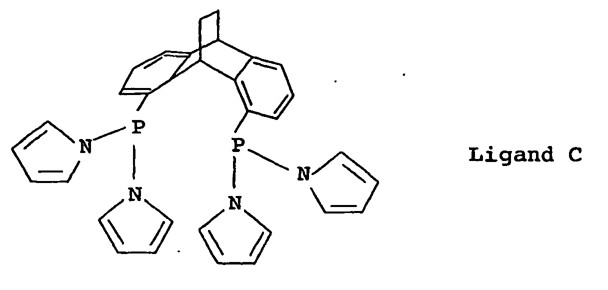

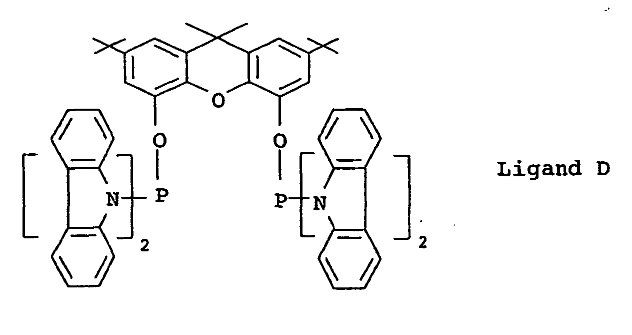

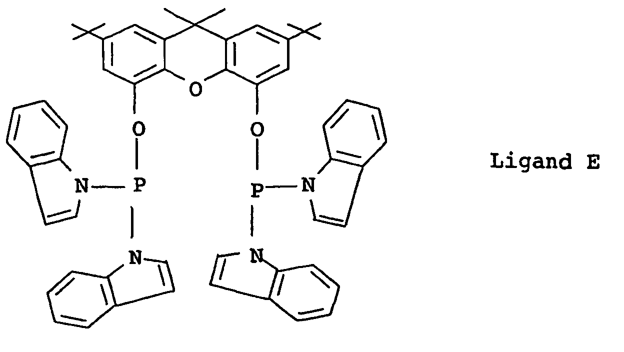

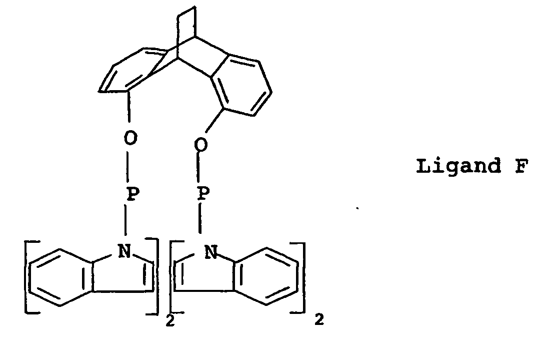

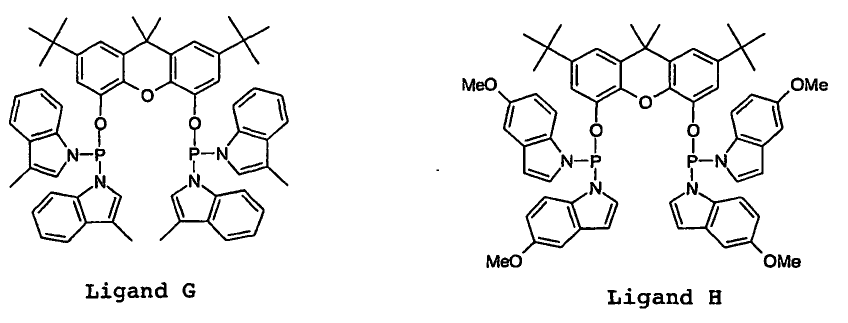

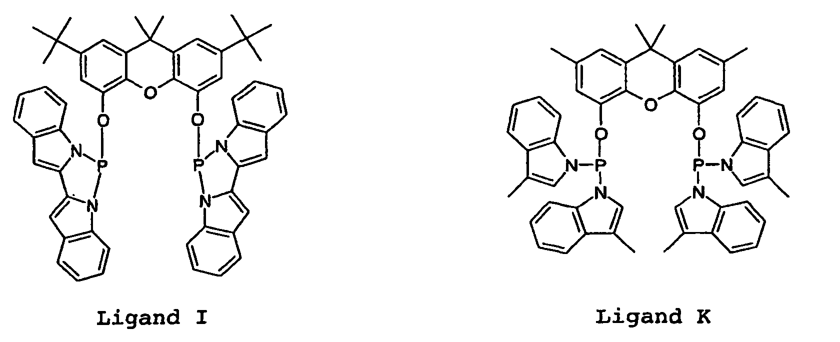

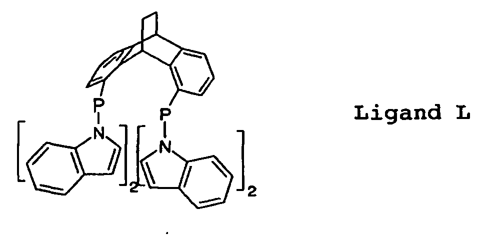

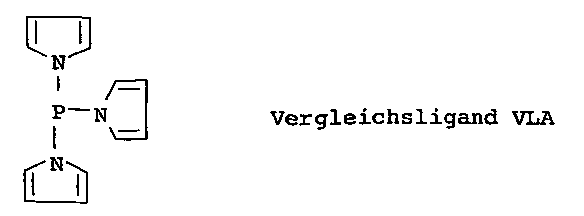

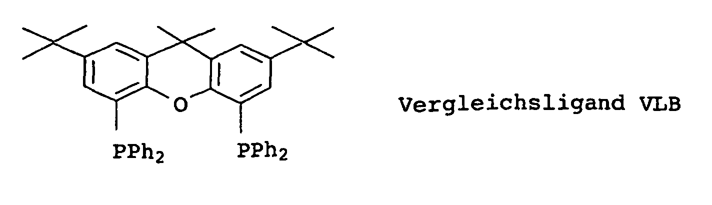

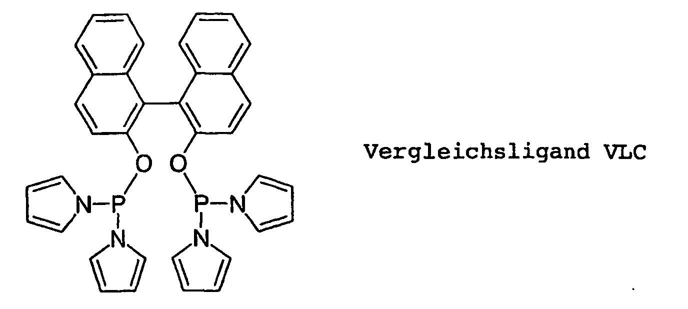

### Beispiel 1

### Synthese von Ligand A

5,2 g (38 mmol) PCl₃ und 5,1 g (76 mmol) Pyrrol wurden in 200 ml getrocknetem Tetrahydrofuran (THF) unter Argon bei -65 °C vorgelegt. 11,5 g (115 mmol) Triethylamin wurden zu diesem Gemisch gegeben und die Reaktionsmischung über Nacht bei Raumtemperatur gerührt. Der entstandene Niederschlag wurde unter vermindertem Druck vom flüssigem Reaktionsgemisch abfiltriert und überschüssiges THF und Triethylamin bei 60 °C Übergangstemperatur abdestilliert. Der Rückstand wurde in 50 ml Toluol aufgenommen und dabei ausgefallenes restliches Triethylamin-Hydrochlorid abfiltriert. Die erhaltene Lösung wurde bis zur weiteren Verarbeitung aufbewahrt.

5 g (13,3 mmol) 1,8-Dibrom-3,6-di-tert.-butyl-xanthen wurden in 150 ml getrocknetem THF bei -50 °C unter Argon vorgelegt. Es wurden 12,3 ml (30,6 mmol) einer 2,5-molaren Lösung von n-Butyllithium in Hexan zugetropft und nach Zugabe noch 2 h bei -50 °C gerührt, wobei sich ein Niederschlag bildete. Anschließend wurde diese Lösung bei -70 °C zur vorher hergestellten Toluollösung gegeben. Die Temperatur der exothermen Reaktion wurde durch Kühlen unterhalb von -50 °C gehalten. Nach 1 h Rühren wurde die Reaktionsmischung bei vermindertem Druck eingeengt, der Rückstand in Dichlormethan aufgenommen, dann erneut eingeengt und nach Zugabe von Hexan gerührt. Der entstandene Feststoff wurde zweimal aus heißem Methanol umkristallisiert. Reiner Ligand A wurde in einer Ausbeute von 1,1 g (13 % der Theorie) erhalten.

### Beispiel 2

### Synthese von Ligand B

5 g (13,3 mmol) 1,8-Dibrom-3,6-di-tert-butylxanthen wurden unter Argon in THF vorgelegt. Bei -50 °C wurden 12,3 ml (30,7 mmol) einer 2,5-molaren n-Butyllithium in Hexanlösung zugegeben. Die Reaktionsmischung wurde 2 h gerührt, wobei eine farblose Suspension entstand. Bei 0 °C wurden 100 ml (100 mmol) einer 1-molaren Lösung von BH₃ in THF zugetropft und die Mischung über Nacht bei 5 bis 15 °C gerührt. Anschließend wurde bei 0 °C mit 25 ml Wasser vorsichtig hydrolysiert. Zur Reaktionsmischung wurde 3 N KOH (aus 7,4 g KOH in 32 g H₂O) und 10,4 g 30%ige H₂O₂-Lösung vorsichtig zugegeben und 2 h am Rückfluss erhitzt. Nach Abkühlen auf. Raumtemperatur wurde mit 18%iger Salzsäure neutralisieret. Nach Phasentrennung wurde die organische Phase zweimal mit NaCl-Lösung gewaschen und über Na₂SO₄ getrocknet und zur Trockene eingeengt. 1,8-Dihydroxy-3,6-di-tert-butylxanthen wurde in 4,7 g Ausbeute (100 % d. Th.) erhalten.

5,2 g (38 mmol) PCl₃ und 5,1 g (76 mmol) Pyrrol wurden unter Argon in THF bei -65 °C vorgelegt. Anschließend wurden langsam 1,5 g (114 mmol) Triethylamin zugegeben und 48 h bei Raumtemperatur gerührt. 4,7 g (13,3 mmol) 1,8-Dihydroxy-3,6-di-tert-butylxanthen in 50 ml THF wurden hierzu bei Raumtemperatur zugegeben, wobei die Temperatur auf 30 °C stieg. Nach Rühren über Nacht wurde der entstandene Feststoff unter vermindertem Druck abfiltriert, der Niederschlag mit THF gewaschen und die vereinigten organischen Phasen eingeengt, wobei ein braunes Öl entstand. Das Öl wurde dreimal aus Hexan umkristallisiert. Ligand B wurde in 1,1 g Ausbeute (12 % d. Th.) erhalten.

### Beispiel 3 (Vergleichsbeispiel)

### Synthese von Ligand VLA (Vergleichsligand) und Lagerung bei Raumtemperatur

Vergleichsligand VLA wurde gemäß K.G. Moloy et al., J. Am. Chem. Soc. 117, S. 7696-7710 (1995) hergestellt. Die Synthese führt zu sauberem Produkt mit einer ³¹P-NMR-Verschiebung von +79 ppm (C₆D₆). Nach Lagerung der Verbindung unter Argon für fünf Tage bei Raumtemperatur konnte eine merkliche Dunkelfärbung festgestellt werden. Nach acht Wochen bildete sich eine teerartige Verbindung, die in der Katalyse nicht mehr eingesetzt werden konnte.

### Beispiel 4

### Synthese von Ligand C

Gemäß Maverick et al. (Inorg. Chem. 36, 5826 (1997)) wurden 66,7 g (240 mmol) 1,8-Dichloranthrachinon, 113,3 g (1,12 mol) KBr, 3,3 g (50 mmol) CuCl₂ und 113,4 ml 85%ige Phosphorsäure in 500 ml Nitrobenzol suspendiert und das Wasser bei 200 °C abdestilliert. Anschließend wurde 72 Stunden unter Rückfluss erhitzt. Nach Abkühlen wurde der feste Austrag mit Methanol und Dichlormethan aufgenommen, zur Trockene eingeengt und mit Aceton gewaschen. Es wurde eine 8:1-Mischung 1,8-Dibromanthrachinon:1-Brom-8-Chloranthrachinon in einer Ausbeute von 46 g (60 % d. Th.) erhalten.

23,6 g (65 mmol) 1,8-Dibromanthrachinon wurden in 250 ml Methanol suspendiert. Bei 10 °C wurden 9,9 g (260 mmol). NaBH₄ langsam zugegeben und anschließend 2 h bei 0 °C gerührt. Die Mischung wurde auf 800 ml Eis gegossen, gerührt, und bei vermindertem Druck filtriert. Der Feststoff wurde anschließend in 400 ml 18%iger Salzsäure suspendiert und 5 h bei 70 °C gerührt, anschließend wieder unter vermindertem Druck abfiltriert, neutral gewaschen und getrocknet. Der Feststoff wurde mit 14,8 g NaBH₄ in 250 ml Isopropanol bei 85 °C (Rückfluss) 3 h gerührt, mit verdünnter Salzsäure auf pH 7 gebracht, abgesaugt und mit Wasser gewaschen. 1,8-Dibromanthracen wurde in einer Ausbeute von 16,2 g (74 % d. Th.) erhalten.

100 g (298 mmol) 1,8-Dibromanthracen und 0,63 g (4 mmol) p-tert-Butylcatechol wurden in 800 g Toluol gelöst und innerhalb 5 min im Autoklaven mit 45 bar Ethen begast. Anschließend wurde bei 150 °C 3 Tage gerührt. Der Reaktionsaustrag wurde mit 10%iger Natronlauge extrahiert und mit destilliertem Wasser gewaschen. Die organische Phase wurde abgetrennt und eingeengt. Der Rückstand wurde zweimal aus Methanol umkristallisiert. 1,8-Dibromethanoanthracen wurde in einer Ausbeute von 60 g (55 % d. Th.) erhalten.

5,2 g (38 mmol) PCl₃ und 5,1 g (76 mmol) Pyrrol wurden unter Argon bei -78 °C in trockenem Tetrahydrofuran vorgelegt. 11,5 g (114 mmol) Triethylamin wurden zugegeben und die Mischung über Nacht bei Raumtemperatur gerührt. Der entstandene Niederschlag wurde abfiltriert und überschüssiges Tetrahydrofuran und Triethylamin abdestilliert. Der Rückstand wurde in Toluol aufgenommen und filtriert. Die Lösung wurde aufbewahrt.

4,8 g (13,3 mmol) 1,8-Dibromethanoanthracen wurden in 150 ml trockenem Tetrahydrofuran bei -50 °C vorgelegt, anschließend wurden 12,3 ml einer 2,5-molaren n-Butyllithium-Lösung in Hexan (30,6 mmol) zugegeben und 2 h bei -50 °C gerührt. Bei -70 °C wurde diese Mischung zur zuvor hergestellten Lösung von Bis-pyrrolyl-phosphorchlorid in Toluol gegeben, wobei die Temperatur auf -50 °C anstieg. Nach 1 h Rühren wurde eingeengt und der Feststoff in Dichlormethan aufgenommen, filtriert, das Filtrat eingeengt und mit Hexan gerührt. Das Produkt wurde aus heißem Methanol umkristallisiert. Ligand C wurde in einer Ausbeute von 1,6 g (23 % d. Th.) erhalten.

### Beispiel 5

### Hydroformylierung eines Buten-/Butangemisches mit Ligand A

3,0 mg Rh(CO)₂acac und 75 mg Ligand A (57 ppm Rh, Ligand/Rhodium-Verhältnis = 10/1) wurden separat eingewogen, in je 5 g Toluol gelöst, vermischt und bei 120 °C mit 10 bar Synthesegas (CO:H₂ = 1:1) begast. Nach 30 min wurde entspannt, dann wurden 11 g Buten-/Butangemisch) (45 % 1-Buten, 40 % 2-Buten, 15 % Butane) zugepresst und mittels CO/H₂ (1:1) ein Reaktionsdruck von 20 bar bei 120 °C eingestellt. Nach 4 h wurde der Autoklav über eine Kühlfalle entspannt und beide Reaktionsausträge (Reaktor und Kühlfalle) mittels Gaschromatographie analysiert. Der Umsatz an Butenen betrug 47 %, die Aldehydselektivität 96 % und die Linearität 95 %. (Linearität: Quotient aus n-Aldehyd zu allen gebildeten Aldehyden x 100)

### Beispiel 6

### Hydroformylierung eines Buten-/Butangemisches mit Ligand B

3,0 mg Rh(CO)₂acac und 79 mg Ligand B (57 ppm Rh, Ligand/Rhodium = 10/1) wurden separat eingewogen, in je 5 g Toluol gelöst, vermischt und bei 120 °C mit 10 bar Synthesegas (CO:H₂ = 1:1) begast. Nach 30 min wurde entspannt, dann wurden 11 g Buten-/Butangemisch (45 % 1-Buten, 40 % 2-Buten, 15 % Butane) zugepresst und 4 h bei 120 °C und 20 bar hydroformyliert. Der Umsatz an Butenen betrug 58 %, die Aldehydselektivität 97 % und die Linearität 94 %.

### Beispiel 7

### Hydroformylierung eines Buten-/Butangemisches mit Ligand B

6,0 mg Rh(CO)₂acac und 161 mg Ligand B (112 ppm Rh, Ligand/Rh = 10/1) wurden separat eingewogen, in je 7,5 g Toluol gelöst, vermischt und bei 100 °C mit 10 bar Synthesegas (CO:H₂ = 1/2) begast. Nach 30 min wurde entspannt, dann wurden 6,2 g Buten-/Butangemisch (45 % 1-Buten, 40 % 2-Buten, 15 % Butane) zugepresst und 4 h bei 100 °C und 22 bar hydroformyliert. Der Umsatz an Butenen betrug 85 %, die Aldehydselektivität 89 % und die Linearität 99 %.

### Beispiel 8

### Hydroformylierung von 2-Buten mit Ligand B

3,6 mg Rh(CO)₂acac und 162 mg Ligand B (116 ppm Rh, Ligand/Rhodium = 10/1) wurden separat eingewogen, in je 7,5 g Toluol gelöst, vermischt und bei 100 °C mit 10 bar Synthesegas (CO:H₂ = 1:2) begast. Nach 30 min wurde entspannt, dann wurden 5,5 g 2-Buten zugepresst und 4 h bei 100 °C und 22 bar hydroformyliert. Der Umsatz an 2-Buten betrug 79 %, die Aldehydselektivität 92 % und die Linearität 95 %.

### Beispiel 9

### Hydroformylierung von 1-Octen mit Ligand B

0,8 mg Rh(CO)₂acac und 20,7 mg Ligand B (60 ppm Rh, Ligand/Rhodium = 11/1) wurden separat eingewogen, in je 1,3 g Toluol gelöst, vermischt und bei 100 °C mit 10 bar Synthesegas (CO:H₂ = 1:1) begast. Nach 30 min wurde entspannt, dann wurden 2,5 g 1-Octen zugegeben und 4 h bei 100 °C und 10 bar hydroformyliert. Der Umsatz betrug 98 %, die Aldehydselektivität 83 % und die Linearität 98 %. Der α-Anteil (n-Nonanal + iso-Nonanal) betrug 100 %.

### Beispiel 10 (Vergleichsbeispiel)

### Hydroformylierung von 1-Octen mit Vergleichsligand VLA

0,9 mg Rh(CO)₂acac und 8 mg Vergleichsligand VLA (60 ppm Rh, Ligand/Rhodium = 10/1) wurden separat eingewogen, in je 1,5 g Xylol gelöst, vermischt und bei 100 °C mit 10 bar Synthesegas (CO:H₂ = 1:1) begast. Nach 30 min wurde entspannt, dann wurden 3,0 g 1-Octen zugegeben und 4 h bei 80 °C und 10 bar hydroformyliert. Der Umsatz betrug 79 %, die Aldehydselektivität 81 % und die Linearität 83 %. Der α-Anteil (n-Nonanal + iso-Nonanal) betrug 99 %.

### Beispiel 11

### Hydroformylierung von 1-Octen mit Ligand C

0,9 mg Rh(CO)₂acac und 18,5 mg Ligand C (60 ppm Rh, Ligand/Rhodium = 10/1) wurden separat eingewogen, in je 1,5 g Diphenylether gelöst, vermischt und bei 100 °C mit 10 bar Synthesegas (CO:H₂ = 1:1) begast. Nach 60 min wurde entspannt, dann wurden 3,0 g 1-Octen zugegeben und 4 h bei 100 °C und 10 bar hydroformyliert. Die Linearität betrug 98 % und der α-Anteil (n-Nonanal + iso-Nonanal) 100 %.

### Beispiel 12

### Hydroformylierung von 2-Octen mit Ligand C

6 mg Rh(CO)₂acac und 124 mg Ligand C (119 ppm Rh, Ligand/Rhodium = 10/1) wurden separat eingewogen, in je 5 g Toluol gelöst, vermischt und bei 100 °C mit 10 bar Synthesegas (CO:H₂ = 1:2) begast. Nach 30 min wurde entspannt, dann wurden 10 g 2-Octen zugegeben und 4 h bei 100 °C und 10 bar hydroformyliert. Die Linearität betrug 99 % und der α-Anteil (n-Nonanal + iso-Nonanal) 100 %.

### Beispiel 13 (Vergleichsbeispiel)

### Hydroformylierung von 2-Octen mit Vergleichsligand VLA

0,9 mg Rh(CO)₂acac und 8 mg Vergleichsligand VLA (60 ppm Rh, Ligand/Rhodium = 10/1) wurden separat eingewogen, in je 1,5 g Xylol gelöst, vermischt und bei 100 °C mit 10 bar Synthesegas (CO:H₂ = 1:1) begast. Nach 30 min wurde entspannt, dann wurden 3,0 g 2-Octen zugegeben und 4 h bei 100 °C und 10 bar hydroformyliert. Die Umsatz betrug 74 %, die Aldehydselektivität 44 % und die Linearität 51 %. Der α-Anteil (n-Nonanal + iso-Nonanal) betrug 85 %.

### Beispiel 14 (Vergleichsbeispiel)

### Hydroformylierung eines Buten-/Butan-Gemisches mit Vergleichsligand VLB

6 mg Rh(CO)₂acac und 96,5 mg Vergleichsligand VLB (102 ppm Rh, Ligand/Rhodium = 6/1) wurden separat eingewogen, in je 5 g Xylol gelöst, vermischt und bei 110 °C mit 20 bar Synthesegas (CO:H₂ = 1:1) begast. Nach 30 min wurde entspannt, dann wurden 13,4 g Buten-/Butan-Gemisch (45 % 1-Buten, 40 % 2-Buten, 15 % Butane) zugegeben und 3,5 h bei 110 °C und 20 bar hydroformyliert. Der Umsatz an Butenen betrug 50 %, die Aldehydselektivität 100 % und die Linearität 95 %. Das nicht umgesetzte Olefin bestand ausschließlich aus 2-Buten, d. h. aus dem Gemisch wurde nur das 1-Buten umgesetzt.

Vergleichsligand VLB wurde gemäß Angew. Chem. 111, 349 (1999) hergestellt.

### Beispiel 15 (Synthese von Ligand D)

5,2 g (38 mmol) PCl₃ und 12,7 g (76 mmol) Carbazol wurden in 200 ml getrocknetem Tetrahydrofuran (THF) unter Argon bei -65 °C vorgelegt. 11,5 g (115 mmol) Triethylamin wurden zu diesem Gemisch gegeben und die Reaktionsmischung über Nacht bei Raumtemperatur gerührt. Der entstandene Niederschlag wurde unter vermindertem Druck vom flüssigem Reaktionsgemisch abfiltriert und überschüssiges THF und Triethylamin bei 60 °C Übergangstemperatur abdestilliert. Der Rückstand wurde in 50 ml Toluol aufgenommen und dabei ausgefallenes restliches Triethylamin-Hydrochlorid abfiltriert. Die erhaltene Lösung wurde bis zur weiteren Verarbeitung aufbewahrt.

5 g (13,3 mmol) 1,8-Dibrom-3,6-di-tert.-butyl-xanthen wurden in 150 ml getrocknetem THF bei -50 °C unter Argon vorgelegt. Es wurden 12,3 ml (30,6 mmol) einer 2,5-molaren Lösung von n-Butyllithium in Hexan zugetropft und nach Zugabe noch 2 h bei -50 °C gerührt, wobei sich ein Niederschlag bildete. Anschließend wurde diese Lösung bei -70 °C zur vorher hergestellten Toluollösung gegeben. Die Temperatur der exothermen Reaktion wurde durch Kühlen unterhalb von -50 °C gehalten. Nach 1 h Rühren wurde die Reaktionsmischung bei vermindertem Druck eingeengt, der Rückstand in Dichlormethan aufgenommen, dann erneut eingeengt und nach Zugabe von Hexan gerührt. Der entstandene Feststoff wurde zweimal aus heißem Methanol umkristallisiert. Reiner Ligand D, der im ³¹P_{-NMR} (CDCl₃) ein Singulett bei +125 ppm zeigte.

### Beispiel 16 (Vergleichsbeispiel)

### Lagerung von Vergleichsligand VLC bei Raumtemperatur

Vergleichsligand VLC wurde gemäß US 5,710,344 hergestellt. Die Synthese führt zu sauberem Produkt mit einer ³¹P-NMR-Verschiebung von +69 ppm (C₆D₆). Nach Lagerung der Verbindung unter Argon für 10 Tage bei Raumtemperatur konnte eine merkliche Dunkelfärbung festgestellt werden. Eine ³¹P-NMR-Untersuchung zeigte einen Ligandabbau von 20 %.

### Beispiel 17 (Vergleichsbeispiel)

### Hydroformylierung von 1-Octen vor Lagerung bei Raumtemperatur

1,6 mg Rh(CO)₂acac (Rhodiumbiscarbonylacetylacetonat) und 36,9 mg Vergleichsligand VLC (106 ppm Rh, Ligand:Metall = 10/1) wurden separat eingewogen, in je 1,5 g Palatinol-AH® (Phthalsäureester von 2-Ethylhexanol der BASF Aktienges.) gelöst, vermischt und in einem 100 ml-Autoklaven bei 100 °C mit 10 bar Synthesegas (CO:H₂ = 1:1) begast. Nach 30 min wurde entspannt, dann wurden 3 g 1-Octen zugegeben und 4 h bei 100 °C und 10 bar hydroformyliert. Der Umsatz betrug 98 %, die Aldehydselektivität 59 % und die Linearität 99 %. Die Selektivität zu internen Octenen betrug 41 %.

### Beispiel 18 (Vergleichsbeispiel)

### Hydroformylierung von 1-Octen nach Lagerung bei Raumtemperatur

1,6 mg Rh(CO)₂acac und 36.9 mg Vergleichsligand VLC (106 ppm Rh, Ligand:Metall = 10/1) wurden separat eingewogen, in je 1,5 g Palatinol-AH® gelöst, vermischt und bei 100 °C mit 10 bar Synthesegas (CO:H₂ = 1:1) begast. Nach 30 min wurde entspannt, dann wurden 3 g 1-Octen mit der Spritze zugegeben und 4 h bei 100 °C und 10 bar hydroformyliert. Der Umsatz betrug 20 %, die Aldehydselektivität 5 % und die Linearität 71 %. Die Selektivität zu internen Octenen betrug 95 %.

### Beispiel 19

### Hydroformylierung von 2-Buten mit Ligand D

6,0 mg Rh(CO)₂acac und 238 mg Ligand D (117 ppm Rh, Ligand:Metall = 9/1) wurden separat eingewogen, in je 7,5 g Toluol gelöst, vermischt und bei 100 °C mit 10 bar Synthesegas (CO:H₂ = 1:2) begast. Nach 30 min wurde entspannt, dann wurden 5,3 g 2-Buten zugepresst und 4 h bei 100 °C und 22 bar hydroformyliert. Der Umsatz betrug 25 %, die Aldehydselektivität 92 % und die Linearität 45 %.

Eine ³¹P-NMR-Untersuchung des Reaktionsaustrages zeigte keine Abbauprodukte des Liganden. Der Reaktionsaustrag war ein gelbe homogene Lösung. Bei Ligand B wurde nach der Hydroformylierung ein roter Niederschlag beobachtet; bei den substituierten Pyrrolen konnte ein roter Niederschlag nicht festgestellt werden.

### Beispiel 20

### Versuche zur Ligandstabilität von Ligand D

Ligand D wurde in CH₂Cl₂ gelöst und mit warmem Wasser in Gegenwart von Luft gewaschen. Es konnte keine Farbveränderung festgestellt werden. Eine ³¹P-NMR-Untersuchung zeigte keine Abbauprodukte. Anschließend wurde der Ligand für vier Wochen bei Raumtemperatur unter Lichteinfluss gelagert. Es konnte auch hier keine Farbveränderung festgestellt werden. Eine ³¹P-NMR-Untersuchung zeigte keine Abbauprodukte.

### Beispiel 21

### Hydroformylierung von 3-Hexen mit Ligand E

5,4 mg Rh(CO)₂acac und 174 mg Ligand E (107 ppm Rh, Ligand:Metall = 9/1) wurden separat eingewogen, in je 7,5 g Toluol gelöst, vermischt und bei 100 °C mit 10 bar Synthesegas (CO:H₂ = 1:2) begast. Nach 30 min wurde entspannt, dann wurden 5 g 3-Hexen zugepresst und 4 h bei 120 °C und 12 bar hydroformyliert (CO:H₂ = 1:1). Der Umsatz betrug 58 %, die Aldehydselektivität 87 % und die Linearität 98 %.

### Beispiel 22

### Hydroformylierung von 1-Octen mit Ligand E

5,1 mg Rh(CO)₂acac und 342 mg Ligand E (100 ppm Rh, Ligand:Metall = 20/1) wurden separat eingewogen, in je 5 g Palatinol-AH® gelöst, vermischt und bei 100 °C mit 10 bar Synthesegas (CO:H₂ = 1:2) begast. Nach 60 min wurde entspannt, dann wurden 10 g 1-Octen zugepresst und 2 h bei 120 °C und 20 bar hydroformyliert (CO:H₂ = 1:1). Der Umsatz betrug 100 %, die Aldehydselektivität 79 % und die Linearität 95 %.

### Beispiel 23

### Hydroformylierung von Buten-/Butangemisch mit Ligand E

5,0 mg Rh(CO)₂acac und 170 mg Ligand E (94 ppm Rh, Ligand:Metall = 10/1) wurden separat eingewogen, in je 6 g Toluol gelöst, vermischt und bei 120 °C mit 10 bar Synthesegas (CO:H₂ = 1:2) begast. Nach 30 min wurde entspannt, dann wurden 9 g Buten-/Butangemisch (45 % 1-Buten, 40 % 2-Buten, 15 % Butane) zugepresst und 4 h bei 120 °C und 17 bar hydroformyliert (CO:H₂ = 1:1). Der Umsatz an Butenen betrug 91 %, die Aldehydselektivität 100 % und die Linearität 91 %.

Eine ³¹P-NMR-Untersuchung des Reaktionsaustrages zeigte keine Abbauprodukte des Liganden. Der Reaktionsaustrag war ein gelbe homogene Lösung. Ein roter Niederschlag konnte nicht festgestellt werden. Der Versuch wurde im selben Autoklaven wiederholt und lieferte dasselbe Ergebnis.

### Beispiel 24

### Versuche zur Ligandstabilität von Ligand E

Ligand E wurde in CH₂Cl₂ gelöst und mit warmem Wasser in Gegenwart von Luft gewaschen. Es konnte keine Farbveränderung festgestellt werden. Eine ³¹P-NMR-Untersuchung zeigte keine Abbauprodukte. Anschließend wurde der Ligand für vier Wochen bei Raumtemperatur unter Lichteinfluss gelagert. Es konnte auch hier keine Farbveränderung festgestellt werden. Eine ³¹P-NMR-Untersuchung zeigte keine Abbauprodukte.

### Beispiel 25

### Reaktion von Ligand E mit Säuren

In einer Stickstoff-Glove-Box wurde Ligand E in Toluol mit Rh(CO)₂acac und wässriger H₃PO₄ 4 h auf 100 °C erhitzt. Anschlie-ßend wurde von der gelben, homogenen Reaktionsmischung ein ³¹P-NMR gemessen. Es konnten keine Spuren von Abbauprodukten festgestellt werden.

### Beispiel 26

### Reaktion von Ligand E mit Luft in Gegenwart von Katalysatoren

Ligand E wurde mit Rh(CO)₂acac in Toluol gelöst und über Nacht bei Raumtemperatur an Luft gerührt. Eine ³¹P-NMR-Untersuchung zeigte, dass der Ligand zu 100 % zum Dioxid oxidiert war. Anschließend wurde H₃PO₄ zugesetzt und erneut über Nacht bei Raumtemperatur gerührt. Eine ³¹P-NMR-Untersuchung zeigte, dass das Dioxid vollständig unverändert vorlag. Aus diesem Versuch ist ersichtlich, dass Ligand E zwar wie alle Phosphor-Liganden in Gegenwart von Metallen und Luft oxidiert werden kann, dass die gebildeten oxide jedoch nicht zu störenden Abbauprodukten weiter reagieren.

### Beispiel 27

### Hydroformylierung von Buten-/Butangemisch mit Ligand F

Ligand F zeigt im ³¹P-NMR (C₆D₆) ein Singulett bei +105 ppm.

5,9 mg Rh(CO)₂acac und 158 mg Ligand E (106 ppm Rh, Ligand:Metall = 10/1) wurden separat eingewogen, in je 7,5 g Toluol gelöst, vermischt und bei 100 °C mit 10 bar Synthesegas (CO:H₂ = 1:2) begast. Nach 60 min wurde entspannt, dann wurden 5,9 g Buten-/Butangemisch (45 % 1-Buten, 40 % 2-Buten, 15 % Butane) zugepresst und 4 h bei 120 °C und 20 bar hydroformyliert (CO:H₂ = 1:1). Der Umsatz an Butenen betrug 87 %, die Aldehydselektivität 90 % und die Linearität 94 %.

Eine ³¹P-NMR-Untersuchung des Reaktionsaustrages zeigte keine Abbauprodukte des Liganden. Der Reaktionsaustrag war ein gelbe homogene Lösung. Ein roter Niederschlag konnte nicht festgestellt werden. Der Versuch wurde im selben Autoklaven wiederholt und lieferte dasselbe Ergebnis.

### Beispiel 28

### Kontinuierliche Hydroformylierung von 1-Octen mit Ligand E

In einer kontinuierlich betriebenen Apparatur bestehend aus Autoklav, Druckabscheider, Wischblattverdampfer (betrieben bei 140 °C) und Mischbehälter wurde 1-Octen bei einem Druck von 20 bar und einer Temperatur von 120 °C (100 ppm Rh, Ligand-Metall-Verhältnis = 20:1) mit Synthesegas (CO:H₂ = 1:2) hydroformyliert. Dabei wurde über einen Zeitraum von 10 Tagen im Mittel folgendes Ergebnis erhalten: 61 % Umsatz, 60 % Aldehydselektivität, 84 % Linearität. Über den gesamten Zeitraum wurden weder Rhodium noch Ligand ergänzt. Nach Beendigung des Versuchs lag eine homogene, hellgelbe Lösung vor. Unspezifische Ligandzersetzung konnte nicht nachgewiesen werden.

### Beispiel 29

### Synthese von Ligand G

15,6 g (118,9 mmol) 3-Methylindol (Ligand G) wurden bei Raumtemperatur in 400 g Toluol gelöst und auf -75 °C abgekühlt. Anschließend wurden zunächst 56,9 g (563,4 mmol) Triethylamin und dann 8,14 g (59,4 mmol) PCl₃ mittels einer Spritze zugegeben. Die Mischung wurde langsam auf Raumtemperatur erwärmt und anschließend 16 h unter Rückfluss gekocht. Danach wurden 13,7 g (38,8 mmol) Xanthendiol als Suspension in Toluol bei Raumtemperatur zugegeben und die Mischung über Nacht unter Rückfluss gekocht. Das entstandene Triethylaminhydrochlorid wurde abfiltriert und einmal mit Toluol nachgewaschen. Nach Einengen der organischen Phasen wurde der Rückstand in Dichlormethan aufgenommen und über eine 4 x 20 cm große Silica 60 Säule mit 300 ml Dichlormethan gewaschen. Die Waschlösung wurde eingeengt, dann wurden 100 ml Methanol zugefügt, wobei eine weiße klebrige Masse entstand, die vom Lösungsmittel abdekantiert wurde. Nach Rühren mit Pentan erhielt man das Produkt als feinen weißen Feststoff in 64 % Ausbeute. (³¹P-NMR: 105 ppm).

### Beispiel 30

### Hydroformylierung von Buten-/Butangemisch mit Ligand G

4,4 mg Rh(CO)₂acac und 156 mg Ligand G (98 ppm Rh, Ligand:Metall = 10/1) wurden separat eingewogen, in je 6,4 g Toluol gelöst, vermischt und bei 100 °C mit 10 bar Synthesegas (CO:H₂ = 1:2) begast. Nach 30 min wurde entspannt, dann wurden 4,9 g Buten-/Butangemisch (45 % 1-Buten, 40 % 2-Buten, 15 % Butane) zugepresst und 4 h bei 110 °C und 15 bar hydroformyliert (CO:H₂ = 1:1). Der Umsatz an Butenen betrug 96 %, die Aldehydselektivität 100 % und die Linearität 93 %.

### Beispiel 31

### Synthese von Ligand H

35,6 g (242 mmol) 5-Methoxyindol wurden bei Raumtemperatur in 1 l Toluol gelöst und auf -75 °C abgekühlt. Anschließend wurden zunächst 123 g (1222 mmol) Triethylamin und dann 16,5 g (120,4 mmol) PCl₃ mittels einer Spritze zugegeben. Die Mischung wurde langsam auf Raumtemperatur erwärmt und anschließend 16 h bei 90 bis 100 °C gerührt. Danach wurden 19 g (52,5 mmol) Xanthendiol als Suspension in Toluol bei Raumtemperatur zugegeben und die Mischung über Nacht bei 90 bis 100 °C gerührt. Das entstandene Triethylaminhydrochlorid wurde abfiltriert und einmal mit Toluol nachgewaschen. Nach Einengen der organischen Phasen wurde der Rückstand in Dichlormethan aufgenommen und über eine 4 x 20 cm große Silica 60 Säule dreimal mit je 250 ml Dichlormethan gewaschen. Die Waschlösung wurde eingeengt, dann wurden 100 ml Methanol zugefügt, wobei eine weiße klebrige Masse entstand, die vom Lösungsmittel abdekantiert wurde. Nach Rühren mit Pentan erhielt man das Produkt als feinen weißen Feststoff in 64 % Ausbeute. (³¹P-NMR: 108 ppm).

### Beispiel 32

### Hydroformylierung von Buten-/Butangemisch mit Ligand H

3,0 mg Rh(CO)₂acac und 61 mg Ligand H (116 ppm Rh, Ligand:Metall = 5/1) wurden separat eingewogen, in je 3,7 g Toluol gelöst, vermischt und bei 120 °C mit 10 bar Synthesegas (CO:H₂ = 1:2) begast. Nach 30 min wurde entspannt, dann wurden 3,0 g Buten-/Butangemisch (45 % 1-Buten, 40 % 2-Buten, 15 % Butane) zugepresst und 4 h bei 120 °C und 17 bar hydroformyliert (CO:H₂ = 1:1). Der Umsatz an Butenen betrug 98 %, die Aldehydselektivität 80 % und die Linearität 93 %.

### Beispiel 33

### Synthese von Ligand I

### a) Synthese von Bisindolyl:

Unter Stickstoff wurden 120 ml THF vorgelegt und 37 ml (425 mmol) Oxalylchlorid zugetropft. Nach Abkühlen auf 0 °C wurde in 1 h eine Lösung von 90 g (840 mmol) o-Toluidin und 117 ml (840 mmol) Triethylamin in 190 ml THF zugetropft. Es wurde 2 h bei 0 °C gerührt, dann ließ man auf Raumtemperatur unter Rühren erwärmen. Vorsichtig wurden ca. 600 ml Wasser, dann 900 ml Ethylacetat zugegeben und auf 100 °C erwärmt. Der entstandene Feststoff wurde abfiltriert und mit Petrolether gewaschen. Ausbeute: 65 %.

Zu 600 ml tert.-Butanol wurden unter Rühren 33,5 g (859 mmol) Kalium gegeben und bei 50 °C 4 h gerührt, bis das Kalium vollständig umgesetzt war. Anschließend wurden 46 g (172 mmol) N,N-Bis-o-tolyloxamid zugegeben. Im Sandbad wurde erhitzt, bei 88 °C destillierte das tert.-Butanol vollständig ab. Als das Solvens entfernt war, stieg die Temperatur auf 190 °C und ein weißer voluminöser Feststoff sublimierte ab. Die Reaktionstemperatur stieg danach auf 300 °C an und wurde über 1 h gehalten. Anschließend wurde auf Raumtemperatur abgekühlt. Vorsichtig wurden 300 ml Wasser zugegeben. Der Feststoff wurde abgesaugt und mit 200 ml Ethanol unter Rückfluss erhitzt. Anschließend wurde der Feststoff abgesaugt, mit Pentan gewaschen und getrocknet. Ausbeute: 50 %.

### b) Synthese von Ligand I:

8,8 g (38 mmol) Bisindolyl wurden bei -78 °C in 150 ml THF vorgelegt und mit 5,2 g (38 mmol) PCl₃ versetzt. Anschließend wurden 15 g (150 mmol) Triethylamin zugegeben, und die Mischung wurde über Nacht auf Raumtemperatur erwärmt. 6,7 g Xanthendiol wurden in 100 ml THF suspendiert, zur Reaktionsmischung hinzugegeben, und anschließend wurde die Mischung über Nacht am Rückfluss gekocht. Das Triethylaminhydrochlorid wurde abfiltriert, vom Filtrat das Lösungsmittel im Vakuum entfernt und der Rückstand in Dichlormethan aufgerührt. Der gebildete Feststoff wurde abfiltriert und getrocknet. Ausbeute: 40 %. (³¹P-NMR: 78 ppm).

### Beispiel 34

### Hydroformylierung von Buten-/Butangemisch mit Ligand I

5,1 mg Rh(CO)₂acac und 175 mg Ligand I (101 ppm Rh, Ligand:Metall = 10/1) wurden separat eingewogen, in je 9 g THF gelöst, vermischt und bei 100 °C mit 10 bar Synthesegas (CO:H₂ = 1:2) begast. Nach 30 min. wurde entspannt, dann wurden 2,5 g Buten-/Butangemisch (45 % 1-Buten, 40 % 2-Buten, 15 % Butane) zugepresst und 4 h bei 120 °C und 17 bar hydroformyliert (CO:H₂ = 1:1). Der Umsatz an Butenen betrug 40 %, die Aldehydselektivität 98 % und die Linearität 68 %.

### Beispiel 35

### Synthese von Ligand K

9 g (69 mmol) Ligand K wurden in Xylol gelöst und bei -20 °C mit 5,4 g (39 mmol) PCl₃ versetzt. Anschließend wurden langsam 20 ml (145 mmol) Triethylamin zugegeben, langsam auf Raumtemperatur erwärmt und über Nacht bei 130 °C gerührt. 6,6 g (17 mmol) Tetramethylxanthendiol wurden bei Raumtemperatur zugegeben, und die Mischung wurde 4 h gerührt, dann über Nacht bei 150 °C gekocht. Das entstandene Triethylaminhydrochlorid wurde abfiltriert, das Xylol im Vakuum entfernt und der Rückstand in Dichlormethan gelöst. Die Lösung wurde über Aktivkohle und Kieselgel gereinigt und das Lösungsmittel im Vakuum entfernt. Ausbeute: 37 %. (³¹P-NMR: 106 ppm)

### Beispiel 36

### Hydroformylierung von Buten-/Butangemisch mit Ligand K

5,1 mg Rh(CO)₂acac und 166 mg Ligand K (101 ppm Rh, Ligand:Metall = 10/1) wurden separat eingewogen, in je 7,5 g Toluol gelöst, vermischt und bei 100 °C mit 10 bar Synthesegas (CO:H₂ = 1:2) begast. Nach 30 min wurde entspannt, dann wurden 5,0 g Buten-/Butangemisch (45 % 1-Buten, 40 % 2-Buten, 15 % Butane) zugepresst und 4 h bei 120 °C und 17 bar hydroformyliert (CO:H₂ = 1:1). Der Umsatz an Butenen betrug 94 %, die Aldehydselektivität 98 % und die Linearität 90 %.

### Beispiel 37

### Hydroformylierung von Buten-/Butangemisch mit Ligand L

Die Synthese von Ligand L erfolgt analog zum Pyrrolderivat.

5,9 mg Rh(CO)₂acac und 171 mg Ligand L (116 ppm Rh, Ligand:Metall = 10/1) wurden separat eingewogen, in je 7,5 g Toluol gelöst, vermischt und bei 100 °C mit 10 bar Synthesegas (CO:H₂ = 1:2) begast. Nach 30 min wurde entspannt, dann wurden 5,9 g Buten-/Butangemisch (45 % 1-Buten, 40 % 2-Buten, 15 % Butane) zugepresst und 4 h bei 100 °C und 17 bar hydroformyliert (CO:H₂ = 1:1). Der Umsatz an Butenen betrug 16 %, die Aldehydselektivität 100 % und die Linearität 94 %.

## Patentansprüche

1. Pnicogenchelatliganden der allgemeinen Formel I 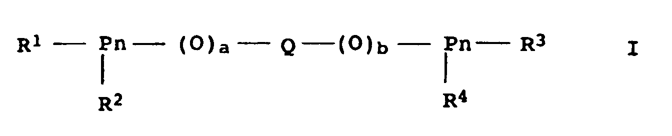 in der
Q eine Brückengruppe der Formel 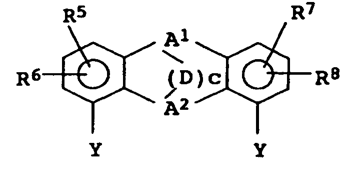
ist,
worin
A¹ und A² unabhängig voneinander für O, S, siR^{a}R^{b}, NR^{c} oder CR^{d}R^{e} stehen, wobei
R^{a}, R^{b} und R^{c} unabhängig voneinander für Wasserstoff, Alkyl, Cycloalkyl, Heterocycloalkyl, Aryl oder Hetaryl stehen,
R^{d} und R^{e} unabhängig voneinander für Wasserstoff, Alkyl, Cycloalkyl, Heterocycloalkyl, Aryl oder Hetaryl stehen oder die Gruppe R^{d} gemeinsam mit einer weiteren Gruppe R^{d} oder die Gruppe R^{e} gemeinsam mit einer weiteren Gruppe R^{e} eine intramolekulare Brückengruppe D bilden,
D eine zweibindige Brückengruppe, ausgewählt aus den Gruppen 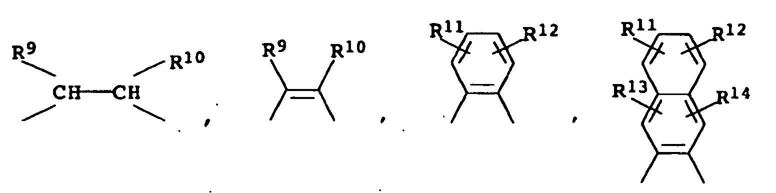
ist, in denen
R⁹ und R¹⁰ unabhängig voneinander für Wasserstoff, Alkyl, Cycloalkyl, Aryl, Halogen, Trifluormethyl, Carboxyl, Carboxylat oder Cyano stehen oder miteinander zu einer C₃- bis C₄-Alkylenbrücke verbunden sind,
R¹¹, R¹², R¹³ und R¹⁴ unabhängig voneinander für Wasserstoff, Alkyl, Cycloalkyl, Aryl, Halogen, Trifluormethyl, COOH, Carboxylat, Cyano, Alkoxy, SO₃H, Sulfonat, NE¹E², Alkylen-NE¹E²E³⁺X⁻, Acyl oder Nitro stehen,
c 0 oder 1 ist,
Y eine chemische Bindung darstellt,
R⁵, R⁶, R⁷ und R⁸ unabhängig voneinander für Wasserstoff, Alkyl, Cycloalkyl, Heterocycloalkyl, Aryl, Hetaryl, COOR^{f}, COO⁻M⁺, SO₃R^{f}, SO⁻₃M⁺, NE¹E², NE¹E²E³⁺X⁻, Alkylen-NE¹E²E³⁺X⁻, OR^{f}, SR^{f}, (CHR^{g}CH₂O)ₓR^{f}, (CH₂N(E¹))ₓR^{f}, (CH₂CH₂N(E¹))ₓR^{f}, Halogen, Trifluormethyl, Nitro, Acyl oder Cyano stehen,
worin
R^{f}, E¹, E² und E³ jeweils gleiche oder verschiedene Reste, ausgewählt unter Wasserstoff, Alkyl, Cycloalkyl oder Aryl bedeuten,
R^{g} für Wasserstoff, Methyl oder Ethyl steht,
M⁺ für ein Kation steht,
X⁻ für ein Anion steht, und
x für eine ganze Zahl von 1 bis 120 steht,
oder
R⁵ und/oder R⁷ zusammen mit zwei benachbarten Kohlenstoffatomen des Benzolkerns, an den sie gebunden sind, für ein kondensiertes Ringsystem, mit 1, 2 oder 3 weiteren Ringen stehen,
a und b unabhängig voneinander die Zahl 0 oder 1 bedeuten
Pn für ein Pnicogenatom ausgewählt aus den Elementen Phosphor, Arsen oder Antimon steht,
und
R¹, R², R³, R⁴ unabhängig voneinander für Hetaryl, Hetaryloxy, Alkyl, Alkoxy, Aryl, Aryloxy, Cycloalkyl, Cycloalkoxy, Heterocycloalkyl, Heterocycloalkoxy oder eine NE¹E²-Gruppe stehen, mit der Maßgabe, dass R¹ und R³ über das Stickstoffatom an das Pnicogenatom Pn gebundene Pyrrolgruppen sind oder worin R¹ gemeinsam mit R² und/oder R³ gemeinsam mit R⁴ eine mindestens eine über das pyrrolische Stickstoffatom an das Pnicogenatom Pn gebundene Pyrrolgruppe enthaltende zweibindige Gruppe E der Formel
Py-I-W
worin
Py eine Pyrrolgruppe ist,
I für eine chemische Bindung oder für O, S, SiR^{a}R^{b}, NR^{c} oder CR^{h}Rⁱ steht,
W für Cycloalkyl, Cycloalkoxy, Aryl, Aryloxy, Hetaryl oder Hetaryloxy steht,
und
R^{h} und Rⁱ unabhängig voneinander für Wasserstoff, Alkyl, Cycloalkyl, Heterocycloalkyl, Aryl oder Hetaryl stehen,
oder eine über die Stickstoffatome an das Pnicogenatom Pn gebundene Bispyrrolgruppe der Formel
Py-I-Py
bilden,
wobei der Ausdruck Pyrrolgruppe unsubstituierte oder substituierte Pyrrolyl -, Imidazolyl-, Pyrazolyl-, Indolyl-, Purinyl-, Indazolyl-, Benzotriazolyl-, 1,2,3-Triazolyl, 1,3,4*-*Triazolyl- und Carbazolylgruppen umfaßt.

2. Pnicogenchelatliganden gemäß Anspruch 1, in denen Pn für ein Phosphoratom steht.

3. Pnicogenchelatliganden gemäß Anspruch 1, in denen R¹ und R³ für unsubstituierte, über das pyrrolische Stickstoffatom an das Pnicogenatom gebundene Pyrrolyl- oder Imidazolyl-Gruppen stehen.

4. Pnicogenchelatliganden gemäß Anspruch 1, in denen R¹, R², R³ und R⁴ für über das pyrrolische Stickstoffatom an das Pnicogenatom gebundene, unsubstituierte Pyrrolyl- oder Imidazolyl-Gruppen stehen.

5. Pnicogenchelatliganden gemäß Anspruch 1, in denen R¹ und R³ für eine über das pyrrolische Stickstoffatom an das Pnicogenatom gebundene, Pyrrolgruppe ausgewählt aus den Gruppen Indolyl, Pyrazolyl, Indazolyl, Benzotriazolyl, Triazolyl, Purinyl oder Carbazolyl stehen.

6. Pnicogenchelatliganden gemäß Anspruch 1, in denen R¹ gemeinsam mit R² und/oder R³ gemeinsam mit R⁴ eine gegebenenfalls substituierte Bisindoldiyl-Gruppe der Formel 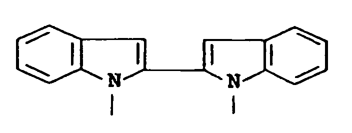 oder eine gegebenenfalls substituierte Bis-pyrroldiyl-methan-Gruppe der Formel 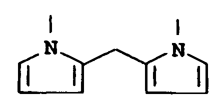 bilden.

7. Pnicogenchelatliganden gemäß Anspruch 1, in denen die Brückengruppe Q für eine Xanthendiyl-Gruppe der Formel 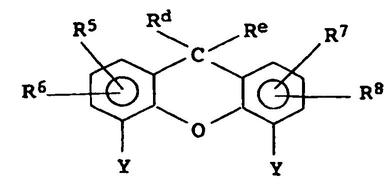 steht, in der R⁵, R⁶, R⁷, R⁸ und Y die in Anspruch 1 genannte Bedeutung haben und R^{d} und R^{e} unabhängig voneinander für Wasserstoff, Alkyl, Cycloalkyl, Heterocyloalkyl, Aryl oder Hetaryl stehen.

8. Pnicogenchelatliganden gemäß Anspruch 1, in denen die Brückengruppe Q für eine Triptycendiyl-Gruppe der Formel 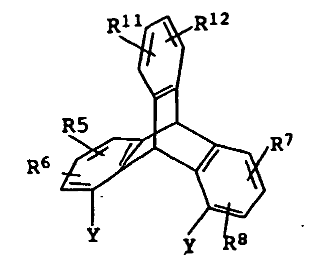 oder der Formel 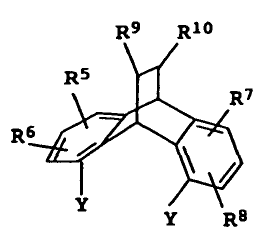 steht, in denen R⁵, R⁶, R⁷, R⁸, R⁹, R¹⁰, R¹¹ und R¹² die in Anspruch 1 genannte Bedeutung haben.

9. Pnicogenchelatliganden gemäß Anspruch 1 allgemeinen Formel II 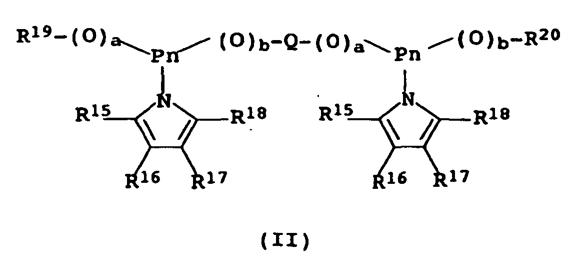 worin
R¹⁵, R¹⁶, R¹⁷ und R¹⁸ unabhängig voneinander für Wasserstoff, Alkyl, Cycloalkyl, Heterocycloalkyl, Aryl, Hetaryl, W'COOR^{k}, W'COO⁻M⁺, W'(SO₃)R^{k}, W'(SO₃)⁻M⁺, W'PO₃(R^{k})(R¹), W'(PO₃)²⁻(M⁺)₂, W'NE⁴E⁵, W'(NE⁴E⁵E⁶)⁺X⁻, W'OR^{k}, W'SR^{k}, (CHR¹CH₂O)_{y}R^{k}, (CH₂NE⁴)_{y}R^{k}, (CH₂CH₂NE⁴)_{y}R^{k}, Halogen, Trifluormethyl, Nitro, Acyl oder Cyano stehen,
worin
W' für eine Einfachbindung, ein Heteroatom oder eine zweiwertige verbrückende Gruppe mit 1 bis 20 Brückenatomen steht,
R^{k}, E⁴, E⁵, E⁶ jeweils gleiche oder verschiedene Reste, ausgewählt unter Wasserstoff, Alkyl, Cycloalkyl oder Aryl bedeuten,
R¹ für Wasserstoff, Methyl oder Ethyl steht,
M⁺ für ein Kationäquivalent steht,
X⁻ für ein Anionäquivalent steht und
y für eine ganze Zahl von 1 bis 240 steht,
wobei jeweils zwei benachbarte Reste R¹⁵, R¹⁶, R¹⁷ und R¹⁸ zusammen mit den Kohlenstoffatomen des Pyrrolrings, an die sie gebunden sind, auch für ein kondensiertes Ringsystem mit 1, 2 oder 3 weiteren Ringen stehen können,
mit der Maßgabe, dass wenigstens einer der Reste R¹⁵, R¹⁶, R¹⁷ oder R¹⁸ nicht für Wasserstoff steht, und dass R¹⁹ und R²⁰ nicht mit einander verknüpft sind,
R¹⁹ und R²⁰ unabhängig voneinander für Cycloalkyl, Heterocycloalkyl, Aryl oder Hetaryl stehen,
a und b unabhängig voneinander die Zahl 0 oder 1 bedeuten,
Pn für ein Pnicogenatom, ausgewählt aus den Elementen Phosphor, Arsen oder Antimon, bevorzugt für Phosphor, steht,
Q eine Brückengruppe der Formel 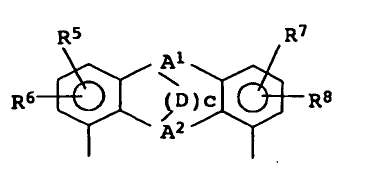
ist,
worin
A¹ und A² unabhängig voneinander für O, S, SiR^{a}R^{b}, NR^{c} oder CR^{d}R^{e} stehen, wobei
R^{a}, R^{b} und R^{c} unabhängig voneinander für Wasserstoff, Alkyl, Cycloalkyl, Heterocycloalkyl, Aryl oder Hetaryl stehen,
R^{d} und R^{e} unabhängig voneinander für Wasserstoff, Alkyl, Cycloalkyl, Heterocycloalkyl, Aryl oder Hetaryl stehen oder die Gruppe R^{d} gemeinsam mit einer weiteren Gruppe R^{d} oder die Gruppe R^{e} gemeinsam mit einer weiteren Gruppe R^{e} eine intramolekulare Brückengruppe D bilden,
D eine zweibindige Brückengruppe, ausgewählt aus den Gruppen 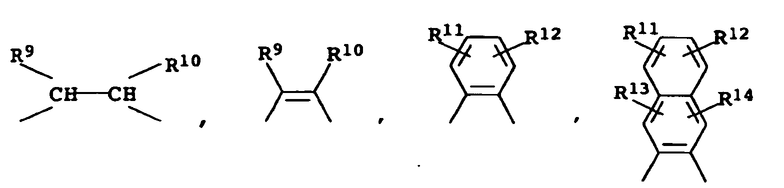
ist, in denen
R⁹ und R¹⁰ unabhängig voneinander für Wasserstoff, Alkyl, Cycloalkyl, Aryl, Halogen, Trifluormethyl, Carboxyl, Carboxylat oder Cyano stehen oder miteinander zu einer C₃- bis C₄-Alkylenbrücke verbunden sind,
R¹¹, R¹², R¹³ und R¹⁴ unabhängig voneinander für Wasserstoff, Alkyl, Cycloalkyl, Aryl, Halogen, Trifluormethyl, COOH, Carboxylat, Cyano, Alkoxy, SO₃H, Sulfonat, NE¹E², Alkylen-NE¹E²E³⁺X⁻, Acyl oder Nitro stehen,
c 0 oder 1 ist,
R⁵, R⁶, R⁷ und R⁸ unabhängig voneinander für Wasserstoff, Alkyl, Cycloalkyl, Heterocycloalkyl, Aryl, Hetaryl, COOR^{f}, COO⁻M⁺, SO₃R^{f}, SO⁻₃M⁺, NE¹E², NE¹E²E³⁺X⁻, Alkylen-NE¹E²E³⁺X⁻, OR^{f}, SR^{f}, (CHR^{g}CH₂O)ₓR^{f}, (CH₂N(E¹))ₓR^{f}, (CH₂CH₂N(E¹))ₓR^{f}, Halogen, Trifluormethyl, Nitro, Acyl oder Cyano stehen,
worin
R^{f}, E¹, E² und E³ jeweils gleiche oder verschiedene Reste, ausgewählt unter Wasserstoff, Alkyl, Cycloalkyl oder Aryl bedeuten,
R^{g} für Wasserstoff, Methyl oder Ethyl steht,
M⁺ für ein Kation steht,
X⁻ für ein Anion steht, und
x für eine ganze Zahl von 1 bis 120 steht,
oder
R⁵ und/oder R⁷ zusammen mit zwei benachbarten Kohlenstoffatomen des Benzolkerns, an den sie gebunden sind, für ein kondensiertes Ringsystem, mit 1, 2 oder 3 weiteren Ringen stehen.

10. Linganden der Formel II gemäß Anspruch 9, die ausgewählt sind unter Verbindungen der allgemeinen Formeln II.1 bis II.3 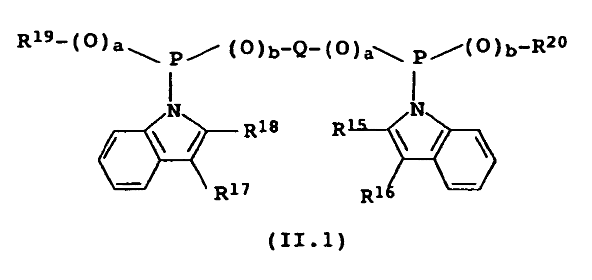 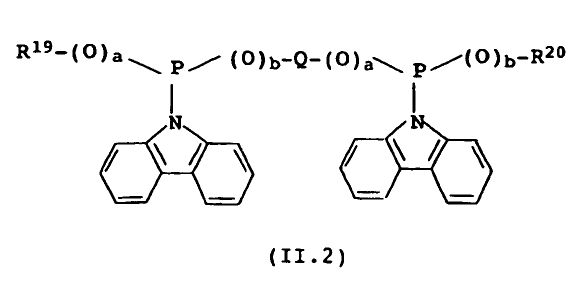 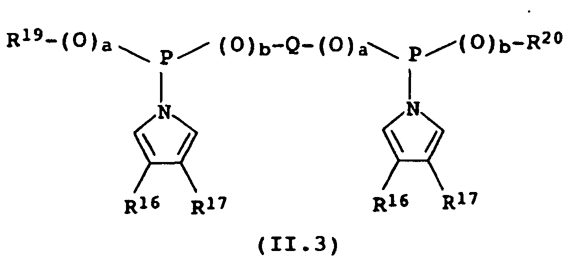 worin
R¹⁵, R¹⁶, R¹⁷, R¹⁸, Q, a und b die in Anspruch 9 angegebenen Bedeutungen besitzen, wobei in der Formel II.3 wenigstens einer der Reste R¹⁶ oder R¹⁷ nicht für Wasserstoff steht,
R¹⁹ und R²⁰ unabhängig voneinander für Cycloalkyl, Heterocycloalkyl, Aryl oder Hetaryl stehen.

11. Katalysatoren, umfassend Pnicogenchelatkomplexe mit einem Metall der VIII. Nebengruppe des Periodensystems der Elemente, die als Liganden mindestens einen Pnicogenchelatliganden gemäß einem der Ansprüche 1 bis 10 enthalten.

12. Katalysatoren gemäß Anspruch 11, in denen das Metall ausgewählt ist aus Kobalt, Rhodium, Ruthenium oder Iridium.

13. Verfahren zur Hydroformylierung von Verbindungen, die wenigstens eine ethylenisch ungesättigte Doppelbindung enthalten durch Umsetzung mit Kohlenmonoxid und Wasserstoff in Gegenwart eines Katalysators, wie in einem der Ansprüche 11 oder 12 definiert.

14. Verfahren zur Herstellung von Aldehyden und/oder Alkoholen durch die Hydroformylierung von C₃-C₂₀-Olefinen bei erhöhtem Druck und erhöhter Temperatur mittels CO/H₂-Gemischen in Gegenwart einer homogen im Reaktionsmedium gelösten Metallkomplexverbindung eines Metalls der VIII. Nebengruppe des Periodensystems der Elemente als Katalysator und freiem Liganden, **dadurch gekennzeichnet, dass** man als Katalysator Pnicogenchelatkomplexe gemäß Anspruch 11 einsetzt und als freien Liganden Pnicogenchelatliganden gemäß einem der Ansprüche 1 bis 10.

15. Verfahren gemäß Anspruch 14, **dadurch gekennzeichnet, dass** man in der Reaktionsmischung ein molares Verhältnis von Ligand zu Metall der VIII. Nebengruppe von 1:1 bis 1000:1 einstellt.

16. Verfahren zur Herstellung von 2-Propylheptanol, bei dem man
a) Buten oder ein Buten enthaltendes C₄-Kohlenwasserstoffgemisch in Gegenwart eines Hydroformylierungskatalysators mit Kohlenmonoxid und Wasserstoff unter Erhalt eines n-Valeraldehyd enthaltenden Hydroformylierungsprodukts hydroformyliert, wobei der Hydroformylierungskatalysator wenigstens einen Komplex eines Metalls der VIII. Nebengruppe mit wenigstens einem Liganden der allgemeinen Formeln I oder II, wie in einem der Ansprüche 1 bis 10 definiert, umfasst.
b) gegebenenfalls das Hydroformylierungsprodukt einer Auftrennung unter Erhalt einer an n-Valeraldehyd angereicherten Fraktion unterzieht,
c) das in Schritt a) erhaltene Hydroformylierungsprodukt oder die in Schritt b) erhaltene an n-Valeraldehyd angereicherte Fraktion einer Aldolkondensation unterzieht,
d) die Produkte der Aldolkondensation mit Wasserstoff katalytisch zu Alkoholen hydriert, und
e) gegebenenfalls die Hydrierprodukte einer Auftrennung unter Erhalt einer an 2-Propylheptanol angereicherten Fraktion unterzieht.

17. Verfahren nach Anspruch 16, wobei der Ligand ausgewählt ist unter Verbindungen der allgemeinen Formel II, wie in einem der Ansprüche 9 oder 10 definiert.

18. Verwendung eines Katalysators, umfassend wenigstens eine Verbindung der allgemeinen Formel I oder II zur Hydroformylierung, Carbonylierung, Hydrocyanierung oder zur Hydrierung.

## Claims

1. A chelating pnicogen ligand of the formula I 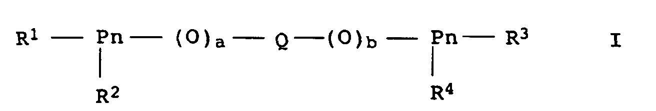 where
Q is a bridging group of the formula 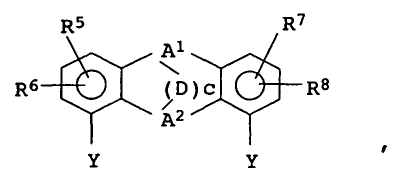
where
A¹ and A² are each, independently of one another, 0, S, SiR^{a}R^{b}, NR^{c} or CR^{d}R^{e}, where
R^{a}, R^{b} and R^{c} are each, independently of one another, hydrogen, alkyl, cycloalkyl, heterocycloalkyl, aryl or hetaryl,
R^{d} and R^{e} are each, independently of one another, hydrogen, alkyl, cycloalkyl, heterocycloalkyl, aryl or hetaryl or the group R^{d} together with a further group R^{d} or the group R^{e} together with a further group R^{e} form an intramolecular bridging group D,
D is a divalent bridging group selected from among the groups 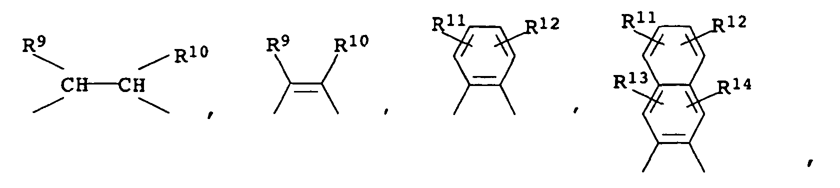
where
R⁹ and R¹⁰ are each, independently of one another, hydrogen, alkyl, cycloalkyl, aryl, halogen, trifluoromethyl, carboxyl, carboxylate or cyano or are joined to one another to form a C₃-C₄-alkylene bridge,
R¹¹, R¹², R¹³ and R¹⁴ are each, independently of one another, hydrogen, alkyl, cycloalkyl, aryl, halogen, trifluoromethyl, COOH, carboxylate, cyano, alkoxy, SO₃H, sulfonate, NE¹E², alkylene-NE¹E²E³⁺X⁻, acyl or nitro,
c is 0 or 1,
Y is a chemical bond,
R⁵, R⁶, R⁷ and R⁸ are each, independently of one another, hydrogen, alkyl, cycloalkyl, heterocycloalkyl, aryl, hetaryl, COOR^{f}, COO⁻M⁺, SO₃R^{f}, SO⁻₃M⁺, NE¹E², NE¹E²E³⁺X⁻, alkylene-NE¹E²E³+X⁻, OR^{f}, SR^{f}, (CHR^{g}CH₂O)ₓR^{f}, (CH₂N(E¹))ₓR^{f}, (CH₂CH₂N(E¹))ₓR^{f}, halogen, trifluoromethyl, nitro, acyl or cyano,
where
R^{f}, E¹, E² and E³ are identical or different radicals selected from among hydrogen, alkyl, cycloalkyl and aryl,
R^{g} is hydrogen, methyl or ethyl,
M⁺ is a cation,
X⁻ is an anion and
x is an integer from 1 to 120,
or
R⁵ and/or R⁷ together with two adjacent carbon atoms of the benzene ring to which they are bound form a fused ring system having 1, 2 or 3 further rings,
a and b are each, independently of one another, 0 or 1,
Pn is a pnicogen atom selected from among the elements phosphorus, arsenic or antimony,
and
R¹, R², R³, R⁴ are each, independently of one another, hetaryl, hetaryloxy, alkyl, alkoxy, aryl, aryloxy, cycloalkyl, cycloalkoxy, heterocycloalkyl, heterocycloalkoxy or an NE¹E² group, with the proviso that R¹ and R³ are pyrrole groups bound via the nitrogen atom to the pnicogen atom Pn, or R¹ together with R² and/or R³ together with R⁴ form a divalent group E of the formula
Py-I-W
containing at least one pyrrole group bound via the pyrrole nitrogen to the pnicogen atom Pn,
where
Py is a pyrrole group,
I is a chemical bond or O, S, SiR^{a}R^{b}, NR^{c} or CR^{h}Rⁱ,
W is cycloalkyl, cycloalkoxy, aryl, aryloxy, hetaryl or hetaryloxy,
and
R^{h} and Rⁱ are each, independently of one another, hydrogen, alkyl, cycloalkyl, heterocycloalkyl, aryl or hetaryl,
or form a bispyrrole group of the formula
Py-I-Py
bound via the nitrogen atoms to the pnicogen atom Pn where the expression pyrrole group comprises unsubstituted or substituted pyrrolyl, imidazolyl, pyrazolyl, indolyl, purinyl, indazolyl, benzotriazolyl, 1,2,3-triazolyl, 1,2,4-triazolyl and carbazolyl groups.

2. The chelating pnicogen ligand according to claim 1 in which Pn is a phosphorus atom.

3. The chelating pnicogen ligand according to claim 1 in which R¹ and R³ are unsubstituted pyrrolyl or imidazolyl groups bound via the pyrrole nitrogen to the pnicogen atom.

4. The chelating pnicogen ligand according to claim 1 in which R¹, R², R³ and R⁴ are unsubstituted pyrrolyl or imidazolyl groups bound via the pyrrole nitrogen to the pnicogen atom.

5. The chelating pnicogen ligand according to claim 1 in which R¹ and R³ are each a pyrrole group bound via the pyrrole nitrogen to the pnicogen atom and selected from among the groups indolyl, pyrazolyl, indazolyl, benzotriazolyl, triazolyl, purinyl and carbazolyl.

6. The chelating pnicogen ligand according to claim 1 in which R¹ together with R² and/or R³ together with R⁴ form an optionally substituted bisindolediyl group of the formula 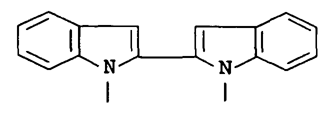 or an optionally substituted bispyrrolediylmethane group of the formula

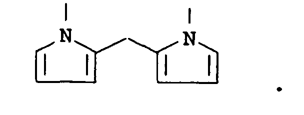

7. The chelating pnicogen ligand according to claim 1 in which the bridging group Q is a xanthenediyl group of the formula 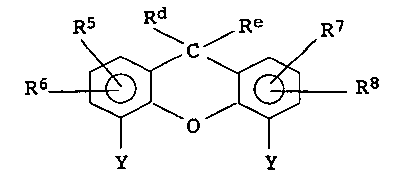 where R⁵, R⁶, R⁷, R⁸ and Y are as defined in claim 1 and R^{d} and R^{e} are each, independently of one another, hydrogen, alkyl, cycloalkyl, heterocyloalkyl, aryl or hetaryl.

8. The chelating pnicogen ligand according to claim 1 in which the bridging group Q is a triptycenediyl group of the formula 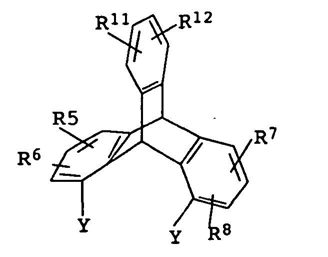 or the formula 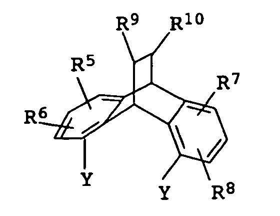 where R⁵, R⁶, R⁷, R⁸, R⁹, R¹⁰, R¹¹ and R¹² are as defined in claim 1.

9. The chelating pnicogen ligand according to claim 1 of the formula II 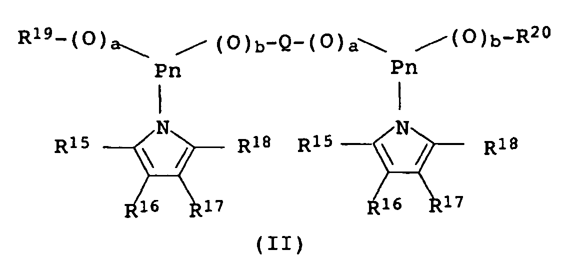 where
R¹⁵, R¹⁶, R¹⁷ and R¹⁸ are each, independently of one another, hydrogen, alkyl, cycloalkyl, heterocycloalkyl, aryl, hetaryl, W'COOR^{k}, W'COO⁻M⁺, W'(SO₃)R^{k}, W'(SO₃)⁻M⁺, W'PO₃(R^{k})(R¹), W'(PO₃)²⁻(M⁺)₂, W'NE⁴E⁵, W'(NE⁴E⁵E⁶)⁺X⁻, W'OR^{k}, W'SR^{k}, (CHR¹CH₂O)_{y}R^{k}, (CH₂NE⁴)_{y}R^{k}, (CH₂CH₂NE⁴)_{y}R^{k}, halogen, trifluoromethyl, nitro, acyl or cyano,
where
W' is a single bond, a heteroatom or a divalent bridging group having from 1 to 20 bridge atoms,
R^{k}, E⁴, E⁵, E⁶ are identical or different radicals selected from among hydrogen, alkyl, cycloalkyl and aryl,
R¹ is hydrogen, methyl or ethyl,
M⁺ is a cation equivalent,
X⁻ is an anion equivalent and
y is an integer from 1 to 240,
where two adjacent radicals R¹⁵, R¹⁶, R¹⁷ and R¹⁸ together with the carbon atom of the pyrrole ring to which they are bound may also form a fused ring system having 1, 2 or 3 further rings,
with the proviso that at least one of the radicals R¹⁵, R¹⁶, R¹⁷ and R¹⁸ is not hydrogen and that R¹⁹ and R²⁰ are not joined to one another,
R¹⁹ and R²⁰ are each, independently of one another cycloalkyl, heterocycloalkyl, aryl or hetaryl,
a and b are each, independently of one another 0 or 1,
Pn is a pnicogen atom selected from among the elements phosphorus, arsenic or antimony, preferably phosphorus,
Q is a bridging group of the formula 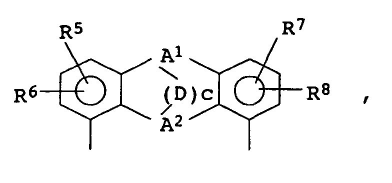
where
A¹ and A² are each, independently of one another, O, S, SiR^{a}R^{b}, NR^{c} or CR^{d}R^{e}, where
R^{a}, R^{b} and R^{c} are each, independently of one another, hydrogen, alkyl, cycloalkyl, heterocycloalkyl, aryl or hetaryl,
R^{d} and R^{e} are each, independently of one another, hydrogen, alkyl, cycloalkyl, heterocycloalkyl, aryl or hetaryl or the group R^{d} together with a further group R^{d} or the group R^{e} together with a further group R^{e} form an intramolecular bridging group D,
D is a divalent bridging group selected from among the groups 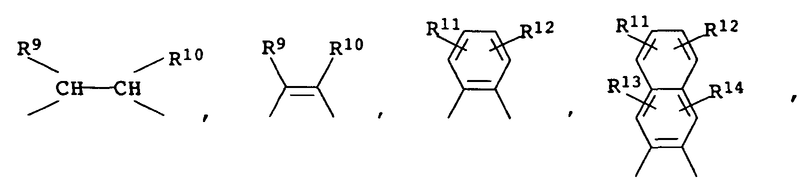
where
R⁹ and R¹⁰ are each, independently of one another, hydrogen, alkyl, cycloalkyl, aryl, halogen, trifluoromethyl, carboxyl, carboxylate or cyano or are joined to one another to form a C₃-C₄-alkylene bridge,
R¹¹, R¹², R¹³ and R¹⁴ are each, independently of one another, hydrogen, alkyl, cycloalkyl, aryl, halogen, trifluoromethyl, COOH, carboxylate, cyano, alkoxy, SO₃H, sulfonate, NE¹E², alkylene-NE¹E²E³⁺X⁻, acyl or nitro,
c is 0 or 1,
R⁵, R⁶, R⁷ and R⁸ are each, independently of one another, hydrogen, alkyl, cycloalkyl, heterocycloalkyl, aryl, hetaryl, COOR^{f}, COO⁻M⁺, SO₃R^{f}, SO⁻₃M⁺, NE¹E², NE¹E²E³⁺X⁻, alkylene-NE¹E²E³⁺X⁻, OR^{f}, SR^{f}, (CHR^{g}CH₂O)ₓR^{f}, (CH₂N(E¹))ₓR^{f}, (CH₂CH₂N(E¹))ₓR^{f}, halogen, trifluoromethyl, nitro, acyl or cyano,
where
R^{f}, E¹, E² and E³ are identical or different radicals selected from among hydrogen, alkyl, cycloalkyl and aryl,
R^{g} is hydrogen, methyl or ethyl,
M⁺ is a cation,
X⁻ is an anion and
x is an integer from 1 to 120,
or
R⁵ and/or R⁷ together with two adjacent carbon atoms of the benzene ring to which they are bound form a fused ring system having 1, 2 or 3 further rings.

10. The ligand of the formula II according to claim 9 which is selected from among compounds of the formulae II.1 to II.3 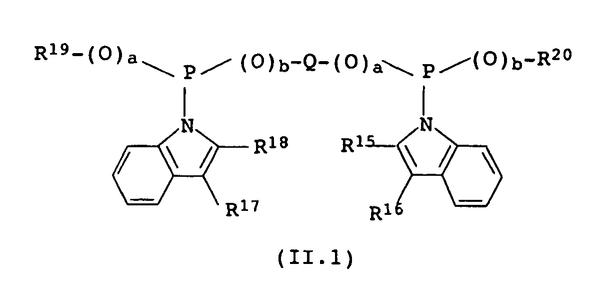 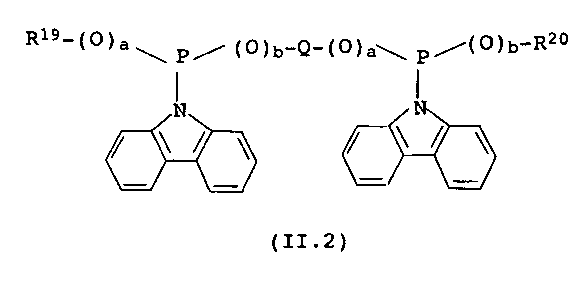 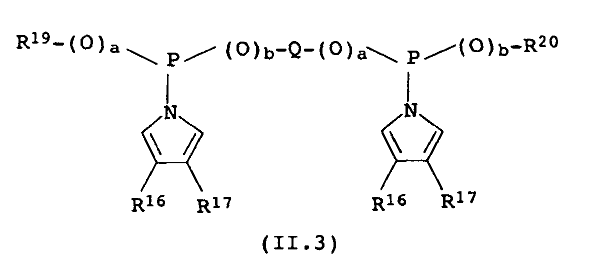 where
R¹⁵, R¹⁶, R¹⁷, R¹⁸, Q, a and b are as defined in claim 9, where, in the formula II.3, at least one of the radicals R¹⁶ and R¹⁷ is not hydrogen,
R¹⁹ and R²⁰ d are each, independently of one another, cycloalkyl, heterocycloalkyl, aryl or hetaryl.

11. A catalyst comprising a pnicogen chelate complex with a metal of transition group VIII of the Periodic Table of the Elements which comprises at least one chelating pnicogen ligand according to any of claims 1 to 10 as ligand.

12. The catalyst according to claim 11 in which the metal is selected from among cobalt, rhodium, ruthenium and iridium.

13. A process for the hydroformylation of compounds comprising at least one ethylenically unsaturated double bond by reaction with carbon monoxide and hydrogen in the presence of a catalyst as defined in claim 11 or 12.

14. The process for preparing aldehydes and/or alcohols by hydroformylation of C₃-C₂₀-olefins at superatmospheric pressure and elevated temperature by means of CO/H₂ mixtures in the presence of a metal complex of a metal of transition group VIII of the Periodic Table of the Elements homogeneously dissolved in the reaction medium as catalyst and free ligand, wherein the catalyst used is a pnicogen chelate complex according to claim 11 and the free ligand used is a chelating pnicogen ligand according to any of claims 1 to 10.

15. The process according to claim 14, wherein a molar ratio of ligand to metal of transition group VIII of from 1:1 to 1000:1 is set in the reaction mixture.

16. The process for preparing 2-propylheptanol, which comprises
a) hydroformylating butene or a butene-containing C₄-hydrocarbon mixture by means of carbon monoxide and hydrogen in the presence of a hydroformylation catalyst to give an n-valeraldehyde-containing hydroformylation product, where the hydroformylation catalyst comprises at least one complex of a metal of transition group VIII with at least one ligand of the formula I or II as defined in any of claims 1 to 10,
b) if appropriate, subjecting the hydroformylation product to a fractionation to give an n-valeraldehyde-enriched fraction,
c) subjecting the hydroformylation product obtained in step a) or the n-valeraldehyde-enriched fraction obtained in step b) to an aldol condensation,
d) catalytically hydrogenating the products of the aldol condensation by means of hydrogen to form alcohols, and
e) if appropriate, subjecting the hydrogenation products to a fractionation to give a 2-propylheptanol-enriched fraction.

17. The process according to claim 16, wherein the ligand is selected from among compounds of the formula II as defined in claim 9 or 10.

18. The use of a catalyst comprising at least one compound of the formula I or II for hydroformylation, carbonylation, hydrocyanation or hydrogenation.

## Revendications

1. Ligands chélates de pnictogène de formule générale 1 : 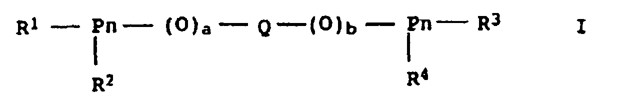 dans laquelle :
Q est un groupe pontant de formule : 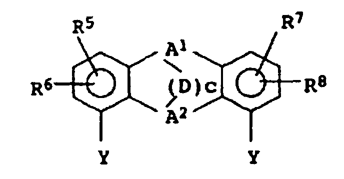
dans laquelle :
A¹ et A² représentent, indépendamment l'un de l'autre, O, S, SiR^{a}R^{b}, NR^{c} ou CR^{d}R^{e}, où
R^{a}, R^{b} et R^{c} représentent, indépendamment l'un de l'autre, de l'hydrogène, un groupement alkyle, cycloalkyle, hétérocycloalkyle, aryle ou hétaryle,
R^{d} et R^{e} représentent, indépendamment l'un de l'autre, de l'hydrogène, un groupement alkyle, cycloalkyle, hétérocycloalkyle, aryle ou hétaryle, ou le groupement R^{d} forme, conjointement avec un autre groupement R^{d}, ou le groupement R^{e}, conjointement avec un autre groupement R^{e} un groupe pontant intramoléculaire D,
D représente un groupement pontant bivalent choisi dans les groupements : 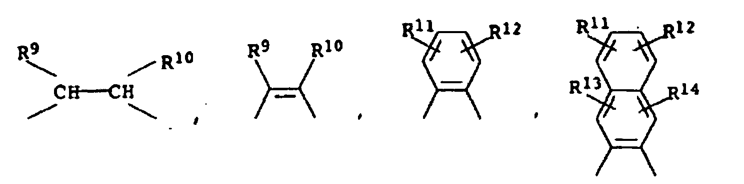
dans lesquels :
R⁹ et R¹⁰ représentent, indépendamment l'un de l'autre, de l'hydrogène, un groupement alkyle, cycloalkyle, aryle, halo, trifluorométhyle, carboxyle, carboxylate ou cyano ou sont fixés l'un à l'autre pour former un pont alkylène en C₃ à C₄,
R¹¹, R¹², R¹³ et R¹⁴ représentent, indépendamment l'un de l'autre, de l'hydrogène, un groupement alkyle, cycloalkyle, aryle, halo, trifluorométhyle, COOH, carboxylate, cyano, alcoxy, SO₃H, sulfonate, NE¹E², alkylène-NE¹E²E³⁺X⁻, acyle ou nitro,
c est 0 ou 1,
Y représente une liaison chimique,
R⁵, R⁶, R⁷ et R⁸ représentent, indépendamment l'un de l'autre, de l'hydrogène, un groupement alkyle, cycloalkyle, hétérocycloalkyle, aryle, hétaryle, COOR^{f}, COO⁻M⁺, SO₃R^{f}, SO⁻₃M⁺, NE¹E², NE¹E²E³⁺X⁻, alkylène-NE¹E²E³⁺X⁻, OR^{f} , SR^{f} , (CHR^{g}CH₂O)ₓR^{f}, (CH₂N(E¹))ₓR^{f}, (CH₂CH₂N(E¹))ₓR^{f}, halo, trifluorométhyle, nitro, acyle ou cyano,
où
R^{f}, E¹, E² et E³ représentent respectivement des radicaux identiques ou différents choisis parmi l'hydrogène, un groupement alkyle, cycloalkyle ou aryle,
R^{g} représente de l'hydrogène, un groupement méthyle ou éthyle,
M⁺ représente un cation,
X⁻ représente un anion, et
x représente un nombre entier de 1 à 120,
ou
R⁵ et/ou R⁷ représentent, conjointement avec deux atomes de carbone adjacents du noyau de benzène, auxquels ils sont fixés, un système cyclique condensé avec 1, 2 ou 3 autres cycles,
a et b représentent, indépendamment l'un de l'autre, le chiffre 0 ou 1,
Pn représente un atome de pnictogène choisi parmi les éléments phosphore, arsenic et antimoine,
et
R¹, R², R³ , R⁴ représentent, indépendamment l'un de l'autre, un groupement hétaryle, hétaryloxy, alkyle, alcoxy, aryle, aryloxy, cycloalkyle, cycloalcoxy, hétérocycloalkyle, hétérocycloalcoxy ou un groupement NE¹E², à condition que R¹ et R³ soient des groupements pyrrole fixés à l'atome de pnictogène Pn via l'atome d'azote ou dans lequel R¹ conjointement avec R² et/ou R³ conjointement avec R⁴ représentent un groupement covalent E de formule :
Py-I-W
contenant au moins un groupement pyrrol fixé à l'atome de pnictogène Pn via l'atome d'azote du pyrrol, formule dans laquelle :
Py est un groupement pyrrole,
I est une liaison chimique ou représente O, S, SiR^{a}R^{b}, NR^{c} ou CR^{h}Rⁱ,
W représente un groupement cycloalkyle, cycloalcoxy, aryle, aryloxy, hétaryle ou hétaryloxy,
et
R^{h} et Rⁱ représentent, indépendamment l'un de l'autre, de l'hydrogène, un groupement alkyle, cycloalkyle, hétérocycloalkyle, aryle ou hétaryle,
ou forment un groupement bispyrrole fixé à l'atome de pnictogène Pn via les atomes d'azote et de formule :
Py-I-Py
où l'expression groupement pyrrole englobe les groupements pyrrolyle, imidazolyle, pyrazolyle, indolyle, purinyle, indazolyle, benzotriazolyle, 1,2,3-triazolyle, 1,3,4-triazolyle et carbazolyle non substitués ou substitués.

2. Ligands chélates de pnictogène selon la revendication 1, dans lesquels Pn désigne un atome de phosphore.

3. Ligands chélates de pnictogène selon la revendication 1, dans lesquels R¹ et R³ désignent des groupements pyrrolyle ou imidazolyle non substitués fixés à l'atome de pnictogène via l'atome d'azote du pyrrol.

4. Ligands chélates de pnictogène selon la revendication 1, dans lesquels R¹, R² R³ et R⁴ représentent des groupements pyrrolyle ou imidazolyle non substitués fixés à l'atome de pnictogène via l'atome d'azote du pyrrol.

5. Ligands chélates de pnictogène selon la revendication 1, dans lesquels R¹ et R³ représentent un groupement pyrrole fixé à l'atome de pnictogène via l'atome d'azote du pyrrol et choisi parmi les groupements indolyle, pyrazolyle, indazolyle, benzotriazolyle, triazolyle, purinyle ou carbazolyle.

6. Ligands chélates de pnictogène selon la revendication 1, dans lesquels R¹ conjointement avec R² et/ou R³ conjointement avec R⁴ forment un groupement bisindoldiyle éventuellement substitué de formule : 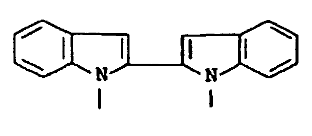 ou un groupement bis-pyrroldiyl-méthane éventuellement substitué de formule :

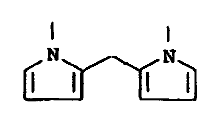

7. Ligands chélates de pnictogène selon la revendication 1, dans lesquels le groupement pontant Q désigne un groupement xanthènediyle de formule : 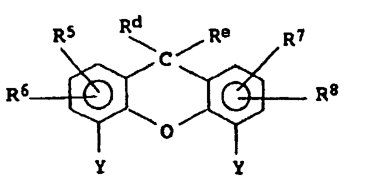 dans laquelle :
R⁵, R⁶, R⁷, R⁸ et Y ont les significations mentionnées dans la revendication 1 et R^{d} et R^{e} représentent, indépendamment l'un de l'autre, de l'hydrogène, un groupement alkyle, cycloalkyle, hétérocycloalkyle, aryle ou hétaryle.

8. Ligands chélates de pnictogène selon la revendication 1, dans lesquels le groupement pontant Q représente un groupement triptycènediyle de formule : 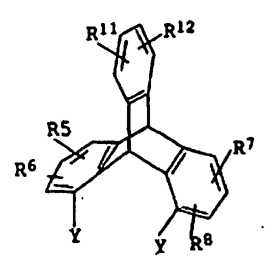 ou de formule : 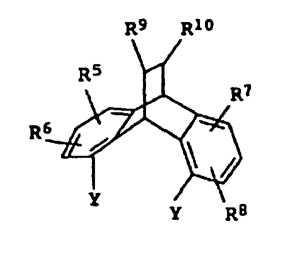 dans lesquelles R⁵, R⁶, R⁷, R⁸, R⁹, R¹⁰, R¹¹ et R¹² ont les significations mentionnées dans la revendication 1.

9. Ligands chélates de pnictogène selon la revendication 1 de formule générale II : 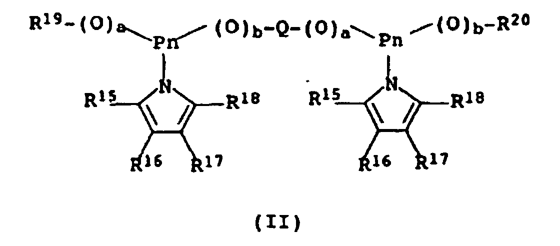 dans laquelle :
R¹⁵, R¹⁶, R¹⁷ et R¹⁸ représentent, indépendamment l'une de l'autre, de l'hydrogène, un groupement alkyle, cycloalkyle, hétérocycloalkyle, aryle, hétaryle, W'COOR^{k}, W'COO⁻M⁺, W'(SO₃)R^{k}, W'(SO₃)⁻M⁺, W'PO₃(R^{k})(R¹), W'(PO₃)²⁻(M⁺)_{2,} W'NE⁴E⁵, W'(NE⁴E⁵E⁶)⁺X⁻, W'OR^{k}, W'SR^{k}, (CHR¹CH₂O)_{y}R^{k}, (CH₂NE⁴)_{y}R^{k}, (CH₂CH₂NE⁴)_{y}R^{k}, halo, trifluorométhyle, nitro, acyle ou cyano,
où
W' représente une liaison simple, un hétéroatome ou un groupement pontant bivalent avec 1 à 20 atomes de pontage,
R^{k} E⁴, E⁵, E⁶ représentent respectivement des radicaux identiques ou différents choisis parmi l'hydrogène, un groupement alkyle, cycloalkyle ou aryle,
R¹ représente de l'hydrogène, un groupement méthyle ou éthyle,
M⁺ représente un équivalent cationique,
X⁻ représente un équivalent anionique, et
y représente un nombre entier de 1 à 240,
où, respectivement, deux radicaux adjacents R¹⁵, R¹⁶, R¹⁷ et R¹⁸, conjointement avec les atomes de carbone du cycle de pyrrole, auxquels ils sont fixés, peuvent représenter également un système cyclique condensé avec 1, 2 ou 3 autres cycles,
à condition qu'au moins l'un des radicaux R¹⁵, R¹⁶, R¹⁷ ou R¹⁸ ne soit pas de l'hydrogène et que R¹⁹ et R²⁰ ne soient pas liés l'un à l'autre,
R¹⁹ et R²⁰ représentent, indépendamment l'un de l'autre, des groupements cycloalkyle, hétérocycloalkyle, aryle ou hétaryle,
a et b représentent, indépendamment l'un de l'autre, le chiffre 0 ou 1,
Pn représente un atome de pnictogène choisi parmi les éléments phosphore, arsenic ou antimoine, de préférence le phosphore,
Q est un groupement pontant de formule : 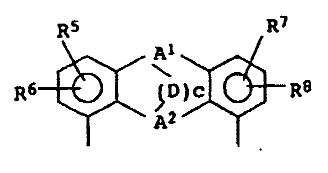 dans laquelle :
A¹ et A² représentent, indépendamment l'un de l'autre, O, S, SiR^{a}R^{b}, NR^{c} ou CR^{d}R^{e},
où
R^{a}, R^{b} et R^{c} représentent, indépendamment l'un de l'autre, de l'hydrogène, un groupement alkyle, cycloalkyle, hétérocycloalkyle, aryle ou hétaryle,
R^{d} et R^{e} représentent, indépendamment l'un de l'autre, de l'hydrogène, un groupement alkyle, cycloalkyle, hétérocycloalkyle, aryle ou hétaryle ou le groupement R^{d}, conjointement avec un autre groupement R^{d}, ou le groupement R^{e}, conjointement avec un autre groupement R^{e} ,forment un groupement pontant intramoléculaire D,
D représente un groupement pontant bivalent choisi parmi les groupes : 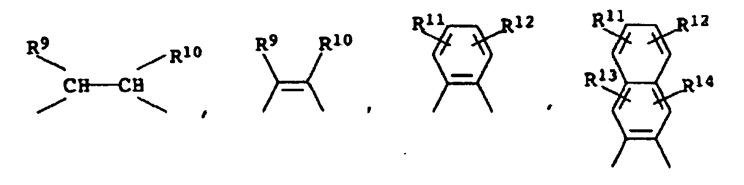 dans lesquels :
R⁹ et R¹⁰ représentent, indépendamment l'un de l'autre, de l'hydrogène, un groupement alkyle, cycloalkyle, aryle, halo, trifluorométhyle, carboxyle, carboxylate ou cyano ou sont fixés l'un à l'autre pour former un pont d'alkylène en C₃ à C₄,
R¹¹, R¹², R¹³ et R¹⁴ représentent, indépendamment l'un de l'autre, de l'hydrogène, un groupement alkyle, cycloalkyle, aryle, halo, trifluorométhyle, COOH, carboxylate, cyano, alcoxy, SO₃H, sulfonate, NE¹E², alkylène-NE¹E²E³⁺X⁻, acyle ou nitro,
c est 0 ou 1
R⁵, R⁶, R⁷ et R⁸ représentent, indépendamment l'un de l'autre, de l'hydrogène, un groupement alkyle, cycloalkyle, hétérocycloalkyle, aryle, hétaryle, COOR^{f}, COO⁻M⁺, SO₃R^{f}, SO⁻₃M⁺, NE¹E², NE¹E²E³⁺X⁻, alkylène-NE¹E²E³⁺X⁻, OR^{f}, SR^{f}, (CHR^{g}CH₂O)ₓR^{f}, (CH₂N(E¹))ₓR^{f}, (CH₂CH₂N(E¹))ₓR^{f}, halo, trifluorométhyle, nitro, acyle ou cyano,
où
R^{f} , E¹, E² et E³ représentent respectivement des radicaux identiques ou différents choisis parmi l'hydrogène, un groupement alkyle, cycloalkyle ou aryle,
R^{g} représente de l'hydrogène, un groupement méthyle ou éthyle,
M⁺ représente un cation,
X⁻ représente un anion, et
x est un nombre entier de 1 à 120,
ou
R⁵ et/ou R⁷, conjointement avec deux atomes de carbone adjacents du noyau de benzène, auquel ils sont fixés, représentent un système cyclique condensé avec 1, 2 ou 3 autres cycles.

10. Ligands de formule II selon la revendication 9, qui sont choisis parmi les composés de formules générales II.1 à II.3 : 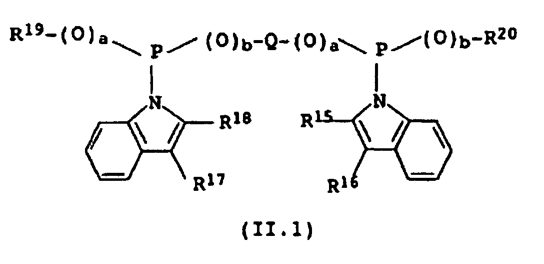 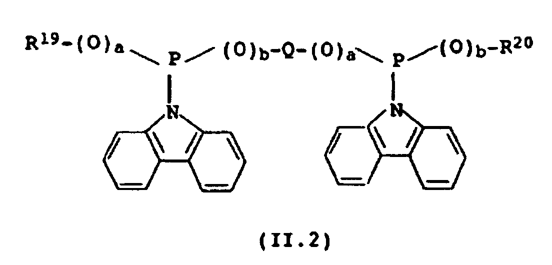 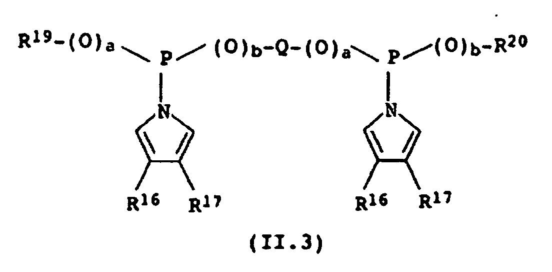 dans lesquelles :
R¹⁵, R¹⁶, R¹⁷, R¹⁸, Q, a et b ont les significations indiquées dans la revendication 9, où, dans la formule II . 3, au moins un des radicaux R¹⁶ ou R¹⁷ n'est pas de l'hydrogène,
R¹⁹ et R²⁰ représentent, indépendamment l'un de l'autre, un groupement cycloalkyle, hétérocycloalkyle, aryle ou hétaryle.

11. Catalyseurs comprenant des complexes chélates de pnictogène avec un métal du groupe secondaire VIII du système périodique des éléments, qui contiennent comme ligands au moins un ligand chélate de pnictogène selon l'une quelconque des revendications 1 à 10.

12. Catalyseurs selon la revendication 11, dans lesquels le métal est choisi parmi le cobalt, le rhodium, le ruthénium ou l'iridium.

13. Procédé d'hydroformylation de composés, qui contiennent au moins une double liaison à insaturation éthylénique par réaction avec du monoxyde de carbone et de l'hydrogène en présence d'un catalyseur, comme défini dans l'une des revendications 11 ou 12.

14. Procédé de fabrication d'aldéhydes et/ou d'alcools par hydroformylation d'oléfines en C₃ à C₂₀ à pression élevée et à température élevée au moyen de mélanges de CO/H₂ en présence d'un composé complexe d'un métal du groupe secondaire VIII du système périodique des éléments dissous de manière homogène dans le milieu réactionnel comme catalyseur et d'un ligand libre, **caractérisé en ce que** l'on utilise comme catalyseur des complexes de chélates de pnictogène selon la revendication 11 et comme ligands libres des ligands chélates de pnictogène selon l'une quelconque des revendications 1 à 10.

15. Procédé selon la revendication 14, **caractérisé en ce qu'**on utilise dans le mélange réactionnel un rapport molaire du ligand au métal du groupe secondaire VIII de 1:1 à 1000:1.

16. Procédé de fabrication de 2-propylheptanol, dans lequel :
a) on hydroformyle du butène ou un mélange d'hydrocarbures en C₄ contenant du butène en présence d'un catalyseur d'hydroformylation avec du monoxyde de carbone et de l'hydrogène pour obtenir un produit d'hydroformylation contenant du n-valéraldéhyde, où le catalyseur d'hydroformylation comprend au moins un complexe d'un métal du groupe secondaire VIII avec au moins un ligand de formules générales I ou II, comme défini dans l'une des revendications 1 à 10;
b) éventuellement le produit d'hydroformylation est soumis à une séparation pour obtenir une fraction enrichie en n-valéraldéhyde,
c) le produit d'hydroformylation obtenu à l'étape
a) ou la fraction enrichie en n-valéraldéhyde obtenue à l'étape b) est soumis(e) à une condensation aldolique,
d) les produits de la condensation aldolique sont hydrogénés avec de l'hydrogène catalytiquement en alcools, et
e) éventuellement les produits d'hydrogénation sont soumis à une séparation pour obtenir une fraction enrichie en 2-propylheptanol.

17. Procédé selon la revendication 16, dans lequel le ligand est choisi parmi les composés de formule générale II, comme défini dans l'une quelconque des revendications 9 ou 10.

18. Utilisation d'un catalyseur, comprenant au moins un composé de formule générale 1 ou II à des fins d'hydroformylation, de carbonylation, d'hydrocyanation ou d'hydrogénation.
